# EUROPEAN PATENT SPECIFICATION

(11) **EP 3 755 362 B1**
(45) Date of publication and mention of the grant of the patent: **19.11.2025**
(21) Application number: 19707740.7
(22) Date of filing: 20.02.2019
(51) Int. Cl.: A61K 38/12, A61P 3/04, G01N 33/48, G01N 33/68

(54) **MC4R AGONIST EFFICACY IN SUBJECTS WITH MC4R DEFICIENCIES AND IMPAIRED NFAT SIGNALING**
MC4R-AGONISTEN-WIRKSAMKEIT BEI PATIENTEN MIT MC4R-MANGEL UND GESTÖRTER NFAT-SIGNALISIERUNG
EFFICACITÉ DE L'AGONISTE MC4R CHEZ DES SUJETS ATTEINTS DE DÉFICIENCES MC4R ET SIGNALISATION NFAT ALTÉRÉE

(30) Priority: 20.02.2018 EP 18157739; 19.04.2018 EP 18168228
(43) Date of publication of application: 30.12.2020
(73) Proprietor: Charité - Universitätsmedizin Berlin, 10117 Berlin (DE)
(72) Inventor: KÜHNEN, Peter, 13465 Berlin (DE); BIEBERMANN, Heike, 12105 Berlin (DE); KRUDE, Heiko, 10589 Berlin (DE)
(74) Representative: Hertin und Partner Rechts- und Patentanwälte PartG mbB
(86) International application number: PCT/EP2019/054186
(87) International publication number: WO 2019/162312

(56) References cited:
- WO-A2-2006/133098
- K�HNEN PETER ET AL: "Melanocortin-4 Receptor Signalling: Importance for Weight Regulation and Obesity Treatment", TRENDS IN MOLECULAR MEDICINE, vol. 25, no. 2, 2018, pages 136 - 148, XP085598864, ISSN: 1471-4914, DOI: 10.1016/J.MOLMED.2018.12.002
- HEYDER NICOLAS A ET AL: "Structures of active melanocortin-4 receptor-Gs-protein complexes with NDP-[alpha]-MSH and setmelanotide", CELL RESEARCH, SPRINGER SINGAPORE, SINGAPORE, vol. 31, no. 11, 24 September 2021 (2021-09-24), pages 1176 - 1189, XP037606830, ISSN: 1001-0602, [retrieved on 20210924], DOI: 10.1038/S41422-021-00569-8
- DATABASE EMBASE [online] ELSEVIER SCIENCE PUBLISHERS, AMSTERDAM, NL; 1 November 2022 (2022-11-01), CLEMENT K ET AL: "Long-term Efficacy of Setmelanotide in Patients With POMC or LEPR Deficiency Obesity", Database accession no. EMB-642673585
- CLEMENT K ET AL: "Long-term Efficacy of Setmelanotide in Patients With POMC or LEPR Deficiency Obesity", JOURNAL OF THE ENDOCRINE SOCIETY 20221101 ENDOCRINE SOCIETY NLD, vol. 6, no. Supplement 1, 1 November 2022 (2022-11-01), pages A14 20220611 to 20220614 Atlanta, GA - A15 CONF, ISSN: 2472-1972
- TINH-HAI COLLET ET AL: "Evaluation of a melanocortin-4 receptor (MC4R) agonist (Setmelanotide) in MC4R deficiency", MOLECULAR METABOLISM, vol. 6, no. 10, 9 July 2017 (2017-07-09), pages 1321 - 1329, XP055471904, ISSN: 2212-8778, DOI: 10.1016/j.molmet.2017.06.015
- JANE E. ROYALTY ET AL: "Investigation of safety, tolerability, pharmacokinetics, and pharmacodynamics of single and multiple doses of a long-acting [alpha]-MSH analog in healthy overweight and obese subjects", JOURNAL OF CLINICAL PHARMACOLOGY., vol. 54, no. 4, 1 April 2014 (2014-04-01), US, pages 394 - 404, XP055522276, ISSN: 0091-2700, DOI: 10.1002/jcph.211
- ANONYMOUS: "Rhythm Initiates Two Phase 2 Clinical Trials of Setmelanotide (RM-493) in Rare Genetic Disorders of Obesity Caused by MC4 Pathway Deficiencies - Rhythm Pharmaceuticals", 4 June 2015 (2015-06-04), pages 1 - 2, XP055319670, Retrieved from the Internet <URL:http://www.rhythmtx.com/news-resources/press-releases/rhythm-initiates-two-phase-2-clinical-trials-of-setmelanotide-rm-493-in-rare-genetic-disorders-of-obesity-caused-by-mc4-pathway-deficiencies/> [retrieved on 20161115]
- GU W ET AL: "IDENTIFICATION AND FUNCTIONAL ANALYSIS OF NOVEL HUMAN MELANOCORTIN-4 RECEPTOR VARIANTS", DIABETES, AMERICAN DIABETES ASSOCIATION, US, vol. 48, no. 3, 1 March 1999 (1999-03-01), pages 635 - 639, XP000866533, ISSN: 0012-1797, DOI: 10.2337/DIABETES.48.3.635

## Description

The invention relates to a Melanocortin-4 receptor (MC4R) agonist that exhibits greater induction of NFAT signaling compared to α-MSH, for use in the treatment and/or prevention of a medical condition associated with MC4R deficiency, in a subject having an MC4R deficiency associated with impaired Nuclear factor of activated T-cells (NFAT) signaling. The present invention further relates to an in vitro method for the diagnosis, prognosis and/or assessment of likelihood of whether a subject with, or at risk of having and/or developing, a medical condition associated with MC4R deficiency, will respond to treatment with an MC4R agonist that exhibits greater induction of NFAT signaling compared to α-MSH, the method comprising (i) providing a sample from said subject, and (ii) determining whether the subject has an MC4R deficiency associated with impaired NFAT signaling by assessing said sample, (iii) wherein the presence of an MC4R deficiency associated with impaired NFAT signaling is indicative that treatment with an MC4R agonist that exhibits greater induction of NFAT signaling compared to α-MSH will be effective in said subject.

### BACKGROUND

Obesity is defined as a state of abnormal or excessive fat accumulation with risk of developing chronic conditions associated with several adverse health consequences such as type 2 diabetes mellitus, hypertension, cardiovascular disease, and certain types of cancer. Obesity has become a global health issue, with 1.46 billion adults overweight and over 200 million men and 300 million women obese worldwide Obesity is commonly caused by imbalanced energy intake and expenditure.

Studies indicated that genetic factors are involved in obesity pathogenesis. Neural melanocortin-4 receptor (MC4R) has been shown to be involved in regulating various physiological processes, such as energy homeostasis, cardiovascular function, reproduction and sexual function, anti-inflammation, and has been extensively studied for its role in obesity pathogenesis. Targeted deletion of Mc4r in mice causes obesity. The conventional signaling pathways of neural MCRs are mediated by the stimulatory G protein Gas, and most of the studies on MC4R focused on this Gαs-cAMP signaling pathway. Until the present time, multiple MC4R variants have been identified, as described e.g. in Tao et al (Endocr Rev. 2010 Aug;31(4):506-43).

However, an increasing number of recent studies have demonstrated that similar to other GPCRs, MC4R can couple to other G proteins or signaling mediators to trigger the downstream signaling pathways, in addition to the conventional or "classical signaling" through Gas-cAMP signaling pathway. The MC4R can also couple to the Gai protein, which inhibits AC activity to decrease cAMP level, and to the Gaq protein, which activates phospholipase C (PLC) and downstream kinase protein kinase C (PKC), as well as to the potassium channel Kir7.1, which occurs independent of G proteins. Such "alternative signaling" pathways, which are for example mediated through Gai or Gaq, will lead to the transcription of NFAT (nuclear factor of activated T-cells), which can be measured experimentally by using an NFAT reporter assay.

Accordingly, alternative signaling pathways downstream of MC4R, which are not mediated through the conventional Gαs pathway, can be mediated through Gαi and Gαq and lead to an induction of NFAT signaling.

Activation of the MC4R by POMC-derived melanocyte stimulating hormone (MSH) peptides represents a critical convergence point in the control of body-weight. The leptin-melanocortin pathway (MC4R-pathway) integrates parallel inputs from the orexigenic peptides ghrelin, Neuropeptide Y (NPY) and Agouti-related-peptide (AgRP), while MC4R-pathway activation dominantly counteracts these orexigens. Limited efficacy of life style intervention or even bariatric surgery in individuals with mutations within the MC4R-pathway demonstrate the dominance of this pathway in body-weight homeostasis^{1,3,4}. In addition to proopiomelanocortin *(POMC)* mutations, which lead to early onset obesity, severe hyperphagia, adrenocorticotropin hormone (ACTH) deficiency and occasionally red hair, other mutations upstream of the MC4R in leptin or the leptin receptor *(LEPR)* similarly result in hyperphagia and early-onset severe obesity in humans^{1,2}. Therapeutics to modulate the MC4R-pathway, until recently, only included leptin administration for leptin-deficient patients³.

Efforts to treat obesity with first-generation MC4R agonists revealed limited efficacy⁴ and were accompanied by adverse side effects including increased blood pressure (BP) and heart rate (HR)^{4,5}. A second-generation MC4R agonist, setmelanotide (also known as RM-493 or BIM-22493), was shown to counteract uncontrollable hyperphagia and profoundly reduced body-weight in patients with POMC deficiency without significant side effects or cardiovascular adverse events⁶. In these subjects, setmelanotide substitutes for the missing endogenous MC4R ligand (alpha-and/or beta-melanocyte-stimulating hormone (MSH)). To date, individuals with POMC deficiency have been treated with setmelanotide for more than 2 years, resulting in profound reductions of hyperphagia and body-weight, without signs of serious adverse side effects.

Early experimental and clinical results have led to the suggestion of treating patients with early onset obesity carrying defects of the POMC-MC4R pathway, which are upstream of MC4R with second-generation MC4R agonists, such as *POMC* or *LEPR* mutations. Unfortunately, such mutations only represent extremely rare cases of early onset obesity and therefore such treatment is only considered for a small subset of patients.

A significant group of patients suffering from early onset obesity carrying mutations in the POMC-MC4R pathway display mutations in the MC4R and account for about 5% of subjects with early onset obesity. However, only 1.7% of them are carriers of loss-of function mutations, which are associated with an impairment of the Gαs downstream signaling of MC4R and have been shown to be at least partially susceptible to setmelanotide treatment¹⁰, since setmelanotide can rescue Gαs downstream signaling of severely impaired mutants. Still, the majority of MC4R mutations do not show a severe impairment of downstream Gαs signaling and such mutations are therefore considered as "functionally wild-type" or "like wild-type". Consequently, it is presently believed that carriers of such "like wild-type" mutations of MC4R, which represent a large group of patients with early onset obesity and mutations in the POMC-MC4R pathway, are not susceptible to treatment with setmelanotide.

Gu et al also disclose the identification and functional analysis of human melanocortin-4 receptor variants (Diabetes, American Diabetes Association, vol. 48, no. 3, 1 March 1999). Nijenhuis et al (Biol. Chem. 2003, 278:22939-45) analysed MC4R variants with poor cell surface expression but otherwise "functionally wild-type" characteristics, e.g. T112M. und I170V.

Various alternative MC4R agonists are described in the art. For example, WO 2006/133098 A2 (Amicus Therapeutics Inc) describes methods of enhancing melanocortin-4 receptor (MC4R) activity and enhancing activity of a mutated MC4R, which affects protein folding and/or processing, by treating with MC4R chaperones that assist with MC4R protein expression, folding or presentation on the cell surface.

Royalty et al (Journal of Clinical Pharmacology, vol. 54, no. 4, 2014, pages 394-404) discloses MC4-NN2-0453 as a selective melanocortin-4-receptor agonist for treatment of obesity. However, neither of these documents teaches the relevant MC4R agonists for treatment of the specific patient group of the present invention. Furthermore these documents do not disclose targeting the modification of any MC4R-related signaling cascade.

Despite these alternative approaches to obesity treatment employing MC4R agonists, there remains a strong need in the art for treatment options for patients with early onset obesity carrying MC4R mutations which are considered as "functionally wild-type" or "like wild-type".

### SUMMARY OF THE INVENTION

In light of the prior art the technical problem underlying the present invention is the provision of means for the treatment of a medical condition associated with MC4R deficiency, such as obesity or hyperphagia. Another problem underlying the invention is the provision of means for the treatment of a medical condition associated with MC4R deficiency, such as obesity of hyperphagia, in a subject having an MC4R deficiency associated with impaired Nuclear factor of activated T-cells (NFAT) signaling. In some embodiments, the problem underlying the invention may be considered providing means for such a treatment in a patient population wherein the MC4R deficiency may be caused by "like wild-type" mutations of MC4R. This patient population is typically not considered treatable using medical agents such as setmelanotide.

This problem is solved by the features of the independent claims. Preferred embodiments of the present invention are provided by the dependent claims.

The invention therefore relates to a Melanocortin-4 receptor (MC4R) agonist, selected from setmelanotide and RM511, for use in the treatment and/or prevention of a medical condition associated with MC4R deficiency, selected from obesity, a metabolic syndrome and/or hyperphagia, in a subject having an MC4R deficiency associated with impaired Nuclear factor of activated T-cells (NFAT) signaling.

The present invention is based on the surprising finding that there is a class of MC4R agonists (including setmelanotide) that induce a "biased downstream signaling" or "alternative signaling" of MC4R that is preferentially mediated through signaling pathways that lead to NFAT signaling, which can be mediated through Gai and Gaq and phospholipase C, wherein "classical signaling" through Gαs and cAMP is only modestly affected in terms of potency. It appears that "classical signaling" through Gαs and cAMP is activated to a much lesser extent by this class of MC4R agonists. The use of this class of alternative MC4R agonists, that exhibit greater induction of NFAT signaling compared to α-MSH, overcomes the known disadvantages of "classical MC4R agonists" that primarily activate the classical Gas-cAMP signaling pathway, such as MSH compounds like LY2112688. The treatment with such classical MC4R agonists leads to increased blood pressure and further cardiovascular side effects and were not further considered for treating severely obese patients.

In experiments leading to the present invention, MC4R signaling was analyzed after stimulation with MSH, LY2112688 (former MSH compound from the company Lilly), and setmelanotide. It turned out that setmelanotide is 100-fold more potent in activating NFAT signaling (downstream signal of Gaq and Gai) compared to MSC and Ly2112688 as demonstrated in the data presented in the examples below. Accordingly, such results have led to the present invention of using MC4R agonists that exhibits greater induction of NFAT signaling compared to α-MSH, selected from setmelanotide and RM511, for use in the treatment and/or prevention of a medical condition associated with MC4R deficiency, selected from obesity, a metabolic syndrome and/or hyperphagia, in a subject having an MC4R deficiency associated with impaired Nuclear factor of activated T-cells (NFAT) signaling.

It has to be stressed that in the last decade, Gαs signaling was primarily analyzed in regard to MC4R signaling and was postulated to be the major driver of MC4R related satiety induction. However, in a surprising finding, Gαq signaling plays an important role in the development of obesity and it was surprisingly found that MC4R deficiency associated with impaired NFAT signaling can occur in subjects with MC4R mutations, which do not display an impaired Gαs signaling and have so far been considered as "like wild-type" variants of the MC4R. As demonstrated in the data presented in the examples below, it was possible to identify MC4R variants in obese children, which do not display any alteration of Gαs signaling, but a significantly reduced NFAT activation compared to wild-type MC4R. Importantly, setmelanotide treatment can rescue this impaired signaling.

It was entirely surprising that that (i) NFAT activation plays a role in body weight regulation and (ii) setmelanotide can rescue this impaired NFAT function. This led to the unexpected finding that MC4R agonists that exhibit greater induction of NFAT signaling compared to α-MSH, such as setmelanotide, can be used in the treatment of a medical condition associated with MC4R deficiency, such as early onset obesity, in a subject having an MC4R deficiency associated with impaired Nuclear factor of activated T-cells (NFAT) signaling.

Embodiments of the invention are therefore based on a combination of two unexpected findings, namely that a class of MC4R agonists (including setmelanotide) induces NFAT activation and that NFAT signaling is defective in a sub-group of obese patients with MC4R deficiency. The treatment of this specific patient group represents a novel treatment in a group of patients previously not considered for treatment with this class of compounds.

In embodiments of the invention, the MC4R agonist for use as a medicament according to the present invention
a. exhibits greater induction of NFAT signaling compared to α-MSH and/or the MC4R agonist LY2112688,
b. preferentially induces Gαq signaling over Gαs signaling, and/or
c. preferentially induces NFAT signaling compared to cAMP signaling, preferably wherein the ratio of NFAT signaling to cAMP signaling is greater compared to the ratio obtained by treatment with α-MSH or the MC4R agonist LY2112688.

According to the invention, the agonist is setmelanotide or RM511.

Compounds such as these may be referred to as having a "setmelanotide profile" or being "setmelanotide-like" (including setmelanotide itself).

According to some embodiments of the present invention, the MC4R deficiency in the subject is not associated with impaired Gαs signaling, or with essentially "functionally wild-type" Gαs signaling. As described above, the majority of MC4R mutations do not show a severe impairment of downstream Gαs signaling and such mutations are therefore considered as "functionally wild-type" or "like wild-type". Consequently, it is presently believed that carriers of such "like wild-type" mutations of MC4R, which represent a large group of patients with early onset obesity and mutations in the POMC-MC4R pathway, are not susceptible to treatment with setmelanotide. The present invention relates in some embodiments to the treatment of this patient group, previously considered not to be treatable with setmelanotide or setmelanotide-like compounds.

The patient group defined for the present invention, namely a medical condition associated with an MC4R deficiency, whereby the MC4R deficiency is associated with impaired Nuclear factor of activated T-cells (NFAT) signaling (after a-MSH challenge), can be determined by a skilled person for any given MC4R genotype. Multiple MC4R variants are known, and more are being discovered. A skilled person can assess NFAT signaling for any given MC4R genotype using the methods described herein without undue effort.

The MC4R signals mainly via activation of the stimulatory G protein (Gs), which activates adenylyl cyclase to convert ATP to cAMP. If a MC4R variant signals in Gs like the wild-type, it is typically termed a "wild-type like" variant. However, *in vitro* studies measuring cAMP levels and cell surface expression after transfection have shown impaired Gαs signaling in only 1.5-1.7% of the identified variants. Therefore, the majority of MC4R mutations do not show a severe impairment of downstream Gαs signaling and such mutations are therefore considered as "functionally wild-type" or "like wild-type". As such it appears that MC4R is involved in alternative signaling pathways beyond Gαs signaling. As demonstrated herein, the inventors have surprisingly identified that NFAT signaling is defective in a sub-group of obese patients with MC4R deficiency. Defective NFAT signaling in MC4R variants can be objectively determined without undue effort.

Determination of the superior efficacy of setmelanotide for NFAT activation prompted a reinvestigation of apparent 'cAMP wild-type'-like MC4R variants that do not alter Gs signaling in obese patients with regard to a-MSH. Interestingly, multiple variants were partial or complete loss-of-function variants for a-MSH-induced NFAT signalling. T112M was one of these variants (refer examples below).

Based on these findings, a classification of MC4R variants is enabled by the present invention, which takes multiple signaling pathways after α-MSH stimulation in vitro into consideration.

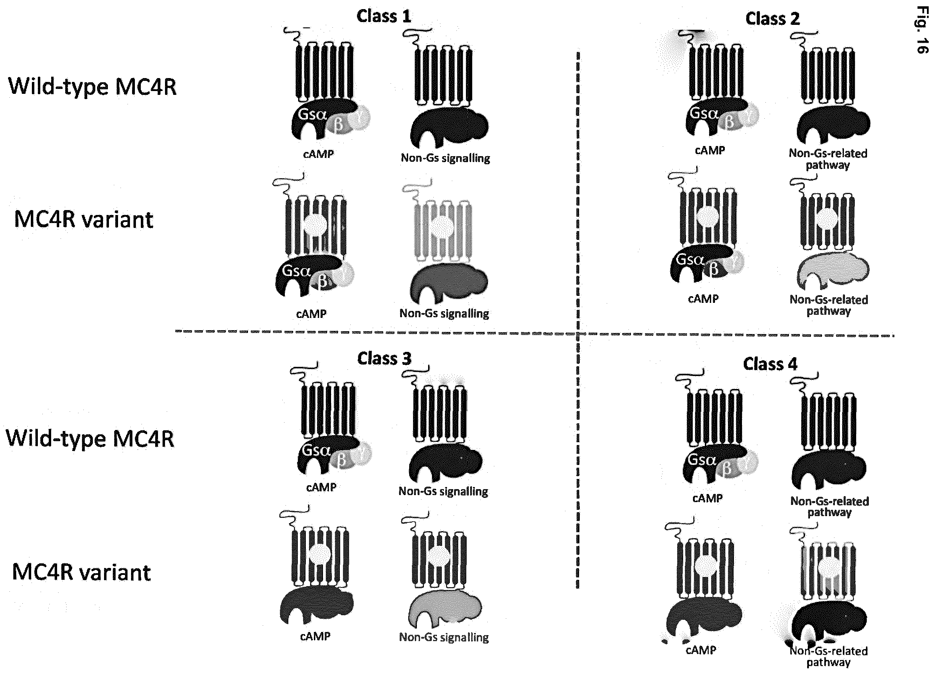

Through this classification, the following classes of MC4R variants can be identified (refer Figure 16):
Class 1: Gs wild type like, non-Gs wild type like;
Class 2: Gs wild type like, non-Gs loss of function;
Class 3: Gs loss of function, non-Gs loss of function;
Class 4: Gs loss of function, non-Gs wild type like.

For this (optional) classification, functional characterisation is preferably performed in heterologous expression systems. This enables an objective and uncomplicated method of assessing which MC4R variants fall into the class of "MC4R deficiency is associated with impaired Nuclear factor of activated T-cells (NFAT) signaling".

In some embodiments, the preferred patient groups (MC4R deficiencies) according to the present invention therefore relate to classes 2 and 3, in which "non-Gs signaling" shows a loss of function, i.e. impaired NFAT signaling. In some embodiments, the preferred patient group (MC4R deficiency) according to the present invention relates to class 2, in which the MC4R variant shows "wild-type like" Gs signaling, but "non-Gs signaling" with a loss of function, i.e. impaired NFAT signaling.

According to the classification described above, and the objective methods available for determining the effect of any given MC4R genotype on Gs and/or NFAT signaling, a skilled person is clearly capable of determining the patient group and particular medical conditions to be treated without undue effort.

In preferred embodiments of the present invention, the MC4R deficiency is a genetic deficiency.

Preferably, the MC4R genetic deficiency is a mutation one or more of the following positions of the MC4R, namely T112, S77, V166, I170, A175, T178, I251 and N274, more preferably S77, I170 and N274.

In some embodiments, the MC4R genetic deficiency is a mutation one or more of the following positions of the MC4R, namely T112, S77, V166, I170, T178, I251 and N274, more preferably S77, I170 and N274.

In some embodiments, the MC4R genetic deficiency is a mutation one or more of the following positions of the MC4R, namely R7, S36, D37, C40, V95, V103, S127, S191, M200, F202, C271 and E308

In some embodiments, the MC4R genetic deficiency is a mutation one or more of the following positions of the MC4R, namely P78L, R165Q, R165W, I125K, C271Y, T11A, A175T, I316L, I316S, I317T, N97D, G98R, N62S, C271R, N97D, Y157S, I102S, L106P, L250Q, Y287X, P299H and S58C.

In some embodiments, the MC4R genetic deficiency is not a mutation one or more of the following positions of the MC4R, namely P78L, R165Q, R165W, I125K, C271Y, T11A, A175T, I316L, I316S, I317T, N97D, G98R, N62S, C271R, N97D, Y157S, I102S, L106P, L250Q, Y287X, P299H and S58C.

In some embodiments, the MC4R genetic deficiency is one or more MC4R mutations at the following positions of the MC4R, namely S4, H6, R7, T11, R18, L23, S30, G32, G34, Y35, S36, S36, D37, C40, E42, P48, V50, F51, L54, G55, S58, E61, N62, V65, I69, A70, N72, K73, N74, H76, S77, P78, M79, C84, A87, D90, S94, V95, N97, G98, T101, I102, V103, L106, T112, I121, I125, D126, S127, V128, S136, I137, A144, D146, R147, T150, A154, Q156, Y157, H158, M161, T162, R165, V166, I169, I170, C172, W174, A175, T178, V179, G181, I186, S191, I194, I195, M200, F201, F202, L207, M208, M215, M218, A219, I226, P230, G231, G231, R236, G238, A239, N240, A244, T246, T248, L250, I251, G252, V253, P260, F261, L263, H264, I269, C271, P272, N274S, P275, F280, M281, I289, C293, S295, P299, L300, I301, Y302, A303, L304, R305, E308, R310, I316, I317, G323, L325, C326, R331 and Y332.

In some embodiments, the MC4R genetic deficiency is one or more MC4R mutations at the following positions of the MC4R, namely S4, H6, R7, R18, L23, S30, G32, G34, Y35, S36, S36, D37, C40, E42, P48, V50, F51, L54, G55, E61, V65, I69, A70, N72, K73, N74, H76, S77, M79, C84, A87, D90, S94, V95, T101, V103, T112, 1121, D126, S127, V128, S136, I137, A144, D146, R147, T150, A154, Q156, H158, M161, T162, V166, I169, I170, C172, W174, T178, V179, G181, I186, S191, 1194, I195, M200, F201, F202, L207, M208, M215, M218, A219, I226, P230, G231, G231, R236, G238, A239, N240, A244, T246, T248, I251, G252, V253, P260, F261, L263, H264, I269, P272, N274S, P275, F280, M281, I289, C293, S295, L300, I301, Y302, A303, L304, R305, E308, R310, G323, L325, C326, R331 and Y332.

In some embodiments, the MC4R genetic deficiency is one or more MC4R mutations at the following positions of the MC4R, namely S4, R7, T11, R18, S30, G32, G34, S36, D37, C40, V50, F51, A70, N74, H76, S77, 1102, V103, T112, I137, D146, A154, Q156, T162, V166, I169, I170, A175, T178, I186, S191, I195, M200, F201, F202, L207, A219, P230, G231, R236, N240, T248, L250, I251, V253, P272, N274, P275, S295, L304, I317, C326, R331 and Y332.

In some embodiments, the MC4R genetic deficiency is one or more MC4R mutations at the following positions of the MC4R, namely S4, R7, R18, S30, G32, G34, S36, D37, C40, V50, F51, A70, N74, H76, S77, V103, T112, I137, D146, A154, Q156, T162, V166, I169, I170, T178, I186, S191, I195, M200, F201, F202, L207, A219, P230, G231, R236, N240, T248, I251, V253, P272, N274, P275, S295, L304, C326, R331 and Y332.

Preferably, the MC4R genetic deficiency is one or more mutation selected from the group consisting of T112M, S77L, V166I, I170V, A175T, T178M, I251L and N274S, more preferably S77L, I170V and N274S.

In some embodiments, the MC4R genetic deficiency is one or more mutations selected from the group consisting of R7C, S36T, D37G, C40Y, V95I, V103I, S127L, S191T, M200del, F202L, C271R and E308L.

In some embodiments, the MC4R genetic deficiency is an MC4R mutation selected from one or more of S4F, H6P, R7C, R7H, T11S, T11A, R18C, R18H, R18L, L23R, S30F, G32E, G34A, Y35C, S36Y, S36T, D37V, D37G, C40R, C40Y, E42K, P48S, V50M, F51L, L54P, G55V, G55D, S58C, S58N, E61K, N62S, V65E, I69M, I69R, I69T, A70T, N72K, K73R, N741, H76R, S77L, P78L, M79I, C84R, A87D, D90N, S94R, S94N, V95I, N97D, G98R, T101A, T101N, I102S, I102T, V103I, L106P, T112M, I121T, I125K, D126Y, S127L, V128L, S136F, S136A, S136P, I137T, A144L, D146H, D146N, R147G, T150F, T150I, A154D, Q156R, Q156P, Y157S, H158R, M161T, T162R, T162I, R165G, R165W, R165Q, V166I, I169S, I170V, C172R, W174C, A175T, T178M, V179A, G181D, I186V, S191T, I194T, I195S, I195V, M200V, F201L, F202L, L207V, M208V, M215L, M218T, A219V, I226T, P230L, G231S, G231V, R236C, G238D, A239V, N240S, A244E, T246A, T248A, L250Q, I251L, G252S, V253I, P260Q, F261S, L263V, H264R, I269N, C271Y, C271R, C271F, P272L, N274S, P275S, F280L, M281V, I289L, C293R, S295P, P299H, P299S, L300Q, I301T, Y302F, A303T, A303P, L304F, R305W, R305S, R305Q, E308K, R310K, I316S, I317T, I317V, G323E, L325F, C326R, R331K, Y332C and Y332H.

In some embodiments, the MC4R genetic deficiency is an MC4R mutation selected from one or more of S4F, H6P, R7C, R7H, T11S, R18C, R18H, R18L, L23R, S30F, G32E, G34A, Y35C, S36Y, S36T, D37V, D37G, C40R, C40Y, E42K, P48S, V50M, F51L, L54P, G55V, G55D, S58N, E61K, V65E, I69M, I69R, I69T, A70T, N72K, K73R, N741, H76R, S77L, M79I, C84R, A87D, D90N, S94R, S94N, V95I, T101A, T101N, I102T, V103I, T112M, I121T, D126Y, S127L, V128L, S136F, S136A, S136P, I137T, A144L, D146H, D146N, R147G, T150F, T150I, A154D, Q156R, Q156P, H158R, M161T, T162R, T162I, R165G, V166I, I169S, I170V, C172R, W174C, T178M, V179A, G181D, I186V, S191T, I194T, I195S, I195V, M200V, F201L, F202L, L207V, M208V, M215L, M218T, A219V, I226T, P230L, G231S, G231V, R236C, G238D, A239V, N240S, A244E, T246A, T248A, I251L, G252S, V253I, P260Q, F261S, L263V, H264R, I269N, C271F, P272L, N274S, P275S, F280L, M281V, I289L, C293R, S295P, P299S, L300Q, I301T, Y302F, A303T, A303P, L304F, R305W, R305S, R305Q, E308K, R310K, I316S, I317V, G323E, L325F, C326R, R331K, Y332C and Y332H.

In some embodiments, the MC4R genetic deficiency is an MC4R mutation selected from one or more of S4F, R7C, T11S, T11A, R18C, R18H, R18L, S30F, G32E, G34A, S36Y, D37G, C40R, C40Y, V50M, F51L, A70T, N741, H76R, S77L, I102T, V103I, T112M, I137T, D146N, A154D, Q156R, Q156P, T162R, T162I, V166I, I169S, I170V, A175T, T178M, I186V, S191T, I195V, M200V, F201L, F202L, L207V, A219V, P230L, G231V, R236C, N240S, T248A, L250Q, I251L, V253I, P272L, N274S, P275S, S295P, L304F, I317T, I317V, C326R, R331K, Y332C and Y332H.

In some embodiments, the MC4R genetic deficiency is an MC4R mutation selected from one or more of S4F, R7C, R18C, R18H, R18L, S30F, G32E, G34A, S36Y, D37G, C40R, C40Y, V50M, F51L, A70T, N741, H76R, S77L, I102T, V103I, T112M, I137T, D146N, A154D, Q156R, Q156P, T162R, T162I, V166I, I169S, I170V, A175T, T178M, I186V, S191T, I195V, M200V, F201L, F202L, L207V, A219V, P230L, G231V, R236C, N240S, T248A, L250Q, I251L, V253I, P272L, N274S, P275S, S295P, L304F, I317V, C326R, R331K, Y332C and Y332H.

The invention therefore encompasses in some embodiments determining one or more of the above-mentioned mutations, as an indicator of whether a subject will respond to treatment. Determining the mutations may therefore relate to determining at protein, DNA or mRNA level the presence of a mutation, or of a nucleic acid sequence encoding such a mutation.

In further embodiments of the invention, the MC4R deficiency is an epigenetic deficiency. Methods for determining epigenetic deficiencies are known to a skilled person, for example by assessing DNA methylation or histone post translation modification in particular genomic regions. Methods for such an analysis are known to a skilled person.

According to the invention, the medical condition associated with MC4R deficiency is obesity (adiposis), a metabolic syndrome and/or hyperphagia.

In further embodiments, the medical condition associated with MC4R deficiency is early onset obesity.

Furthermore, the invention preferably relates to a MC4R agonist, selected from setmelanotide and RM511, for use as a medicament as described herein, comprising testing a sample obtained from the subject and determining whether the individual has an MC4R deficiency associated with impaired NFAT signaling, and providing treatment if the subject is identified as having said MC4R deficiency.

In some embodiments, the determining whether the individual has an MC4R deficiency associated with impaired NFAT signaling comprises a method as described herein.

In some embodiments, determining whether the individual has an MC4R deficiency associated with impaired NFAT signaling comprises identifying any one or more MC4R mutations, or epigenetic abnormalities, in said subject, and comparing these to a list mutations or epigenetic abnormalities that are known to be associated with impaired NFAT signaling, and thereby determining whether the individual has an MC4R deficiency associated with impaired NFAT signaling.

In some embodiments, determining whether the individual has an MC4R deficiency associated with impaired NFAT signaling comprises identifying any one or more MC4R mutations, or epigenetic abnormalities, evident in said subject, and testing whether these MC4R mutations, or epigenetic abnormalities are associated with impaired NFAT signaling.

Suitable methods are disclosed herein, for example by using a reporter system incorporating nucleic acid sequences corresponding to those determined from patient material.

In a further aspect, the present invention relates to a method for determining whether a subject will respond to a treatment comprising the administration of an MC4R agonist, selected from setmelanotide and RM511, that exhibits greater induction of NFAT signaling compared to α-MSH (setmelanotide-like compounds). Such methods are based primarily on determining whether the subject has an MC4R deficiency associated with impaired NFAT signaling.

Therefore, in some embodiments, the present invention relates to an in vitro method for the diagnosis, prognosis and/or assessment of likelihood of whether a subject with, or at risk of having and/or developing, a medical condition associated with MC4R deficiency, selected from obesity, a metabolic syndrome and/or hyperphagia, will respond to treatment with an MC4R agonist, selected from setmelanotide and RM511, that exhibits greater induction of NFAT signaling compared to α-MSH, the method comprising:
- providing a sample from said subject, and
- determining whether the subject has an MC4R deficiency associated with impaired NFAT signaling by assessing said sample,
- wherein the presence of an MC4R deficiency associated with impaired NFAT signaling is indicative that treatment with an MC4R agonist that exhibits greater induction of NFAT signaling compared to α-MSH will be effective in said subject.

It is a particular advantage of the method of the present invention that it is possible to identify subjects or patients that are suffering or are at risk of having or developing a medical condition associated with MC4R deficiency and are likely to respond to treatment with a setmelanotide-like MC4R agonist. This can be achieved by analyzing a sample form said person and determining whether there is a MC4R deficiency associated with impaired NFAT signaling.

In some embodiments, assessing said sample to determine whether the individual has an MC4R deficiency associated with impaired NFAT signaling comprises identifying any one or more MC4R mutations, or epigenetic abnormalities, evident in said subject, and comparing these to a list mutations or epigenetic abnormalities that are known to be associated with impaired NFAT signaling, and thereby determining whether the individual has an MC4R deficiency associated with impaired NFAT signaling. Non-limiting examples of particular positions of mutation or specific mutations are provided herein.

In some embodiments, assessing said sample to determine whether the individual has an MC4R deficiency associated with impaired NFAT signaling comprises identifying any one or more MC4R mutations, or epigenetic abnormalities, evident in said subject, and testing whether these MC4R mutations, or epigenetic abnormalities are associated with impaired NFAT signaling. Suitable methods are disclosed herein, for example by using a reporter system incorporating nucleic acid sequences corresponding to those determined from patient material.

A suitable sample may be any kind of sample from said subject comprising genomic DNA that can be analyzed for genetic or epigenetic deficiencies of MC4R. Alternatively, it may be a sample comprising MC4R expressing cells, wherein the activity and signaling properties of MC4R can be assessed directly in the sample. A skilled person is aware of suitable samples.

The analysis of the sample can involve an analysis of the nucleic acid sequence encoding MC4R, an analysis of the epigenetic state of the genetic material encoding for NFAT, or on an analysis of the protein level and/or function.

A skilled person is aware of suitable means for analysis, for example sequencing analysis of the nucleic acid sequences encoding MC4R or analysis on the protein level for example be mass spectrometry or antibody-mediated techniques such as ELISA and western blot. Sequence information gained through such analysis can be used to test MC4R variants encoded and/or expressed by said subject in suitable signaling assays for assessing NFAT signaling meditated by the subject-specific variants of MC4R. For example, NFAT reporter assays comprising the use of NFAT specific reporter constructs can be performed in cells, in which the patient-specific variant of MC4R, which has been determined by analyzing the sample provided from the patient, is expressed. Such a reporter assay is described in the examples below and a skilled person is able to identify and develop further assays for determining whether a certain MC4R deficiency is associated with impaired NFAT signaling on the basis of the disclosure of the examples and the patent description.

The present invention also relates to a method for determining whether a subject has, or is at risk of having and/or developing, a medical condition associated with MC4R deficiency, selected from obesity, a metabolic syndrome and/or hyperphagia, the method comprising providing a sample from said subject and determining whether the subject has an MC4R deficiency associated with impaired NFAT signaling by assessing said sample.

In embodiments of the methods of the present invention, assessing the sample comprises assessing NFAT signaling in a reporter system comprising nucleic acid sequences corresponding to those determined in patient-specific MC4R genetic material. Suitable reporter systems, without limitation, are those described in the examples below.

According to further embodiments of the methods of the invention, the reporter system comprises means for determining phospholipase C (PLC) activation. Alternatively, other NFAT signaling components may be assessed when assessing NFAT signaling in a reporter system.

In additional embodiments of the methods of the present invention, assessing the sample comprises determining nucleic acid sequence characteristics of patient-specific MC4R genetic material and preferably incorporating nucleic acid sequences corresponding to those determined in patient-specific MC4R genetic material in a reporter system.

Also disclosed herein is a method of improving the bodily appearance of a mammal, wherein said mammal has an MC4R deficiency associated with impaired Nuclear factor of activated T-cells (NFAT) signaling, the method comprising administering to said mammal a melanocortin-4 receptor (MC4R) agonist that exhibits greater induction of NFAT signaling compared to α-MSH in a dosage effective to reduce appetite in said mammal.

Preferably, said method comprises repeating administering said dosage of said MC4R agonist until a cosmetically beneficial loss of body weight has occurred.

Additionally, disclosed herein is a method for reducing body weight of a mammal, wherein said mammal has an MC4R deficiency associated with impaired Nuclear factor of activated T-cells (NFAT) signaling, the method comprising administering to said mammal a melanocortin-4 receptor (MC4R) agonist that exhibits greater induction of NFAT signaling compared to α-MSH in a dosage effective to reduce appetite in said mammal.

The features of embodiments directed to an agonist for use in treatment and methods of treatment apply also to embodiments directed to methods for determining whether a subject will respond to treatment, and vice versa.

### DETAILED DESCRIPTION OF THE INVENTION

As used herein, the "patient" or "subject" may be a vertebrate. In the context of the present invention, the term "subject" includes both humans and animals, particularly mammals, and other organisms.

According to the present invention, the term "indicate" in the context of "is indicative that treatment with an MC4R agonist that exhibits greater induction of NFAT signaling compared to α-MSH will be effective in said subject" is intended as a measure of probability and/or likelihood. Preferably, the "indication" of the effectiveness intended as an assessment of likelihood, and is typically not to be construed in a limiting fashion as to point definitively to the certainty of the effectiveness of the treatment.

As used herein, the terms "diagnosis", "prognosis" and "assessment of likelihood" relate to determining the probability of whether a subject with, or at risk of having and/or developing, a medical condition associated with MC4R deficiency, will respond to treatment with an MC4R agonist that exhibits greater induction of NFAT signaling compared to α-MSH, wherein the presence of an MC4R deficiency associated with impaired NFAT signaling in said subject is correlated or is indicative of an increased probability of responding to the treatment.

As used herein, the term "obese" refers to a subject having a body mass index (BMI) within the ranges defined as "obese" by the Center for Disease Control (see, e.g., URL.cdc.gov/obesity/defining.html and www.cdc.gov/obesity/childhood/defining.html, last accessed on August 19, 2015) or as defined by "Clinical Guidelines on the Identification, Evaluation, and Treatment of Overweight and Obesity in Adults" from the National Institutes of Health.

BMI is obtained by dividing a subject' s weight, e.g., in kilograms (kg) by the square of the subject's height, e.g., in meter (m). For example, an adult who has a BMI of 30 kg/m or higher is considered obese. For example, an adult with a BMI of 25.0 to 29.9 kg/m is considered overweight; an adult with a BMI of 18.5 to 24.9 kg/m is considered to have a normal or healthy weight range; and an adult with a BMI of less than 18.5 kg/m is considered to be underweight. For example, an adult having a height of 5 feet, 9 inches with a body weight of 203 pounds or more is considered obese. For children and teens, obese refers to a subject having a BMI at or above the 85^{th} to 95^{th} percentile for children and teens of the same age and sex.

A subject having obesity refers in some embodiments to a subject having BMI of 30.0 or more, or 30.0 to 34.9 kg/m. A "severely obese" subject or a subject having "severe obesity" refers to a subject having a BMI of 35 kg/m 2 or higher, e.g., 40 kg/m 2 or higher. For example, a severely obese subject is over 100% over the ideal (normal, healthy) body weight.

In non-adults the definition of obesity is related to a BMI above the 95^{th} BMI percentile (severe obesity above 99^{th} percentile) (REF CDC).

As used herein "early onset", e.g., as in early onset obesity, refers to an onset (e.g., first occurrence of one or more symptoms of a disorder, e.g., a disorder described herein, e.g., obesity, PWS, POMC-null obesity) that occurs in a subject before adulthood, e.g., during childhood, e.g., when the subject is 18 years of age or younger (e.g., 18, 17, 16, 15, 14, 13, 12, 11, 10, 9, 8, 7, 6, 5, 4, 3, 2, or 1 year of age or younger, or during adolescence, e.g., when the child is younger than 12 years of age or when the child is younger than 6 years of age). Preferably, early onset obesity relates to an occurrence of obesity before the age of 3 years, more preferably 2 years.

As used herein, the term "metabolic syndrome" refers to a group of symptoms that occur together and increase the risk for coronary artery disease, stroke, and type 2 diabetes. According to the American Heart Association and the National Heart, Lung, and Blood Institute, metabolic syndrome also referred to as Syndrome X) is present if a subject has three or more of the following signs:
1. Blood pressure equal to or higher than 130/85 mmHg;
2. Fasting blood sugar (glucose) equal to or higher than 100 mg/dL;
3. Large waist circumference (length around the waist):
   - Men - 40 inches or more;
   - Women - 35 inches or more;
4. Low HDL cholesterol:
   - Men - under 40 mg/dL;
   - Women - under 50 mg/dL;
5. Triglycerides equal to or higher than 150 mg/dL. Metabolic syndrome can be diagnosed by testing subject' s blood pressure, blood glucose level, HDL cholesterol level, LDL cholesterol level, total cholesterol level, and triglyceride level.

As used herein "treating" includes achieving a therapeutically relevant result, for example one or more of the following results: reducing the body weight (as measured, for example, by a body mass index (BMI) and/or body weight), e.g., compared to a control (e.g., body weight before treatment or a predetermined body weight); reducing the waist circumference, e.g., compared to a control (e.g., waist circumference before treatment or a predetermined waist circumference); reducing the hunger level, e.g., compared to a control (e.g., hunger level before treatment or a predetermined hunger level); increasing the resting energy expenditure (REE), e.g., compared to a control (e.g., REE before treatment or a predetermined REE); decreasing the food intake, e.g., compared to a control level (e.g., before treatment or a predetermined food intake); ameliorating or improving a clinical symptom or indicators associated with a disorder described herein such as obesity, PWS, POMC-null obesity, e.g., type-II diabetes, pre-diabetic condition, blood level of haemoglobin AIC (HbA1c) above 6%, hyperinsulimenia, hyperlipidemia, insulin insensitivity, or glucose intolerance; delaying, inhibiting or preventing the progression of obesity and/or obesity related indications; or partially or totally delaying, inhibiting or preventing the onset or development of obesity or a obesity related indication.

Delaying, inhibiting or preventing the progression of obesity is one aspect of the invention, and includes for example, delaying, inhibiting or preventing the progression of a subject having normal weight to obesity. In embodiments, a control is a value of a parameter measured before treatment by a MC4R agonist described herein or a predetermined value. The term "treating" further includes partially or totally reducing the risk for coronary artery disease, stroke, and type 2 diabetes associated with the metabolic syndrome as well as ameliorating or improving a clinical symptom or signs of metabolic syndrome associated with metabolic syndrome, such as any one or more of the five indicators listed above. For example, the term "treating" includes delaying, inhibiting or preventing the progression of parameters associated with the metabolic syndrome, including insulin resistance, glucose clearance and parameters of cardiovascular disease including heart rate and blood pressure.

"Prophylactic treatment" refers to treatment before onset of obesity to prevent, inhibit or reduce its occurrence, or reduce the risk of its occurrence or reduce the severity of the condition progressing.

In accordance with any method described herein, in certain embodiments, the subject is obese, e.g., prior to administration of an agonist described herein, e.g., at the time the agonist is prescribed, or at the time of the first administration of the agonist. In embodiments, the subject is a severely obese, pediatric or adult patient e.g., prior to administration of an agonist described herein, e.g., at the time the agonist is prescribed or at the time of the first administration of the agonist. In embodiments, the subject is hyperphagic, e.g., prior to administration of an agonist described herein, e.g., at the time the agonist is prescribed, or at the time of the first administration of the agonist.

In embodiments, the subject (e.g., adult subject) has a body mass index (BMI) greater than 25 kg/m² or 30 kg/m² (e.g., > 25, 26, 27, 28, 29, 30, 31, 32, 33, 34, 35, 36, 37, 38, 39, 40, 41, 42, 43, 44, 45, 46, 47, 48, 49, 50 kg/m² or greater) prior to administration of the agonist, e.g., at the time the agonist is prescribed, or at the time of the first administration. In embodiments, the subject (e.g., pediatric subject) has a body mass index (BMI) higher than 85-95 percentile prior to administration of the agonist, e.g., at the time the agonist is prescribed, or at the time of the first administration.

In embodiments, the subject has a body weight of at least about 5 kg, e.g., at least about 5 kg, 10 kg, 20kg, 30, 40, 50, 60, 70, 80, 90, 100, 110, 120, 130, 140,145, 150, 155, 160, 165, 170, 175, 180, 185, 190, 200, 205, 210, 215, 220 kg or greater, e.g., prior to administration of the agonist, e.g., at the time the agonist is prescribed, or at the time of the first administration. In embodiments, the subject has a body weight of a least 20 kg, at least 60 kg, or at least 100 kg, e.g., prior to administration of the agonist, e.g., at the time the agonist is prescribed, or at the time of the first administration.

In embodiments, the subject is an adult, e.g., 18 years of age or older, e.g., 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, or older. In embodiments, the subject is a pediatric subject, e.g., less 18 years of age or younger (e.g., 18, 17, 16, 15, 14, 13, 12, 11, 10, 9, 8, 7, 6, 5, 4, 3, 2, or 1 year of age or younger.

In embodiments, the subject has or is identified as having a defect, e.g., genetic defect, or a mutation, in one or more genes of the POMC-MC4R pathway. In embodiments, the subject has or is identified as having one or more mutations in the POMC, PCSK1, MAGEL2, leptin receptor, leptin, 5-HT2c receptor, NhHL2, pro-hormone convertase, CPE, MC4R, or Sim1 genes or other genes that impair functioning of the POMC-MC4R pathway. In embodiments, the subject has or is identified as having a hypermethylated POMC gene (e.g., hypermethylated at a POMC intron, e.g., at a CpG island of the POMC gene, e.g., comprising a methylated cytosine, e.g., a 5'methyl cytosine).

In embodiments, methods described herein result in one or more outcomes, including a reduction of weight (e.g., body weight), a reduction in hunger level, no detectable decrease in energy expenditure (e.g., resting energy expenditure), an increase in energy expenditure (e.g., resting energy expenditure), a reduction in daily/weekly/monthly food intake, a reduction in waist circumference, no detectable increase in blood pressure, or a reduction in blood pressure in a subject, e.g., relative to a control.

In embodiments, the control is the measurement of the parameter in the subject prior to administration of (treatment with) a MC4R agonist. In embodiments, the control is a predetermined value, e.g., the value of the parameter in an average obese human population, e.g., of like age and gender as the subject; or the value of the parameter measured in the subject at a previous time point (e.g., at a previous visit, e.g., to a physician, medical facility or laboratory).

In embodiments, a therapeutic outcome, or "outcome" may be achieved via treatment with the agonist as described herein. The outcome (e.g., the reduction, increase, no detectable decrease, or no detectable increase in a given parameter) may be measured in the subject 1, 2, 3, 4, 5, 6, 7, 8, 9, 10 weeks or more after initiation of treatment with a MC4R agonist. In other embodiments, the outcome (e.g., the reduction, increase, no detectable decrease, or no detectable increase in a given parameter) is measured in the subject over a period of time (e.g., over a period of 1-2 weeks, 2-4 weeks, 4-6 weeks, 6-8 weeks, 8-12 weeks, or 12-16 weeks) during a course of treatment. In embodiments, methods described herein result in a reduction of weight (e.g., body weight) in the subject compared to a control (e.g., weight of the subject before treatment or a predetermined value, e.g., average weight of an obese human population of like age and gender as the subject not subjected to therapeutic intervention, or the weight of the subject at a previous measurement, e.g., at a previous visit). In embodiments, the reduction is about 1 kg to 3 kg after 1 week of treatment, about 1 kg to 6 kg after 2 weeks of treatment, about 2 kg to 12 kg after 4 weeks of treatment, about 4 kg to 24 kg after 8 weeks of treatment, or about 8 kg to 48 kg after 16 weeks of treatment. In embodiments, the reduction is at a rate of loss of about 1-2 kg/week, e.g., about 2 kg/week, e.g., over a period of 1-2 weeks of treatment or longer, 2-4 weeks of treatment or longer, 4-8 weeks of treatment or longer, 8-16 weeks of treatment, or 16-32 weeks of treatment, or longer. Measurement of weight, e.g., body weight, can be performed using standard methods in the art.

In embodiments, methods described herein result in a reduction in hunger level in the subject compared to a control (e.g., hunger level of the subject before treatment or a predetermined hunger level, e.g., average hunger level of an obese human population of like age and gender as the subject or the hunger level of the subject at a previous measurement, e.g., at a previous visit). In embodiments, the methods described herein result in abolishment of hunger in the subject.

In embodiments, hunger is measured by a scale, such as a Likert hunger scale, which ranges from 0 to 10 and is described herein. In embodiments, methods described herein result in a reduction in hunger score in the subject compared to a control (e.g., hunger level of the subject before treatment or a predetermined hunger level, e.g., average hunger level of an obese human population of like age and gender as the subject or the hunger level of the subject at a previous measurement, e.g., at a previous visit). In embodiments, methods described herein result in a lower score on the Likert hunger scale, e.g., a lower score by at least 1, 2, 3, 4, 5, 6, 7, 8, 9 or 10 points, compared to the control (e.g., hunger level of the subject before treatment or a predetermined hunger level, e.g., average hunger level of an obese human population of like age and gender as the subject or the hunger level of the subject at a previous measurement, e.g., at a previous visit). In embodiments, methods described herein result in a score of 0 on the Likert hunger scale after treatment.

In embodiments, the reduction in hunger level is measured/observed after 1 to 2 weeks of treatment or longer, 2-4 weeks of treatment or longer, 4-8 weeks of treatment or longer, or 8-16 weeks of treatment or longer. REE (resting energy expenditure) is a measure of the basal metabolic rate of the subject and can be determined using methods such as those described in Chen et al. J. Clin. Endocrinol. Metab. 100.4(2015): 1639-45. In embodiments, the REE can be determined by placing the subject in a whole-room indirect calorimeter (also called a metabolic chamber) at a certain time after treatment (e.g., after 3, 4, 5, 6, 7 days, or 1, 2, 3, 4, or more weeks). In embodiments, the REE is measured in 30-minute measurements periods, and in some cases, REE values from several 30-minute periods are averaged to generate an average REE. In embodiments, the REE can be determined after a 10-12 hour fasting period, at thermoneutrality (e.g., around 25 deg C), where the subject is awake without psychological or physical stress. In embodiments, REE is measured in units of energy per unit time (e.g., kcal/h or kcal/day). In embodiments, the REE is measured relative to kg lean body mass in a subject (e.g., REE/kg lean mass). In further embodiments, the REE can be measured by calorimetry.

In embodiments, methods described herein result in no change or no decrease in energy expenditure, e.g., resting energy expenditure (REE), in the subject over an hourly, daily (e.g., in 24 hours), weekly (e.g., in 7 days), or monthly (e.g., in 30 days) period compared to a control REE (e.g., the REE in the subject prior to treatment or a predetermined REE, e.g., average REE of an obese human population of like age and gender and normalized for weight as the subject or the REE of the subject at a previous measurement, e.g., previous visit), e.g., as measured after 3, 4, 5, 6, 7 days, or 1, 2, 3, 4, or more weeks of treatment. In embodiments, methods described herein result in no detectable change or no detectable decrease in energy expenditure, e.g., resting energy expenditure (REE) per kg lean body mass, in the subject over an hourly, daily (e.g., in 24 hours), weekly (e.g., in 7 days), or monthly (e.g., in 30 days) period compared to the control REE (e.g., the REE in the subject prior to treatment or a predetermined REE, e.g., average REE of an obese human population of like age and gender as the subject or the REE of the subject at a previous measurement, e.g., previous visit), e.g., as measured after 3, 4, 5, 6, 7 days, or 1, 2, 3, 4, or more weeks of treatment.

In embodiments, methods described herein result in an increase in energy expenditure, e.g., resting energy expenditure (REE), in the subject over a hourly, daily (e.g., in 24 hours), weekly (e.g., in 7 days), or monthly (e.g., in 30 days) period compared to a control REE (e.g., the REE in the subject prior to treatment or a predetermined REE, e.g., average REE of an obese human population of like age and gender and normalized for weight as the subject or the REE of the subject at a previous measurement, e.g., previous visit), e.g., as measured after 3, 4, 5, 6, 7 days, or 1, 2, 3, 4, or more weeks of treatment.

In embodiments, the increase in REE in the subject is at least 20 kcal/day (e.g., at least 20, 30, 40, 50, 60, 70, 80, 90, 100, 110, 120, 130, 140, 150 kcal/day or more), e.g., as measured after 3, 4, 5, 6, 7 days, or 1, 2, 3, 4, or more weeks of treatment.

In embodiments, the increase in REE in the subject is at least 2% (e.g., at least 2%, 3%, 4%, 5%, 6%, 7%, 8%, 9%, 10%, 11%, 12%, 13%, 14%, 15% or more), e.g., as measured after 3, 4, 5, 6, 7 days, or 1, 2, 3, 4, or more weeks of treatment, compared to the REE in the subject prior to treatment.

In embodiments, the REE in the subject (e.g., adult subject) after treatment with a MC4R agonist (e.g., after 3, 4, 5, 6, 7 days, or 1, 2, 3, 4, or more weeks of treatment) is at least 1800 kcal/day (e.g., at least 1800, 1825, 1850, 1875, 1900, 1925, 1950, 1975, 2000, 2025, 2050, 2100, 2150, 2200, 2250, 2300, 2400 kcal/day, or more), e.g., for an adult subject. In embodiments, the REE in the subject (e.g., pediatric subject) after treatment with a MC4R agonist (e.g., after 3, 4, 5, 6, 7 days, or 1, 2, 3, 4, or more weeks of treatment) is at least 200 kcal/day (e.g., at least 200, 225, 250, 275, 300, 325, 350, 375, 400, 450, 500 kcal/day or more), e.g., for pediatric patients. In embodiments, the REE can be reduced in response to treatment through a balancing regulatory mechanism.

In embodiments, methods described herein result in a reduction in food intake by the subject compared to a control (e.g., the food intake of the subject prior to treatment or a predetermined food intake level, e.g., the food intake of an average human obese population or the food intake of the subject at a previous measurement, e.g., at a previous visit), e.g., where the food intake is measured as daily food intake or food intake over a period of 24 hours, or one week,. In embodiments, the reduction is at least 100 kilocalories, e.g., at least 100, 125, 150, 175, 200, 225, 250, 275, 300, 325, 350, 375, 400, 425, 450, 475, 500, 525, 550, 575, 600, 1000 kilocalories or more, e.g., for daily food intake or food intake over a period of 24 hours, or one week, or 30 days or for longer time periods, e.g., for an adult subject. In embodiments, mean food intake can decrease from a baseline at or above about 100 kcal/kg/day to about 90, 80, 70, 60, 50, 40, 30, 20 or 10 kcal/kg/day or lower after treatment with a MC4R agonist, e.g., setmelanotide, e.g., in a pediatric subject at about 1 year of age. In embodiments, mean food intake can decrease from a baseline at or above about 40 kcal/kg/day to about 35, 30, 20 or 10 kcal/kg/day or lower after treatment with a MC4R agonist, e.g., setmelanotide, e.g., in a pediatric subject in late adolescence. Food intake can be determined by standard methods, e.g., as described in Rutishauser. Pub. Health Nutr. 8.7A(2005): 1100-07.

In embodiments, methods described herein result in a reduction in waist circumference of the subject compared to a control (e.g., the waist circumference of the subject prior to treatment or the waist circumference of the subject at a previous measurement, e.g., previous visit), as measured 1, 2, 3, 4, 5, 6, 7, 8, 9, 10 weeks or more after initiation of treatment.

In embodiments, the reduction in waist circumference is at least 2 cm (e.g., at least 2, 3, 4, 5, 6, 7, 8, 9, 10 cm or more) in the subject (e.g., adult subject) compared to a control (e.g., the waist circumference of the subject prior to treatment or a predetermined waist circumference, e.g., the waist circumference of an average obese human population of like age and gender or the waist circumference of the subject at a previous measurement, e.g., previous visit), as measured 1, 2, 3, 4, 5, 6, 7, 8, 9, 10 weeks or more after initiation of treatment.

In embodiments, the waist circumference is measured using standard methods. In embodiments, the waist circumference is the largest circumference around a subject's midsection, e.g., around a subject's abdomen. In other embodiments, the waist circumference is measured around the natural waist (e.g., in between the lowest rib and the top of the hip bone), the umbilicus, or at the narrowest point of the midsection.

In embodiments, methods described herein result in no detectable increase in blood pressure (e.g., diastolic and/or systolic blood pressure) of the subject compared to a control blood pressure (e.g., the blood pressure of the subject prior to treatment or a predetermined blood pressure, e.g., the blood pressure of an average obese human population of like age and gender or the blood pressure of the subject at a previous measurement, e.g., previous visit), as measured 1, 2, 3, 4, 5, 6, 7, 8, 9, 10 weeks or more after initiation of treatment.

In embodiments, methods described herein result in a reduction in blood pressure (e.g., diastolic and/or systolic blood pressure) of the subject a control blood pressure (e.g., the blood pressure of the subject prior to treatment or a predetermined blood pressure, e.g., the blood pressure of an average obese human population of like age and gender or the blood pressure of the subject at a previous measurement, e.g., previous visit), as measured 1, 2, 3, 4, 5, 6, 7, 8, 9, 10 weeks or more after initiation of treatment. In embodiments, the reduction in blood pressure, e.g., systolic blood pressure, is at least 3 mmHg (e.g., at least 3, 3.5, 4, 4.5, 5, 5.5, 6, 6.5, 7 mmHg or more) compared to the blood pressure of the subject prior to treatment, as measured 1, 2, 3, 4, 5, 6, 7, 8, 9, 10 weeks or more after initiation of treatment. In embodiments, the reduction in blood pressure, e.g., diastolic blood pressure, is at least 4 mmHg (e.g., at least 4, 7, 7.5, 8, 8.5, 9, 9.5, 10 mmHg or more) compared to the blood pressure of the subject prior to treatment, as measured 1, 2, 3, 4, 5, 6, 7, 8, 9, 10 weeks or more after initiation of treatment. In embodiments, the methods described herein do not result in an adverse effect on heart rate or blood pressure.

As used herein, the term "mutation" can refer to an altered nucleic acid sequence of a gene or fragment thereof compared to a wild-type sequence. For example, a mutation can include a point mutation, frame-shift mutation, missense mutation, inversion, deletion, insertion, truncation, chromosomal translocation. In embodiments, a mutation can result in the gene or fragment thereof coding for a non-functional protein, a protein with reduced activity (or a partially functional protein), or a protein with altered activity. For example, a "loss of function" mutation refers to a mutation that results in the gene or fragment thereof coding for a non-functional protein, which has substantially reduced activity compared to its wild-type counterpart (e.g., a non-functional protein has less than 10%, 9%, 8%, 7%, 6%, 5%, 4%, 3%, 2%, 1% or less activity than its wild-type counterpart). For example, "partial loss of function" mutation refers to a mutation that results in the gene or fragment thereof coding for a partially functional protein, which has reduced activity compared to its wild-type counterpart (e.g., without limitation, a partially functional protein has less than 90%, 80%, 70%, 60% or 50% and greater than for example 10% of the activity of its wild-type counterpart).

As used herein "heterozygous" refers to the presence of two different alleles (having different nucleic acid sequences) for a given gene in a subject. In some embodiments, "heterozygous mutation" can refer to the presence of a mutation on one allele for a given gene and the lack of a mutation on the other allele of the same gene in a subject (e.g., one mutant allele and one wild type allele for a given gene). In other embodiments, a "heterozygous mutation" can be a "compound heterozygous" mutation, which refers to the presence of a mutation (e.g., loss of function mutation or partial loss of function mutation) on one allele for a given gene and a different (e.g., loss of function mutation or partial loss of function mutation) on the other allele for the same gene (e.g., two different alleles that are both mutated, e.g., non- functional or partially functional). In embodiments, where a compound heterozygous mutation includes two non-functional alleles, the genotype can be a null genotype or functionally deficient genotype.

As used herein "homozygous" refers to the presence of two identical alleles for a given gene. In some embodiments, a "homozygous mutation" refers to the presence of two mutant alleles for a given gene, where the two mutant alleles are identical.

As used herein "unit dosage" refers to a physically discrete unit suited as unitary doses for a subject to be treated. Each unit contains a predetermined quantity of active compound calculated to produce the desired therapeutic effect in association with the required pharmaceutical carrier.

As used herein "dosage" refers to a quantity or amount of a therapeutic agent. In some embodiments, a dosage is the amount administered to the subject in a single administration, e.g., in a single injection, a single infusion, or single administration of one or more unit dosages. In embodiments, a dosage is the amount administered to the subject in multiple administrations, e.g., multiple injections, multiple infusions, or multiple administrations of one or more unit dosages. In other embodiments, a dosage can refer to the total amount administered to the subject in a certain time period, e.g., per day. In such examples, the dosage is typically referred to as "daily dosage" or dosage in terms of quantity per day.

As used herein "hunger" or "hunger level" refers to a subject's appetite, desire to consume food, or perceived need for food. In embodiments, the hunger or hunger level of a subject can be quantified by using a scale to obtain a hunger score. In embodiments, the scale for hunger assigns a higher score for a subject that more frequently (e.g., often or always) feels unbearable hunger and a lower score for a subject that less frequently (e.g., sometimes or never) feels unbearable hunger. See, e.g., Sibilia. Psychologicol Topics 19 (2010), 2, 341-354. For example, a Likert scale for hunger can be used that assigns scores from 0 to 10 points (0=no hunger; 10=severe hunger). In other examples, a Likert scale for hunger can be used that assigns scores from 1 to 4 points, where a subject who never feels unbearable hunger is assigned a score of 1, where a subject who sometimes feels unbearable hunger is assigned a score of 2, where a subject who often feels unbearable hunger is assigned a score of 3, and where a subject who always feels unbearable hunger is assigned a score of 4.

As used herein, the term "sample" is a biological sample that is obtained or isolated from the patient or subject. "Sample" as used herein may, e.g., refer to a sample of bodily fluid or tissue obtained for the purpose of diagnosis, prognosis, or evaluation of a subject of interest, such as a patient. Preferably herein, the sample is a sample of a bodily fluid, such as blood, serum, plasma, cerebrospinal fluid, urine, saliva, sputum, pleural effusions, cells, a cellular extract, a tissue sample, a tissue biopsy, a stool sample and the like.

Melanocortin-4 receptor (MC4R) is a protein that in humans is encoded by the MC4R gene. It is a G protein-coupled receptor that binds α-melanocyte stimulating hormone (α-MSH). In murine models MC4R has been found to be involved in feeding behaviour, the regulation of metabolism, sexual behavior, and male erectile function. MC4R mutations were reported to be associated with inherited human obesity. They were found in heterozygotes, suggesting an autosomal dominant inheritance pattern.

The Melanocortin-4 receptor (MC4R) protein sequence is known to a skilled person. For example, the NCBI database discloses under Accession number AAB33341 a 332-amino acid protein melanocortin-4 receptor [Homo sapiens]. The Melanocortin-4 receptor (MC4R) gene and its sequence is known to a skilled person. For example, the NCBI database discloses under Accession number NG_016441 an 8438-nucleic acid sequence encoding the melanocortin-4 receptor [Homo sapiens]. RefSeq also discloses under Gene ID 4160 the MC4R melanocortin 4 receptor [Homo sapiens (human)] gene. Analysis of one or more of said protein or nucleic acid sequences (gene or encoding RNA) sequences, or their associated regulatory sequences, is encompassed by embodiments of the present invention.

The term "MC4R agonist" refers to any class of molecules that lead to activation of downstream signaling of MC4R. On the cell membrane, upon agonist binding, MC4R is activated and undergoes conformational changes, thereby generating intracellular signaling, including both G protein-dependent and -independent signaling. Like other Gas-coupled GPCR family members, once MC4R is activated, the α-subunit of Gs protein will disassociate from the βγ heterodimer and then activate adenylyl cyclase (AC) to increase the intracellular cAMP levels and subsequently activate protein kinase A (PKA). This Gas-protein mediated signaling is the conventional or classical intracellular signaling and also the most commonly investigated in MC4R intracellular signaling studies.

In addition to Gas, the MC4R can also couple to other G proteins, such as Gai protein, which inhibits AC activity to decrease cAMP level, and Gaq protein, which activates phospholipase C-β (PLC) and downstream kinase protein kinase C (PKC). It was observed in hypothalamic cell line GT1-7 cells that α-MSH-induced incorporation of GTPyS35 (a measure of G protein activation) can be partially (~50%) blocked by pertussis toxin (PTX) and α-MSH-induced cAMP accumulation is increased due to the PTX treatment by 53% (PTX is a Gαi inhibitor), suggesting that both Gαs and Gai proteins are activated through MC4R upon α-MSH stimulation. It was confirmed that MC4R mediates increase of intracellular Ca2+ level through Gaq-PLC dependent signaling in another hypothalamic cell line, GT1-1 cells.

Activated PLC acting downstream of alternative MC4R signaling pathways including Gαq activation selectively catalyzes the hydrolysis of the phospholipid, phosphatidylinositol 4,5-bisphosphate (PIP2), on the glycerol side of the phosphodiester bond. There is the formation of a weakly enzyme-bound intermediate, inositol 1,2-cyclic phosphodiester, and release of diacyl glycerol (DAG). The intermediate is then hydrolyzed to inositol 1,4,5-trisphosphate (IP3). Thus the two end products are DAG and IP3. DAG and IP3 are important second messengers that control diverse cellular processes and are substrates for synthesis of other important signaling molecules. When PIP2 is cleaved, DAG remains bound to the membrane, and IP3 is released as a soluble structure into the cytosol. IP3 then diffuses through the cytosol to bind to IP3 receptors, particularly calcium channels in the smooth endoplasmic reticulum (ER). This causes the cytosolic concentration of calcium to increase, causing a cascade of intracellular changes and activity. In addition, calcium and DAG together work to activate protein kinase C, which goes on to phosphorylate other molecules, leading to altered cellular activity. End-effects include taste, tumor promotion, as well as vesicle exocytosis, superoxide production from NADPH oxidase, and JNK activation.

A further effect of PLC-induced Ca2+ release is the induction of NFAT signaling. Calcium signaling activates the phosphatase calcineurin and induces movement of NFATc proteins into the nucleus, where they cooperate with other proteins to form complexes on DNA. Activated calcineurin dephosphorylates and induces the nuclear localization of the cytoplasmic components (NFATc proteins) of NFAT transcription complexes. In the nucleus, NFAT transcription complexes assemble on DNA to activate genes. Nuclear factor of activated T-cells (NFAT) is a family of transcription factors shown to be important in immune response, which is also involved in the development and regulation of cardiac, skeletal muscle, and nervous systems. The NFAT transcription factor family comprises at least five members NFATc1, NFATc2, NFATc3, NFATc4, and NFAT5, which can be regulated by calcium signaling. Calcium signaling is critical to NFAT activation because calmodulin (CaM), a well-known calcium sensor protein, activates the serine/threonine phosphatase calcineurin (CN) and activated CN rapidly dephosphorylates the serine-rich region (SRR) and SP-repeats in the amino termini of NFAT proteins, resulting in a conformational change that exposes a nuclear localization signal, resulting in NFAT nuclear import, where NFAT proteins can bind to genetic elements on the DNA and can regulate gene expression.

The term NFAT signaling relates to the Ca2+-mediated activation of NFAT proteins, which occurs in response to activation of alternative signaling pathways of MC4R. NFAT signaling can be measured for example by using genetic reporter systems comprising genetic elements that enable binding of NFAT proteins, leading to the subsequent transcriptional activation and expression of a reporter gene, such as a fluorescent reporter or a luciferase gene, located downstream and/or under the control of the genetic elements enabling binding of NFAT proteins. Accordingly, such reporter systems can be used to assess alternative activation of MC4R, leading to PLC activation, for example through Gαq, and subsequent NFAT signaling.

Assays for measuring classical activation of MC4R resulting in cAMP accumulation as well as assays for measuring alternative activation of MC4R leading to PLC activation and subsequent NFAT signaling are known to the person skilled in the art. Exemplary assays for measuring such a classical and alternative activation are described in the examples below. However, alternative assays that measure molecules or reactions that are specific to either the classical or alternative activation of MC4R may be employed.

Other signaling pathways are also identified in cells expressing MC4Rs, including extracellular signal-regulated kinases 1/2 (ERK1/2), c-Jun N-terminal kinases (JNK), 5'-AMP-activated protein kinase (AMPK), and protein kinase B (PKB or AKT). ERK1/2 pathway is one of three mitogen-activated protein kinases (MAPK) pathways, and the other two pathways are JNK and p38. ERK1/2 activation through MC4R varies based on cell types and ligands used. In GT1-7 cells, ERK1/2 activation through MC4R induced by α-MSH is only PKA-dependent. In rat solitary nucleus neurons, the PKA-dependent ERK1/2 activation through MC4R is increased after central administration of MCR agonist, Melanotan II (MTII). NDP-α-MSH induces ERK1/2 activation via Gi protein in HEK293 cells expressing MC4Rs, but through Ca2+ /PKC pathway in GT1-1 cells. In CHO-KI cells, ERK1/2 activation through MC4R is mediated by phosphoinositide 3-kinase (PI3K) rather than PKA. The diversity of ERK1/2 activation through MC4R makes this signaling pathway complicated. Although β-arrestin mediated ERK1/2 activation was observed in MC5R and other GPCRs such as β2-adrenergic receptor (β2-AR), V2 vasopressin receptor (V2R), parathyroid hormone receptor, subtype 1 (PTH1R), and angiotensin II receptor type 1A (AT1R), and even β-arrestin mediated MC4R endocytosis was demonstrated, there is still no evidence of β-arrestin-mediated ERK1/2 activation in MC4R. For the JNK pathway, NDP-α-MSH does-dependently inhibits JNK in HEK293 cells stably expressing MC4R. AMPK phosphorylation level was demonstrated to be decreased by MC4R activation with MTII in PVH neurons and NDP-α-MSH stimulation in MC4R-transfected HEK293T cells. AKT activation is induced by NDP-α-MSH in GT1-7 cells. Ion channels on the cell membrane are thought to be effectors in a variety of signaling pathways initiated by GPCRs. Regulation of ion channels by G protein can be indirect (via second messengers and activation of downstream kinase) or direct (via G protein βγ subunits). One recent study showed that MC4R in PVH neurons can couple to potassium channel Kir7.1 independent of G protein, further expanding the intracellular signaling effectors of MC4R.

Known MC4R agonists comprise, without limitation, the α-MSH, β-MSH, γ-MSHj, ACTH, Afamelanotide, Bremelanotide, Melanotan II, Modimelanotide, Setmelanotide, AZD2820, LY-2112688, MK-0493, PF-00446687, PG-931, PL-6983, Ro 27-3225, THIQ.

Activation of MC4R is thought to occur according to a multi-state model of receptor activation and differs from previous ternary complex models suggesting the receptor has a single signaling-competent conformation resulting in activation of all signaling pathways. According to the multi-state model receptor activation is a highly dynamic process in which multiple active conformations can be induced by different molecules to mediate different signaling pathways, thus introducing the concept of biased signaling: the activated receptor selectively triggers a distinct signaling pathway rather than others. Biased signaling, as an important concept in GPCR intracellular signaling, has been identified in many members of GPCR family. Neural MCRs and in particular MC4R are able to selectively stabilize particular receptor active conformations and preferentially trigger distinct signaling pathways, consistent with the multi-state model of receptor activation and biased signaling in other GPCRs.

Melanocortin-4 receptor (MC4R) agonists that exhibit greater induction of NFAT signaling compared to α-MSH relate to a class of MC4R agonists that induce a biased downstream signaling of MC4R leading to a greater or stronger induction of alternative signaling or NFAT signaling, which may be mediated through Gαi or Gαq, as compared to stimulation with MSC.

Such MC4R agonists are also referred to as MC4R agonists with a setmelanotide profile and include setmelanotide, RM511 and the α-MSH analogue MC4-NN2-0453 as well as analogues thereof.

Furthermore, multiple MC4R agonists that (potentially) exhibit greater induction of NFAT signaling compared to α-MSH comprising setmelanotide are disclosed in WO2017/059076A1 and can be described by the general formula
(R²R³)-A¹-o(A²-A³-A⁴-A⁵-A⁵-A⁷-A⁸-A⁹)-A¹⁰-R¹, wherein:
- A¹ is Acc, HN- (CH₂)ₘ- C(O) , L- or D -amino acid, or deleted;
- A² is Cys, D-Cys, hCys, D-hCys, Pen, D-Pen, Asp, or Glu;
- A³ is Gly, Ala, β-Ala, Gaba, Aib, D -amino acid, or deleted;
- A⁴ is H is, 2-Pal, 3-Pal, 4-Pal, Taz, 2-Thi, 3-Thi, or (X¹, X², X³, X⁴, X⁵)Phe;
- A⁵ is D-Phe, D-I-Nal, D-2-Nal, D-Trp, D-Bal, D-( X¹, X², X³, X⁴, X⁵)Phe, L-Phe or D-(Et)Tyr;
- A⁶ is Arg, hArg, Dab, Dap, Lys, Orn, or HN-CH((CH₂)ₙ-N(R⁴R⁵))-C(0);
- A⁷ is Trp, 1-Nal, 2-Nal, Bal, Bip, D-Trp, D-2-Nal, D-Bal or D-Bip;
- A⁸ is Gly, D-Ala, Acc, Ala, 13-Ala, Gaba, Apn, Ahx, Aha, HN-(CH₂)₈-C(0), or deleted; A⁹ is Cys, D-Cys, hCys, D-hCys, Pen, D-Pen, Dab, Dap, Orn, or Lys;
- A¹⁰ is Acc, HN-(CH₂)ᵣC(0), L- or D-amino acid, or deleted;
- R¹ is OH or NH₂;
- each of R² and R³ is, independently for each occurrence, selected from the group consisting of H, (C₁-C₃₀)alkyl, (C₁-C₃₀)heteroalkyl, (C₁-C₃₀)acyl, (C₂-C₃₀)alkenyl, (C₂-C₃₀)alkynyl, aryl(C₁-C₃₀)alkyl, aryl(C₁-C₃₀)acyl, substituted (C₁-C₃₀)alkyl, substituted (C₁-C₃₀)heteroalkyl, substituted (C₁-C₃₀)acyl, substituted (C₂-C₃₀)alkenyl, substituted (C₂-C₃₀)alkynyl, substituted aryl(C₁-C₃₀)alkyl, and substituted aryl(C₁-C₃₀)acyl;
- each of R⁴ and R⁵ is, independently for each occurrence, H, (C₁-C₄₀)alkyl, (C₁-C₄₀)heteroalkyl, (C₁-C₄₀)acyl, (C₂-C₄₀)alkenyl, (C₂-C₄₀)alkynyl, aryl(C₁-C₄₀)alkyl, aryl(C₁-C₄₀)acyl, substituted (C₁-C₄₀)alkyl, substituted (C₁-C₄₀)heteroalkyl, substituted (C₁-C₄₀)acyl, substituted (C₂-C₄₀)alkenyl, substituted (C₂-C₄₀)alkynyl, substituted aryl(C₁-C₄₀)alkyl, substituted aryl(C₁-C₄₀)acyl, (C₁-C₄₀)alkylsulfonyl, or -C(NH)-NH₂;
- m is, independently for each occurrence, 1, 2, 3, 4, 5, 6 or 7;
- n is, independently for each occurrence, 1, 2, 3, 4 or 5;
- s is, independently for each occurrence, 1, 2, 3, 4, 5, 6, or 7;
- t is, independently for each occurrence, 1, 2, 3, 4, 5, 6, or 7;
- X¹, X², X³, X⁴ and X⁵ each is, independently for each occurrence, H, F, Cl, Br, I, (C₁-C₁₀)alkyl, substituted (C₁-₁₀)alkyl, (C₂-₁₀)alkenyl, substituted (C₂-₁₀)alkenyl, (C₂-₁₀)alkynyl, substituted (C₂-₁₀)alkynyl, aryl, substituted aryl, OH, NH₂, NO₂, or CN.

"Acyl" refers to R"-C(0)-, where R" is H, alkyl, substituted alkyl, heteroalkyl, substituted heteroalkyl, alkenyl, substituted alkenyl, aryl, alkylaryl, or substituted alklyaryl, and is indicated in the general formula of a particular embodiment as "Ac". "Alkyl" refers to a hydrocarbon group containing one or more carbon atoms, where multiple carbon atoms if present are joined by single bonds. The alkyl hydrocarbon group may be straight-chain or contain one or more branches or cyclic groups.

"Hydroxy alkyl" refers to an alkyl group wherein one or more hydrogen atoms of the hydrocarbon group are substituted with one or more hydroxy radicals, such as hydroxymethyl, hydroxyethyl, hydroxypropyl, hydroxybutyl, hydroxypentyl, hydroxyhexyl and the like.

"Substituted alkyl" refers to an alkyl wherein one or more hydrogen atoms of the hydrocarbon group are replaced with one or more substituents selected from the group consisting of halogen, (i.e., fluorine, chlorine, bromine, and iodine), -OH, -CN, -SH, amine (e.g., -NH2, -NHCH3), -NO2, guanidine, urea, amidine, and -C₁₋₂₀ alkyl, wherein said -C₁₋₂₀ alkyl optionally may be substituted with one or more substituents selected, independently for each occurrence, from the group consisting of halogens, -CF3, -OCH3, -OCF3, and -(CH2)₀₋₂₀-COOH. In different embodiments 1, 2, 3 or 4 substituents are present. The presence of -(CH2) ₀₋₂₀-COOH results in the production of an alkyl acid. Non-limiting examples of alkyl acids containing, or consisting of, -(CH2)₀₋₂₀-COOH include 2-norbornane acetic acid, tert-butyric acid, 3-cyclopentyl propionic acid, and the like.

The term "halo" encompasses fluoro, chloro, bromo and iodo.

"Heteroalkyl" refers to an alkyl wherein one of more of the carbon atoms in the hydrocarbon group is replaced with one or more of the following groups: amino, amido, -O- ,-S- or carbonyl. In different embodiments 1 or 2 heteroatoms are present.

"Substituted heteroalkyl" refers to a heteroalkyl wherein one or more hydrogen atoms of the hydrocarbon group are replaced with one or more substituents selected from the group consisting of halogen, (i.e., fluorine, chlorine, bromine, and iodine), -OH, -CN, -SH, -NH2, -NHCH3, -N02, and -C₁₋₂₀ alkyl, wherein said -C₁₋₂₀ alkyl optionally may be substituted with one or more substituents selected, independently for each occurrence, from the group consisting of halogens, -CF3, -OCH3, -OCF3, and -(CH2) ₀₋₂₀-COOH. In different embodiments 1, 2, 3 or 4 substituents are present. "Alkenyl" refers to a hydrocarbon group made up of two or more carbons where one or more carbon-carbon double bonds are present. The alkenyl hydrocarbon group may be straight-chain or contain one or more branches or cyclic groups.

"Substituted alkenyl" refers to an alkenyl wherein one or more hydrogens are replaced with one or more substituents selected from the group consisting of halogen (i.e., fluorine, chlorine, bromine, and iodine), -OH, -CN, -SH, -NH2, -NHCH3, -N02, and -C₁₋₂₀ alkyl, wherein said -C₁₋₂₀ alkyl optionally may be substituted with one or more substituents selected, independently for each occurrence, from the group consisting of halogens, -CF3, -OCH3, -OCF3, and -(CH2) ₀₋₂₀-COOH. In different embodiments 1, 2, 3 or 4 substituents are present.

"Aryl" refers to an optionally substituted aromatic group with at least one ring having a conjugated pi-electron system, containing up to three conjugated or fused ring systems. Aryl includes carbocyclic aryl, heterocyclic aryl and biaryl groups. Preferably, the aryl is a 5- or 6-membered ring. Preferred atoms for a heterocyclic aryl are one or more sulfur, oxygen, and/or nitrogen. Non-limiting examples of aryl include phenyl, 1-naphthyl, 2-naphthyl, indole, quinoline, 2-imidazole, 9-anthracene, and the like. Aryl substituents are selected from the group consisting of -C₁₋₂₀alkyl, - C₁₋₂₀ alkoxy, halogen (i.e., fluorine, chlorine, bromine, and iodine), -OH, -CN, -SH, -NH2, -N02, C1₋₂₀ alkyl substituted with halogens, -CF3, -OCF3, and -(CH2)₀₋₂₀-COOH. In different embodiments the aryl contains 0, 1, 2, 3, or 4 substituents.

"Alkylaryl" refers to an "alkyl" joined to an "aryl".

The term "(C₁₋₁₂)hydrocarbon moiety" encompasses alkyl, alkenyl and alkynyl and in the case of alkenyl and alkynyl there is C2-C12.

For the avoidance of doubt, unless otherwise indicated, the term "substituted" means substituted by one or more defined groups. In the case where groups may be selected from a number of alternative groups, the selected groups may be the same or different. For the avoidance of doubt, the term independently means that where more than one substituent is selected from a number of possible substituents, those substituents may be the same or different.

MC4R agonists of the present invention, selected from setmelanotide and RM511, are further characterized as exhibiting greater induction of NFAT signaling compared to Ly2112688, which is a strong activator of classical MC4R signaling.

MC4R agonists of the present invention, selected from setmelanotide and RM511, can further be characterized as preferentially inducing Gαq signaling over Gαs signaling, as can be determined by various assays known to a person skilled in the art.

An MC4R agonist inducing greater NFAT signaling compared to α-MSH can also be identified by measuring NFAT signaling and cAMP signaling after stimulation with a respective agonist and comparing the ratio of NFAT signaling over cAMP signaling to the ratio of NFAT signaling over cAMP signaling obtained after stimulation with α-MSH or LY2112688. This method of forming the ratio represents an objective method of determining the preferred signaling pathway of a given agonist in comparison to other agonists, irrespective of the employed agonist concentrations and the readouts of NFAT and cAMP signaling. Similarly, such comparisons can be made with other readouts of classical and alternative MC4R signaling.

Functional characterization of MC4R agonists can be carried out using established methods. For example, as was carried out for setmelanotide in the examples, characterization of any given MC4R agonist is possible using the following techniques:
HEK293 cells for cAMP accumulation assays and PLC activation (NFAT-luc assays) are cultured in L-glutamine containing MEM Earle's media supplemented with 5 % FBS (Biochrom AG, Berlin, Germany) and 1x non-essential amino acids (Biochrom AG, Berlin, Germany) at 37 °C with 5 % CO₂. For cAMP and NFAT-luc measurements, 1.5 *10⁴ *HEK293* cells per well were seeded in poly-L-lysine-coated (Biochrom AG, Berlin, Germany) 96-well plates (15,000 cells/well).

Measurement of cAMP accumulation can be carried out to assess whether any given MC4R agonist preferentially induces NFAT signaling compared to α-MSH, or preferentially induces NFAT signaling over cAMP signaling, or preferentially induces Gαq signaling over G_{αs} signaling.

By way of example, Gαs signaling is determined by measuring cAMP accumulation. Forty-eight hours following transfection in *HEK293* cells, stimulation is conducted with compounds diluted in a HEPES-buffered solution containing 1 mM 3-isobutyl-1-methylxanthine (IBMX, Sigma Aldrich, St. Louis, MO) to inhibit cAMP degradation by phosphodiesterases. Cells are incubated for 40 minutes with a compound of interest, setmelanotide, LY2112688 or alpha-MSH in concentrations ranging from10⁻⁶ M to 10⁻¹² M in order to stimulate adenylyl cyclase. Substance incubation is performed in triplicates and at 37 °C with 5 % CO₂ and stopped by aspirating the medium. Cells are then lysed at 4 °C on a shaking platform with lysis buffer containing 5 nM HEPES, 0.1% BSA, 0.3% Tween20 and 1 mM IBMX. Intracellular cAMP accumulation is determined by a competitive immunoassay based on the AlphaScreen technology (Perkin-Elmer Life Science, Boston, MA) as previously described¹⁸.

Measurement of PLC by luciferase reporter gene assay can also be employed in order to determine whether any given MC4R agonist preferentially induces NFAT signaling compared to α-MSH, or preferentially induces NFAT signaling over cAMP signaling, or preferentially induces Gαq signaling over Gαs signaling.

By way of example, PLC activation is determined by NFAT-luc assays (Promega, Fitchburg, WI). This method requires the co-transfection of equal amounts (0.45 ng/µL DNA of each plasmid) of MC4R and the reporter constructs pGL4.30[luc2P/NFAT-RE/Hygro] (Promega, Fitchburg, WI) containing a response element and firefly luciferase gene under the control of NFAT. Two days post-transfection, cells are incubated for six hours with ligands in supplement-free MEM at 37 °C with 5 % CO₂. In case of determination the effect of G activation of Gi/o, blocking with PTX (50 ng/ml) is carried out 18 hours before the experiment. In case of AgRP interfering with PLC activation, co-stimulation of ligands in the presence of 100 nM AgRP is performed. The reaction is terminated by aspirating of the media. Cells are lysed for 15 minutes on a shaking platform at room temperature using 1x passive lysis buffer (Promega, Fitchburg, WI). Measurement is conducted with automatic luciferase substrate injection of 40 µL in a black 96-well plate using a Berthold Microplate Reader (Berthold Technologies GmbH & Co KG, Bad Wildbad, Germany).

The methods above are exemplary non-limiting means for reliably determining the desired functional properties of the compound.

The invention further relates to a pharmaceutical composition for the use as described herein comprising the agonists described herein. The agonist may also be referred to as a compound. Administration of a compound or pharmaceutically acceptable salt thereof or a composition comprising a compound or pharmaceutical salt of a compound of the invention useful to practice the methods described herein, can be continuous, hourly, four times daily, three time daily, twice daily, once daily, once every other day, twice weekly, once weekly, once every two weeks, once a month, or once every two months, or longer or some other intermittent dosing regimen.

Examples of administration of a compound or composition comprising a compound or pharmaceutical salt of a compound of the invention include peripheral administration. Examples of peripheral administration include oral, subcutaneous, intraperitoneal, intramuscular, intravenous, rectal, transdermal or intranasal forms of administration.

As used herein, peripheral administration can include all forms of administration of a compound or a composition comprising a compound of the instant invention which excludes intracranial administration. Examples of peripheral administration include, but are not limited to, oral, parenteral (e.g., intramuscular, intraperitoneal, intravenous or subcutaneous injection, extended release, slow release implant, depot and the like), nasal, vaginal, rectal, sublingual or topical routes of administration, including transdermal patch applications and the like. The pharmaceutical composition may be administered according to the dosage or unit dosage described herein.

The term "medical condition associated with MC4R deficiency" comprises, without limitation, obesity (adiposis), in particular early onset obesity, metabolic syndrome and/or hyperphagia.

In the context of the present invention, the term "MC4R deficiency" relates to any modification or alteration of the function of MC4R protein, which may be due to genetic or epigenetic alterations or deficiencies as well as deficiencies or alterations occurring on the transcriptional or posttranslational level or alterations at any other stage leading to altered or abolished function of MC4R. In particular, MC4R deficiencies may lead to impaired or biased downstream signaling of MC4R. Such deficiencies may lead to an abolishment of downstream signaling in general, or they may involve selective impairment and/or enhancement of specific downstream signaling pathways. In particular, alternative or classical signaling of MC4R may be differentially affected by MC4R deficiency.

In the context of the present invention, it was an entirely surprising finding that MC4R deficiencies displaying intact "like wild-type" classical Gαs-cAMP signaling and impaired alternative NFAT signaling, which can be mediated by Gαq, suffer from early onset obesity and can be treated by MC4R agonists of the present invention, such as setmelanotide.

In preferred embodiments, MC4R deficiencies involve genetic deficiencies. Genetic deficiencies comprise mutations of the MC4R coding nucleic acid sequence of the genome as well as epigenetic deficiencies, leading to alteration of MC4R.

Epigenetic modifications involve, without limitation, covalent modifications of either DNA (e.g. cytosine methylation and hydroxymethylation) or of histone proteins (e.g. lysine acetylation, lysine and arginine methylation, serine and threonine phosphorylation, and lysine ubiquitination and sumoylation).

MC4R deficiencies that are based on genetic or other mutations leading to variants of MC4R in which one or more amino acids have been replaced by other amino acids or have been deleted or leading to frameshift mutations are known and comprised by the present invention.

Multiple point mutations of MC4R proteins have been described and are comprised by the MC4R deficiencies present invention as well as corresponding mutations of the nucleic acid sequence encoding MC4R leading to these mutations on the protein level. MC4R mutations of the present invention comprise S4F, H6P, R7C, R7H, T11S, T11A, R18C, R18H, R18L, L23R, S30F, G32E, G34A, Y35C, S36Y, S36T, D37V, D37G, C40R, C40Y, E42K, P48S, V50M, F51L, L54P, G55V, G55D, S58C, S58N, E61K, N62S, V65E, I69M, I69R, I69T, A70T, N72K, K73R, N741, H76R, S77L, P78L, M79I, C84R, A87D, D90N, S94R, S94N, V95I, N97D, G98R, T101A, T101N, I102S, I102T, V103I, L106P, T112M, I121T, I125K, D126Y, S127L, V128L, S136F, S136A, S136P, I137T, A144L, D146H, D146N, R147G, T150F, T150I, A154D, Q156R, Q156P, Y157S, H158R, M161T, T162R, T162I, R165G, R165W, R165Q, V166I, I169S, I170V, C172R, W174C, A175T, T178M, V179A, G181D, I186V, S191T, I194T, I195S, I195V, M200V, F201L, F202L, L207V, M208V, M215L, M218T, A219V, I226T, P230L, G231S, G231V, R236C, G238D, A239V, N240S, A244E, T246A, T248A, L250Q, I251L, G252S, V253I, P260Q, F261S, L263V, H264R, I269N, C271Y, C271R, C271F, P272L, N274S, P275S, F280L, M281V, I289L, C293R, S295P, P299H, P299S, L300Q, I301T, Y302F, A303T, A303P, L304F, R305W, R305S, R305Q, E308K, R310K, I316S, I317T, I317V, G323E, L325F, C326R, R331K, Y332C and Y332H.

In preferred embodiments of the invention, the mutation is a "like wild-type" mutation, which does not display a severe impairment of the classical MC4R signaling pathway mediated by Gαs and cAMP, and is selected preferably selected from the group comprising S4F, R7C, T11S, T11A, R18C, R18H, R18L, S30F, G32E, G34A, S36Y, D37G, C40R, C40Y, V50M, F51L, A70T, N741, H76R, S77L, I102T, V103I, T112M, I137T, D146N, A154D, Q156R, Q156P, T162R, T162I, V166I, I169S, I170V, A175T, T178M, I186V, S191T, I195V, M200V, F201L, F202L, L207V, A219V, P230L, G231V, R236C, N240S, T248A, L250Q, I251L, V253I, P272L, N274S, P275S, S295P, L304F, I317T, I317V, C326R, R331K, Y332C and Y332H. Such "like wild-type" mutations have been extensively described in the literature.

In another preferred embodiment of the invention, the mutation is a "like wild-type" mutation that is associated with an impaired alternative, PLC or NFAT signaling of MC4R, and is preferentially selected from the group comprising T112M, S77L, V166I, I170V, A175T, T178M, I251L and N274S.

In some embodiments, a sample must be tested, or it must be determined whether the individual has an MC4R deficiency associated with impaired NFAT signaling by assessing a sample obtained from a subject, ie a patient to be assessed whether they will respond to the treatment described herein. The invention thereby encompasses in embodiments acquiring a sequence of a subject.

The testing or assessing of a sample, in some embodiments, may involve obtaining or providing a sample (e.g., a blood, serum, urine, or tissue (e.g., biopsy) sample) from the subject. In embodiments, the MC4R sequence, e.g., mutation, is detected in a nucleic acid in the sample by a method chosen from one or more of: a nucleic acid hybridization assay, an amplification-based assay, a PCR-RFLP assay, real-time PCR, sequencing, next generation sequencing, screening analysis, FISH, spectral karyotyping or MFISH, comparative genomic hybridization, in situ hybridization, SSP, HPLC or mass-spectrometric genotyping, pyrosequencing or Sanger sequencing.

In some embodiments, the presence or absence of a MC4R mutation is to be determined in a subject. Knowledge of the presence or absence of a MC4R mutation can be acquired in a number of ways. In some embodiments, a sequence is acquired, e.g., by obtaining possession of a nucleotide sequence, by "directly acquiring" or "indirectly acquiring" the sequence.

"Directly acquiring a sequence" means performing a process (e.g., performing a synthetic or analytical method) to obtain the sequence, such as performing a sequencing method (e.g., a Next Generation Sequencing (NGS) method). "Indirectly acquiring a sequence" refers to receiving information or knowledge of, or receiving, the sequence from another party or source (e.g., a third party laboratory that directly acquired the sequence). The sequence acquired need not be a full sequence, e.g., sequencing of at least one nucleotide, or obtaining information or knowledge, that identifies a genotype or predetermined sequence, e.g., mutation, disclosed herein as being present in a subject constitutes acquiring a sequence.

In embodiments, the sequence can be directly acquired. Directly acquiring a sequence includes performing a process that includes a physical change in a physical substance, e.g., a starting material, such as a tissue sample, e.g., a blood sample or tissue biopsy, or analysis of an isolated nucleic acid (e.g., DNA or RNA) sample. Exemplary changes include making a physical entity from two or more starting materials, shearing or fragmenting a substance, such as a genomic DNA fragment; separating or purifying a substance (e.g., isolating a nucleic acid sample from a tissue); combining two or more separate entities into a mixture, performing a chemical reaction that includes breaking or forming a covalent or non-covalent bond. Directly acquiring a value includes performing a process that includes a physical change in a sample or another substance as described above.

In some embodiments, acquiring knowledge of the certain genotype or predetermined sequence, e.g., mutation, can comprise acquiring a sample, e.g., from which the genotype or predetermined sequence, e.g., mutation, is determined. "Acquiring a sample" as the term is used herein, refers to obtaining possession of a sample, e.g., a tissue sample or nucleic acid sample, by "directly acquiring" or "indirectly acquiring" the sample. "Directly acquiring a sample" means performing a process (e.g., performing a physical method such as a surgery or extraction) to obtain the sample. "Indirectly acquiring a sample" refers to receiving the sample from another party or source (e.g., a third party laboratory that directly acquired the sample). Directly acquiring a sample includes performing a process that includes a physical change in a physical substance, e.g., a starting material, such as a tissue, e.g., a tissue in a human patient or a tissue that has was previously isolated from a patient.

In some embodiment, the method comprises assessing NFAT signaling in a reporter system comprising nucleic acid sequences corresponding to those determined in patient-specific MC4R genetic material. This may, without limitation, encompass determining the sequence of MC4R genetic material from a subject, cloning the sequences into a suitable vector, introducing the vector into cells to be assessed and analyzing NFAT signaling.

Functional characterization of *MC4R* variants, in order to determine whether a mutation is an MC4R deficiency associated with impaired NFAT signaling, is therefore possible for any given MC4R sequence.

In some embodiments, MC4R variants are generated by standard mutagenesis techniques and verified by sequencing. Transient transfection of *HEK293* cells with *MC4R* plasmid-DNA or co-transfection with *NFAT* reporter gene (0.45 ng/µl of each plasmid) is performed 24 hours after seeding in supplement-free advanced MEM (Life technologies, Carlsbad, CA), using Metafectene (Biontex, Munich, Germany) according to the manufactures protocol. *CHO-K1* cells stably expressing MC4R (Discoverx Corp, Fremont CA, USA; Product code 05-0050E2 for cAMP Hunter^{™} eXpress cells, tested for mycoplasma every four weeks) are utilized to study the antagonism of setmelanotide and LY2112688 response by AGRP-83-132-amide (Anaspec (Fremont, CA). These cells were plated in a 96-well plate for the assay as per manufacturer's instructions.

In some embodiments, the method may comprise PLC activation as a readout for NFAT signaling. In some embodiments, PLC activation is determined by NFAT-luc assays (such as those sold by Promega, Fitchburg, WI). This method may encompass the co-transfection of equal amounts (eg 0.45 ng/µL DNA of each plasmid) of MC4R and the reporter constructs pGL4.30[luc2P/NFAT-RE/Hygro] (Promega, Fitchburg, WI) containing a response element and firefly luciferase gene under the control of NFAT. Two days post-transfection, cells are incubated with ligands. Cells are lysed. Measurement is conducted with automatic luciferase substrate injection using a suitable Microplate Reader. Alternative assays are known to a skilled person and may be employed to determine whether the genetic material of a subject has a MC4R deficiency associated with deficient NFAT signaling.

The methods above are exemplary non-limiting means for reliably determining whether any given MC4R mutation is an MC4R deficiency associated with impaired Nuclear factor of activated T-cells (NFAT) signaling.

Unless defined otherwise, all technical and scientific terms used herein have the same meaning as commonly understood by one of ordinary skill in the art to which this invention belongs.

### FIGURES

The invention is further described by the following figures. These are not intended to limit the scope of the invention, but represent preferred embodiments of aspects of the invention provided for greater illustration of the invention described herein.

### Brief description of the figures:

Figure 1: Body weight course and hunger-score during Setmelanotide treatment.
Figure 2: Weight course of LEPR patients before setmelanotide treatment start.
Figure 3: Blood pressure and heart rate before and during setmelanotide treatment.
Figure 4: Skin and hair color.
Figure 5: Determination of setmelanotide, alpha-MSH and first generation MSH LY2112688 induced effect on cAMP formation.
Figure 6: Determination of phospholipase C activation after setmelanotide, alpha-MSH and LY2112688 challenge.
Figure 7: Controls for off-target effect of solvents for alpha-MSH, setmelanotide and LY2112688 and effects at MC3R.
Figure 8: Structural models of MC4R-ligand complexes.
Figure 9: Comparison between binding modes of setmelanotide and alpha-MSH at hMC4R.
Figure 10: Structural homology model of alpha-MSH/hMC4R/Gprotein complex with highlighted positions of mutants reported to be "like wild-type" for Gas mediated signaling.
Figure 11: Functional characterization of like wild-type MC4R variants in Gas and PLC signaling after alpha-MSH and setmelanotide challenge.
Figure 12: Functional characterization of like wild-type MC4R variants in Gas and PLC signaling after alpha-MSH and setmelanotide challenge.
Figure 13: MC4R deficiency associated with impaired NFAT.
Figure 14: Investigation of NFAT signaling in mouse embryonic fibroblasts (MEF) deficient in Gq (MEF Dq).
Figure 15: Test of further MC4R variants.
Figure 16. Schematic illustration of the classification of melanocortin-4 receptor (MC4R) variants.

### Detailed description of the figures:

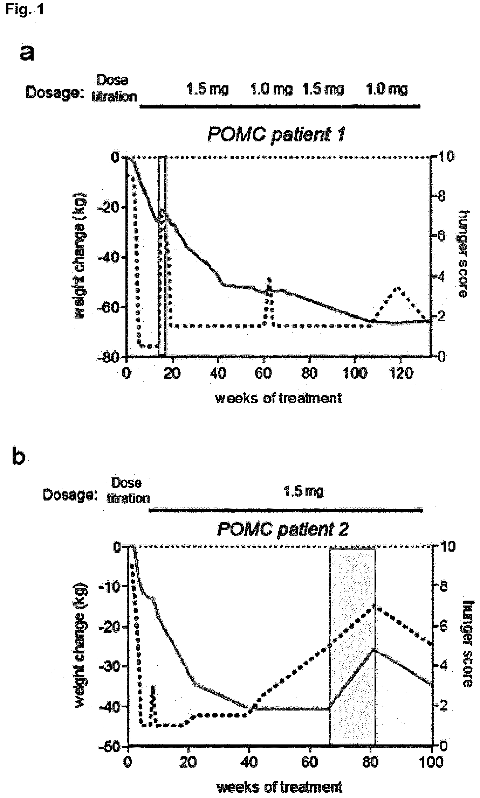

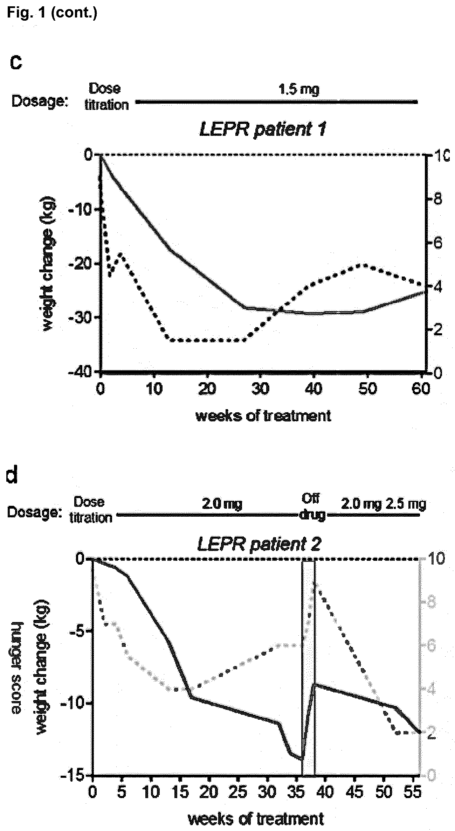

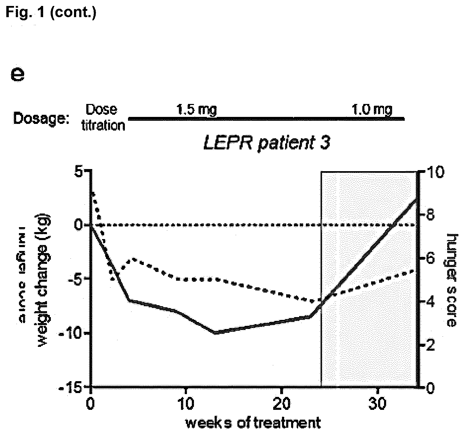

**Fig. 1****.** Weight course (colored line) and hunger-score (dotted grey line) after treatment start of two individuals with POMC deficiency (a,b) and three individuals with LEPR deficiency (c-e). The green area (a, b, d, e) represents off-drug periods or phases in which external factors (compromised compliance or changes of co-medication as hydrocortisone) led to increased hunger. In subject 2 with POMC deficiency a non-serious febrile infection made an increase of the hydrocortisone treatment necessary. Due to a misunderstanding, this planned temporary elevated dosage of hydrocortisone was not returned to her basal dose after a few days as planned; as a result, the hunger feelings started to increase followed by weight gain. After adaptation of the hydrocortisone dosage during her next visit the hunger feeling and weight decreased again. The green area for subject 3 with LEPR deficiency is described in the main body of the text.

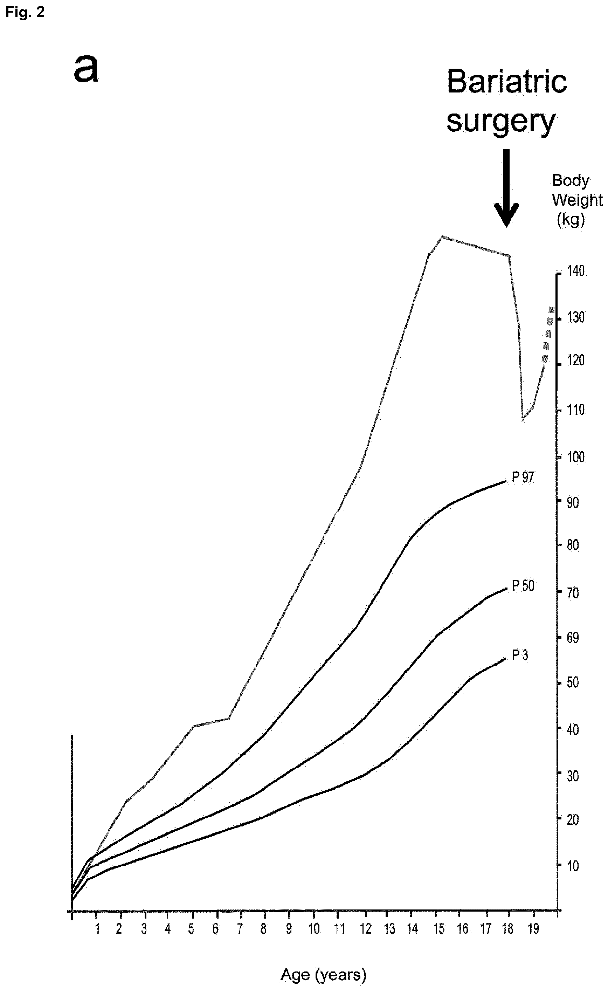

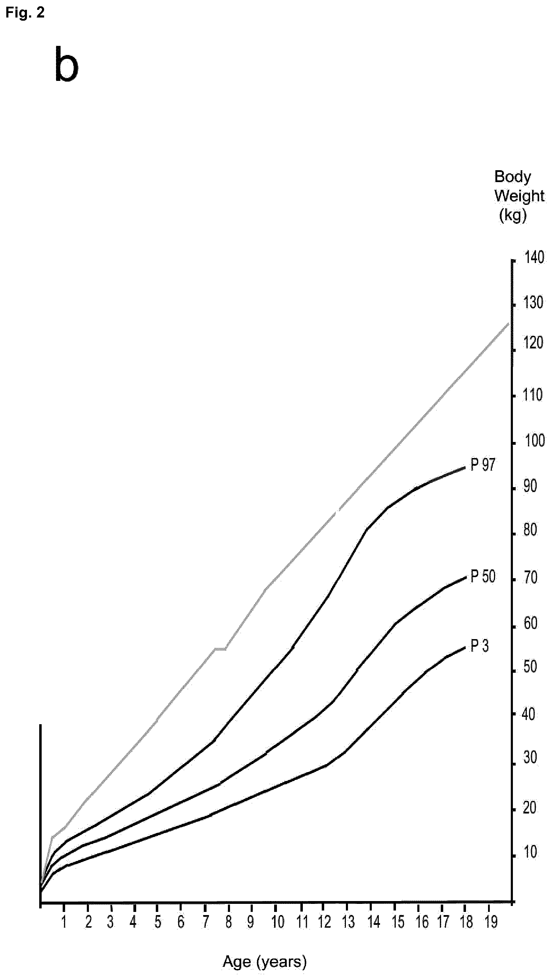

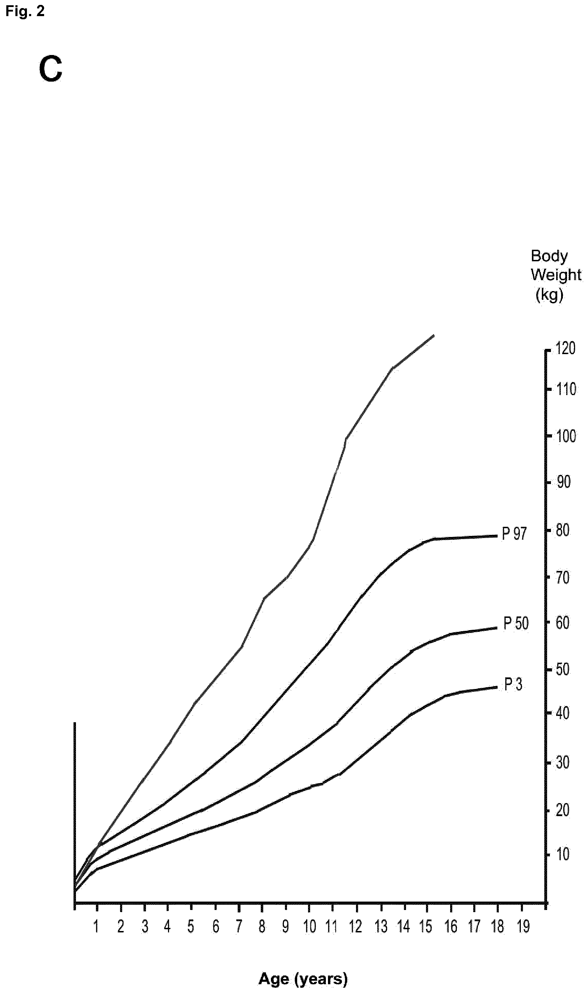

**Fig. 2****.** Weight course of the three included subjects with LEPR deficiency (a-c) before the study start. Standard percentiles were used²⁷. All of them had a history of early onset obesity and severe hunger and were not able - despite tremendous effort - to stabilize the body weight over a longer period of time. Subject 1 with LEPR deficiency (a) had a gastric banding operation, which led to a short interval of weight loss and subsequent weight regain. The dashed blue line visualized the weight gain months before the treatment start and is not adapted to the time scale of the y-axis. Additionally for subject 2 weight data are not available between 8 and 19 years of age.

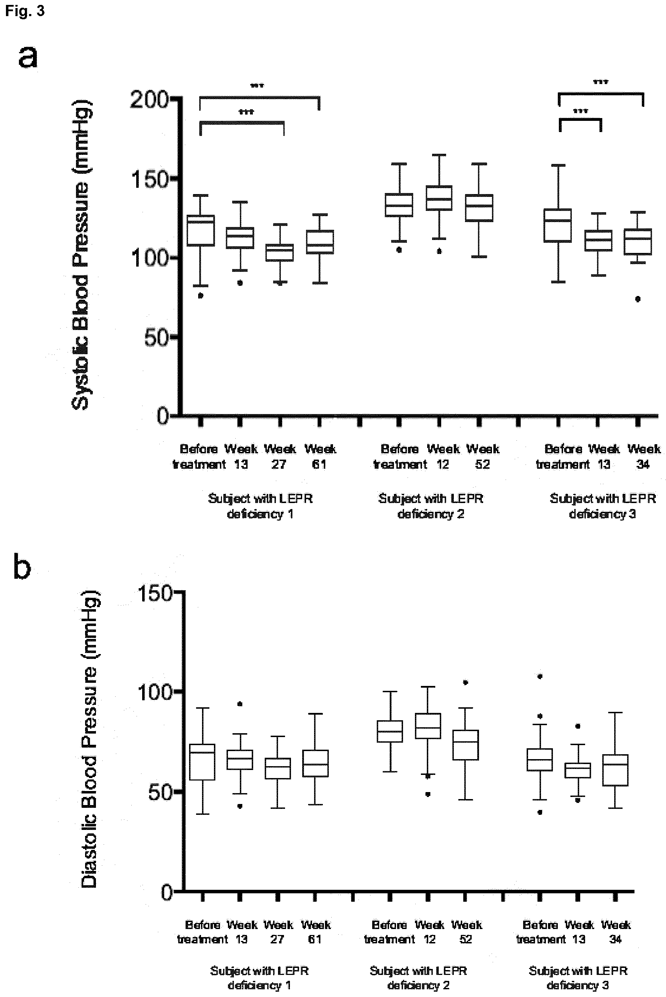

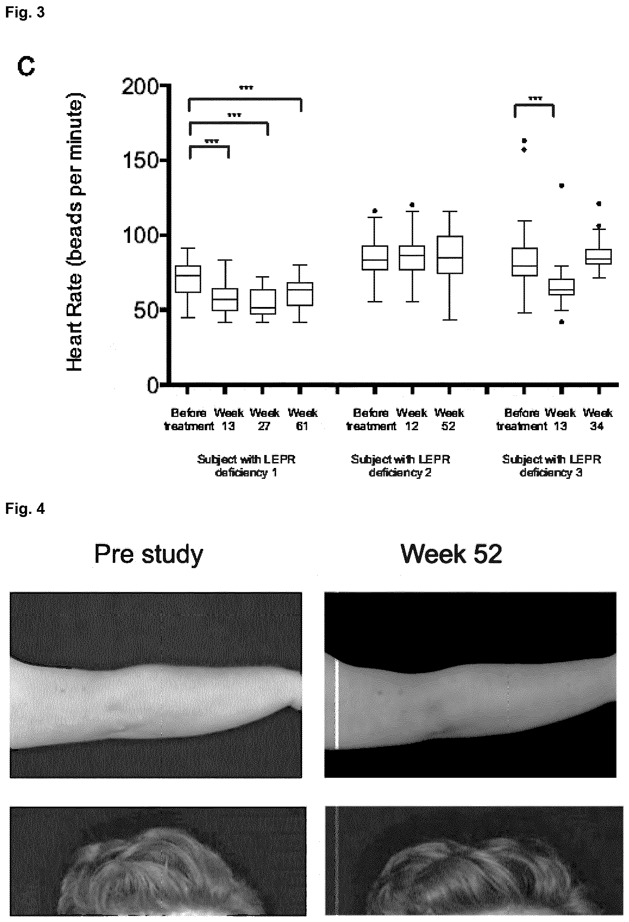

**Fig. 3****.** 24-hours ambulatory blood pressure monitoring before and during treatment with setmelanotide in three enrolled individuals with LEPR deficiency (a-c). Statistical analysis was performed using one-sided t-test analyzing "before treatment" data against each outlined week of treatment separately (*** = p < 0.001). Subject 1: before treatment n=67 individual measurements, week 13 n=68 individual measurements, week 27 n=54 individual measurements, week 61 n=51 individual measurements; subject 2: before treatment n=57 individual measurements, week 12 n=59 individual measurements, week 52 n=87 individual measurements; subject 3: before treatment n=54 individual measurements, week 13 n=33 individual measurements, week 34 n=22 individual measurements. In the box plots horizontal line inside each box indicates median and the top and bottom of the box indicate the interquartile range, The bars indicate the 5th and 95th percentiles and squares indicate outliers.

**Fig. 4****.** Examples of changes in skin color during the MC4R agonist treatment before and after 52 treatment weeks in individual 2 with LEPR deficiency. Changes in skin color are evident at week 52. Additionally, in subject 2 with LEPR deficiency the treatment led additionally to a change of the hair color from red to brown.

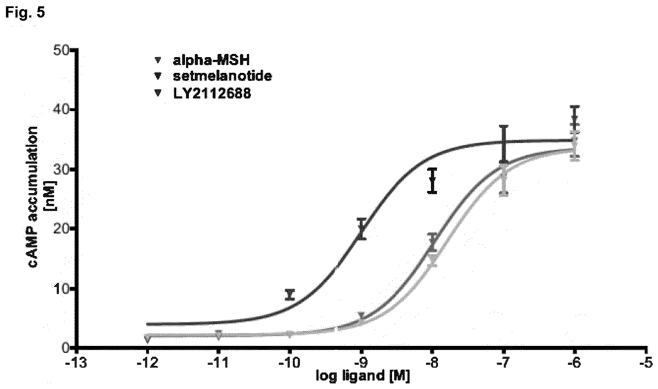

**Fig. 5****.** HEK293 cells were transiently transfected with MC4R and stimulated with indicated ligand concentrations. Results of seven independent experiments performed in triplicated were shown as raw data, data are mean ± s.e.m.. Statistical analysis was performed using a one-way ANOVA with Kruskal-Wallis test at a concentration of 10⁻⁸ M. Setmelanotide was tested against alpha-MSH and LY2112688: **** for alpha-MSH, and ** for LY2112688, ** p ≤ 0.01, **** p ≤ 0.0001.

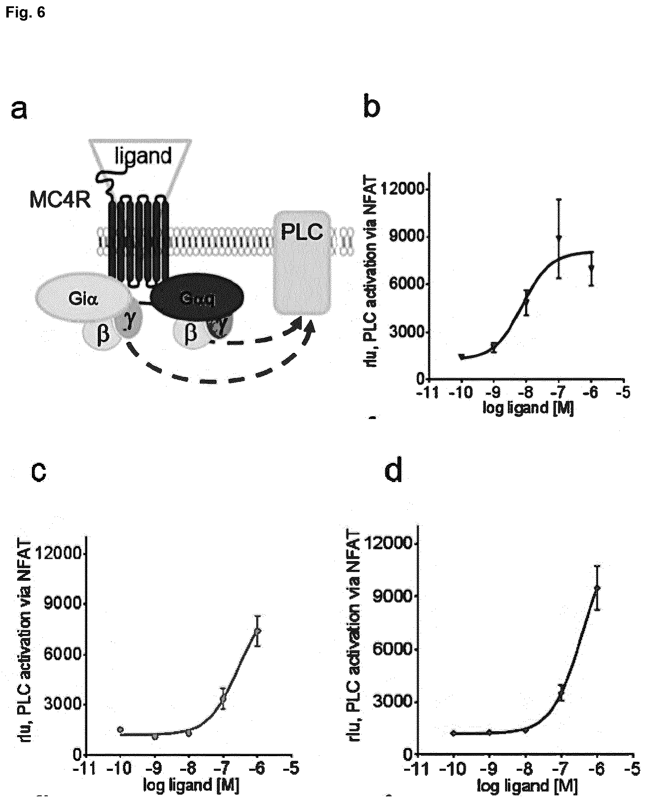

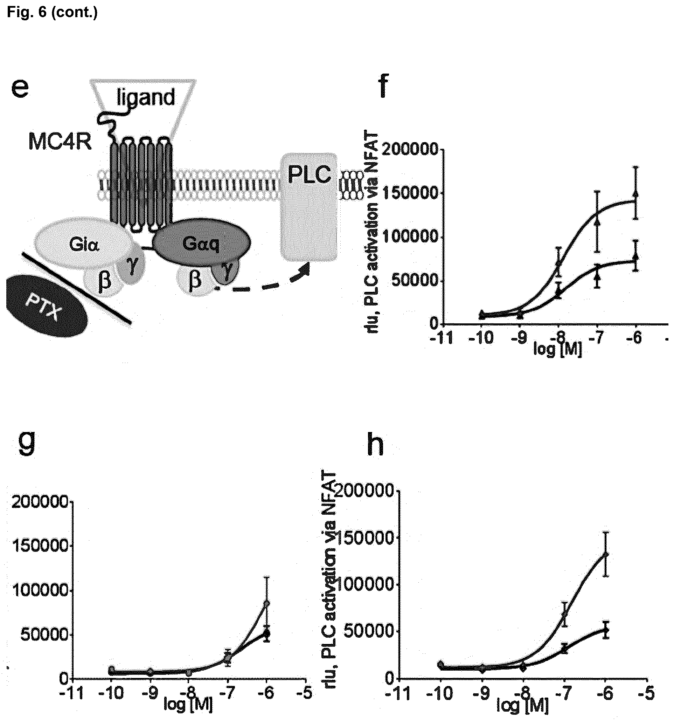

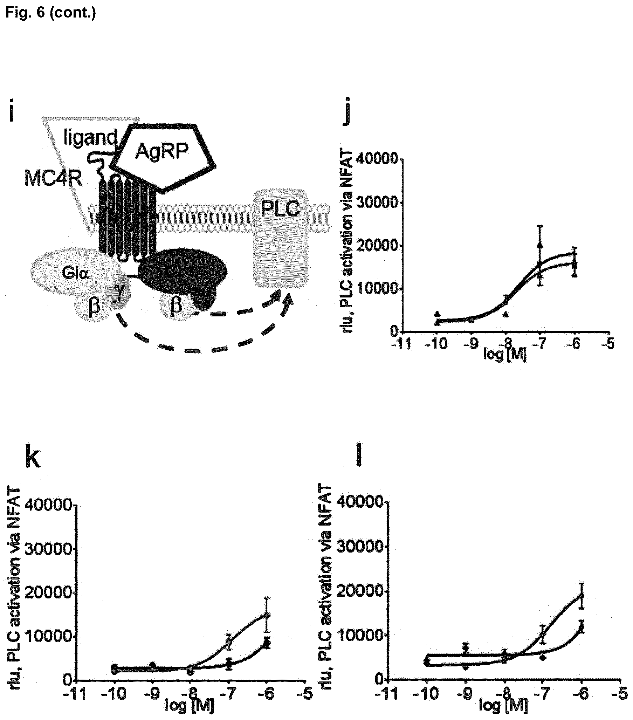

**Fig. 6****.** All functional data are raw data of four independent experiments performed in triplicates and are given as mean ± s.e.m.. *HEK293* cells were transiently transfected with MC4R and a reporter gene construct for determination of PLC activity in the absence (b-c) or presence of 50 ng/µl PTX (f-h) or 100 nM AgRP (j-l). Setmelanotide is indicated as red triangle, alpha-MSH as grey circle and LY2112688 as green diamond. (a): Cartoon representation illustrating phospholipase C (PLC) activation after stimulation of MC4R with different ligands. (b-d): Statistical analysis was performed using a one-way ANOVA with Kruskal-Wallis test at a concentration of 10⁻⁸M. Setmelanotide was tested against alpha-MSH (p ≤ 0.001) and LY2112688 (p ≤ 0.01). (e): Cartoon representation illustrating the effect of PTX on NFAT activation. In each graph the PTX effect on ligands is indicated in black by using the same symbols as the ligand (f-h). Statistical analysis of PTX-treated versus untreated was done by unpaired t-test with Welsh correction. (f): Setmelanotide: not significant (g): alpha-MSH: not significant. (h): LY2112688: significant (p ≤ 0.01). (i): Cartoon representation illustrating the effect of AgRP on displacing MC4R ligands. The effect of AgRP co-stimulation of each ligand is indicated in black by using the same symbol as the ligand (j-l). Statistical analysis was done for ligand stimulation versus ligand/AgRP co-stimulation. (j) Setmelanotide is not displaced by AgRP, n.s., (k): minor displacement of alpha-MSH by AgRP, n.s., (I): displacement of LY2112688 by AgRP, p ≤ 0.05. n.s.: not significant. Differences in raw activation of PLC are due to the fact that raw data are shown and rlu is dependent on several issues such as surrounding temperature, lot number and age of the substrate. In control experiments (see Fig. 6) with mock transfected cells, rlu activity never exceeded more than 5% of the activity observed in main experiments with the different ligands.

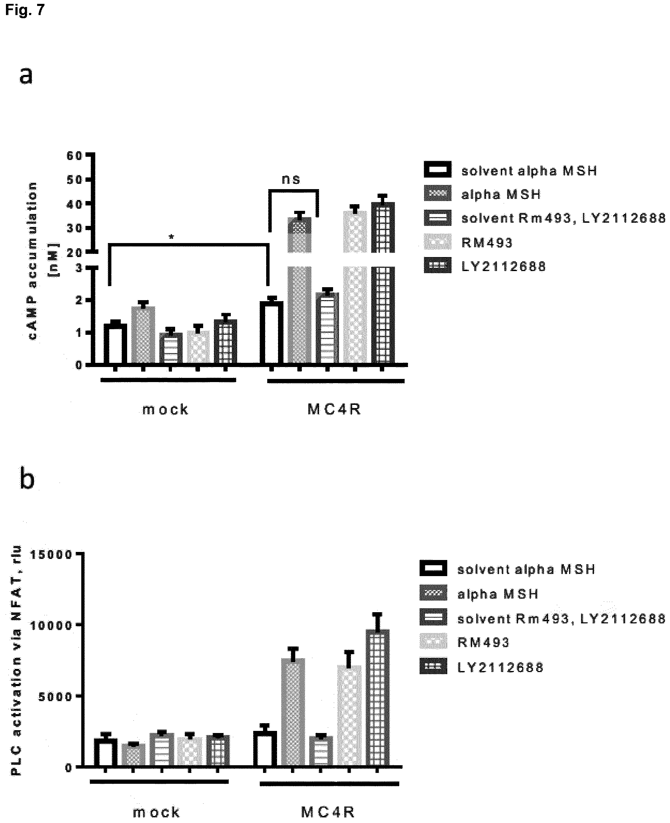

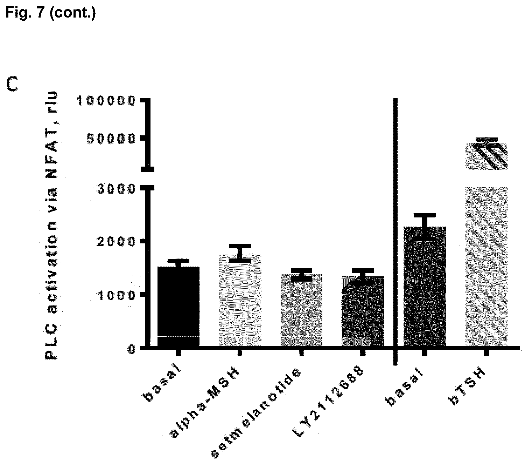

**Fig. 7****.** HEK293 cells were transfected with empty vector (mock) and MC4R (a, b) or MC3R and TSHR (c). The result of four independent experiments performed in triplicates is shown as raw data, data represents mean ± s.e.m.. (a) mock transfected cells were stimulated with the solvent for alpha-MSH (PBS with 0.001% BSA) and the solvent for setmelanotide and LY2112688 (PBS with 0.1% DMSO). Statistical analysis was performed using a one-way ANOVA with Kruskal-Wallis test and reveled no statistical difference. MC4R transfected cells were stimulated with alpha-MSH (1 µM) and the solvent for ligands. Basal activity of MC4R tested against mock transfection by unpaired t-test with Welsh correction and solvent effects at MC4R were tested against MC4R under basal condition by one-way ANOVA with Kruskal-Wallis test and revealed no statistical significance. (b) Identical set of experiments and statistical tests for activation for PLC as is indicated in (a). (c) HEK293 cells were cotransfected with MC3R or TSHR and a reporter gene for NFAT and PLC activation was tested. TSHR was stimulated with 100 mU/ml bTSH and served as control. MC3R was stimulated with indicated ligands (1 µM). Statistical significance was tested by one-way ANOVA with Kruskal-Wallis test and revealed no difference.

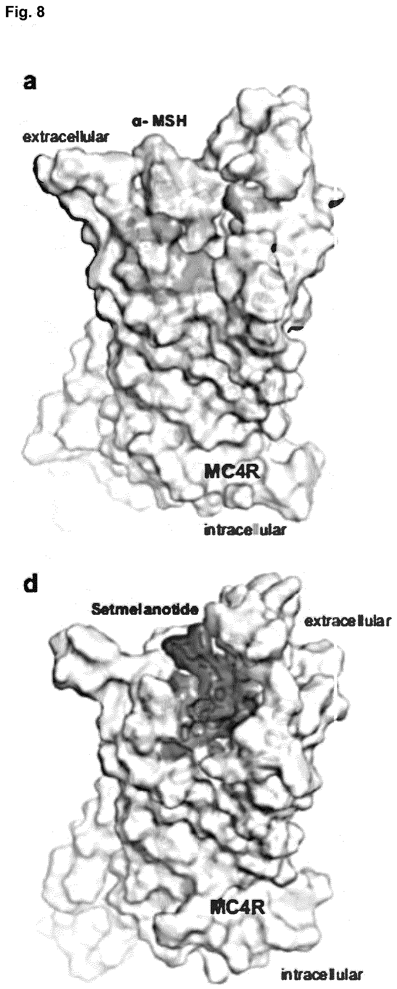

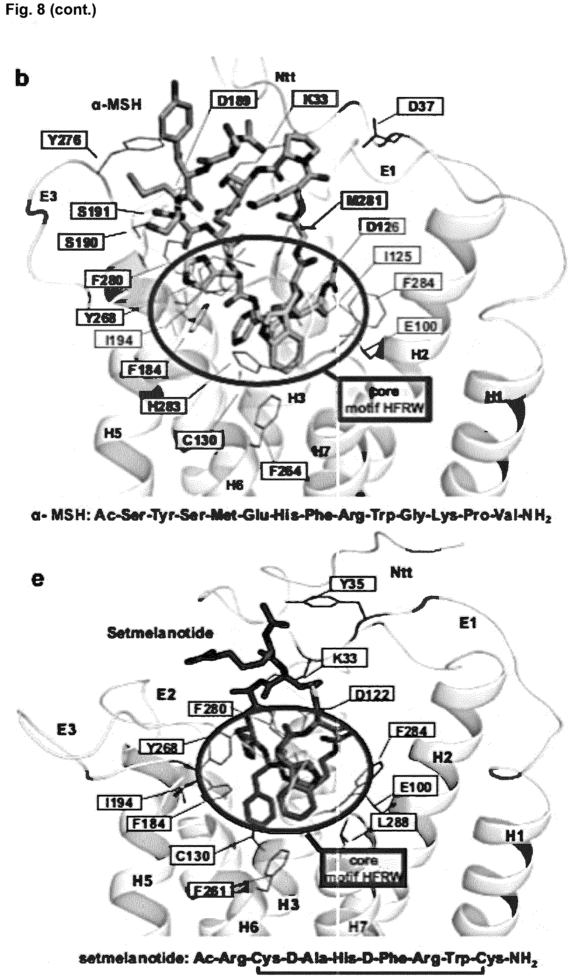

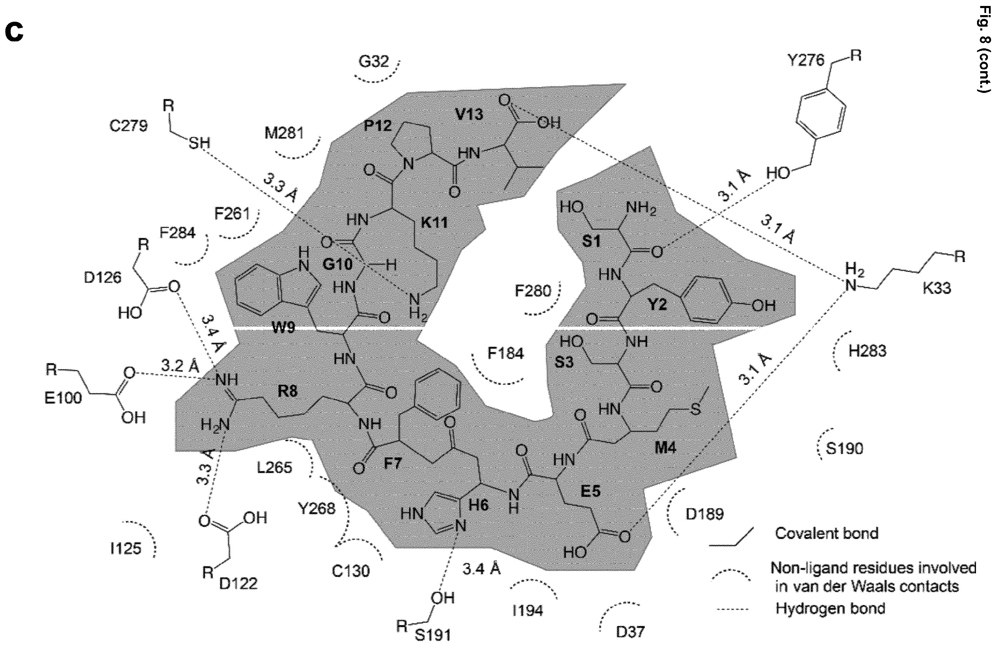

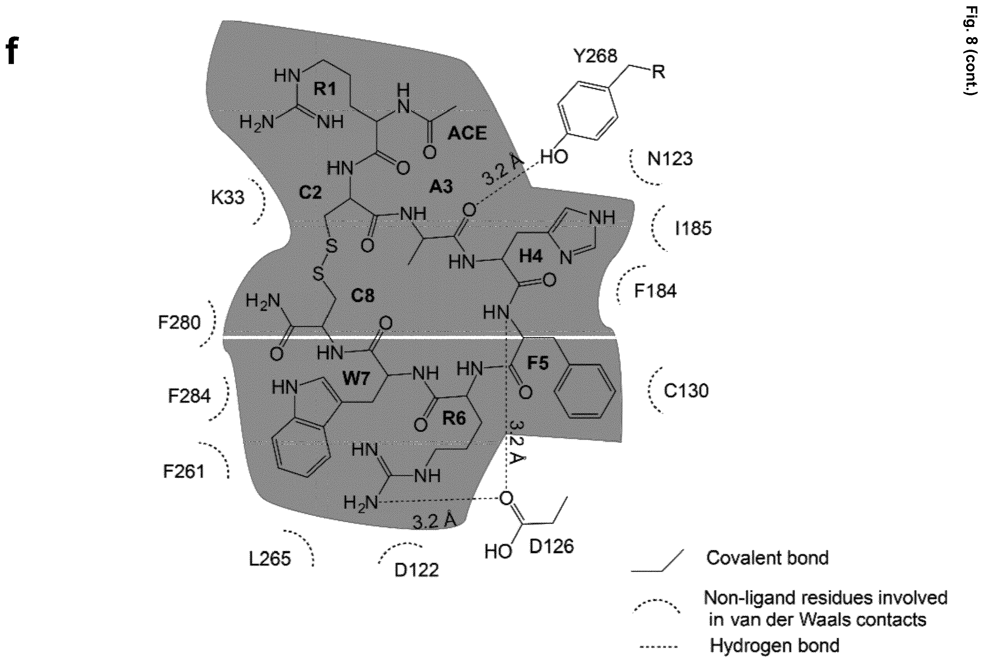

**Fig. 8****.** (a) The human hMC4R/alpha-MSH complex model (in surface representation) reveals that the peptidic agonist binds into a cleft between the extracellular loops (E1-3) and specific transmembrane helices (H). Amino acids covering the ligand binding pocket are colored pale-green. (b) Approximately twenty hMC4R amino acids (green lines) in the transmembrane region and in the E2 or E3 constituting the ligand binding pocket. Importantly, in alpha-MSH (amino acid sequence of alpha-MSH below the model-figure a central amino acid motif 6HFRW9 is involved in ligand recognition and induction of ligand effects²⁸. This specific ligand motif is located between the helices and their extracellular ends. (c) The detailed interaction map for the MC4R/alpha-MSH complex shows a schematic overview of supposed interactions. (d) The surface representation of the MC4R/setmelanotide complex model highlights the general localization of this ligand bound to the hMC4R. Amino acids covering the supposed setmelanotide binding pocket are colored green. (e) The cyclic peptide agonist setmelanotide (amino acid sequence below the model-figure) interacts, comparable to alpha-MSH, with the MC4R by a supposed salt bridge between the ligand Arg6 in the central core and the negatively charged receptor residues Glu100 (TMH2), Asp122 and Asp126 (TMH3) in MC4R. (f) The interaction map between setmelanotide and MC4R shows a different interaction pattern as supposed for alpha-MSH at MC4R, however, there is partial overlap in participating MC4R amino acids such as Asp122, Glu100 or Phe284. Potential hydrogen bonds were analyzed using HBPLUS²⁸ as implemented in the program LIGPLOT+ 1.45 ²⁹. Residues with distances less than 3.9 Å were considered to be in van der Waals contact.

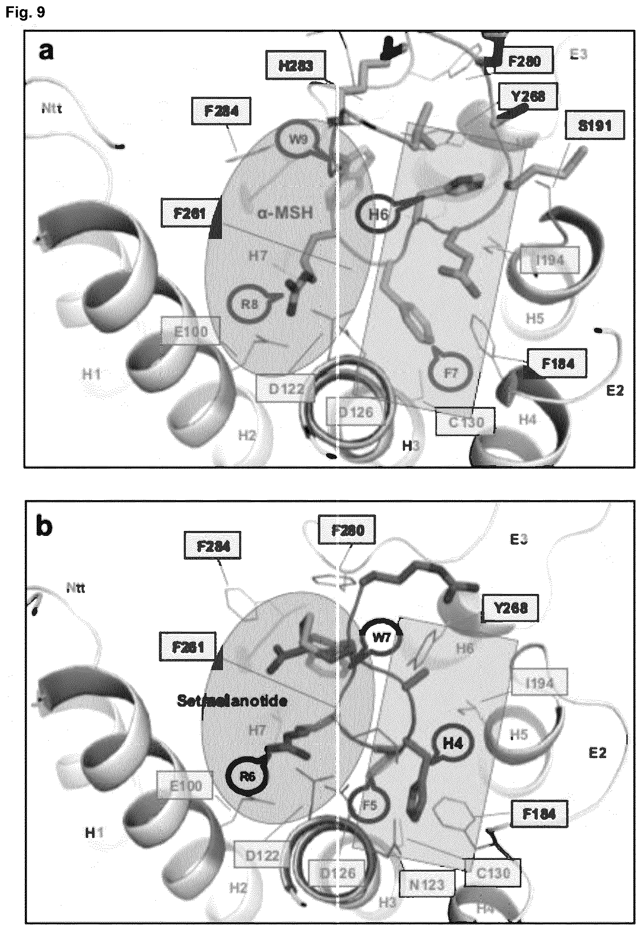

**Fig. 9****.** The putative binding modes of (a) alpha-MSH and (b) setmelanotide at hMC4R reveal similarities and differences between both ligands (shown as Cα-backbone cartoon representation and clipped models). Of note, in contrast to α-MSH, setmelanotide is a cyclic peptide with a disulfide bridge between two cysteines. A specific emphasis relays on the interactions and localization of the conserved ligand motif HFRW. The Arg6 (setmelanotide) and Arg8 (α-MSH) interact with negativelycharged side chains of Glu100 (TMH2) and Asp122/Asp126 in H3 of the receptor. Moreover, Trp9 in alpha-MSH and corresponding Trp7 in setmelanotide are both embedded by aromatic interactions with Phe261 in H6 and Phe284 in H7, in close proximity also to a activation related receptor residues^{24,25} Tyr268 in H6 and His283 in the H7-E3 transition. In the MC4R/ setmelanotide complex additionally Phe280 participates in the interaction with Trp7. However, specifically the histidine (shown with red label) of the ligands is oriented differently in both docking poses. The histidine in setmelanotide is located between Asn123 (H3) and Phe184 (H4), but in the α-MSH/MC4R complex the corresponding His6 is directed between Tyr268 and Ser191 of the receptor. Moreover, alpha-MSH Phe7 interacts with Cys130 in H3 and Phe184 in H4, whereby the corresponding Phe5 of setmelanotide is oriented towards H6 and interacts additionally with Phe261. It can be concluded that (i) both ligands potentially share specific interactions of the highly conserved motif HFRW with MC4R (shown as red filled translucent circle), on the other hand (ii) the corresponding ligand histidines and phenylalanines in this motif are different in detailed interactions with side chains in receptor helices 4-6 (shown as blue filled translucent rectangle).

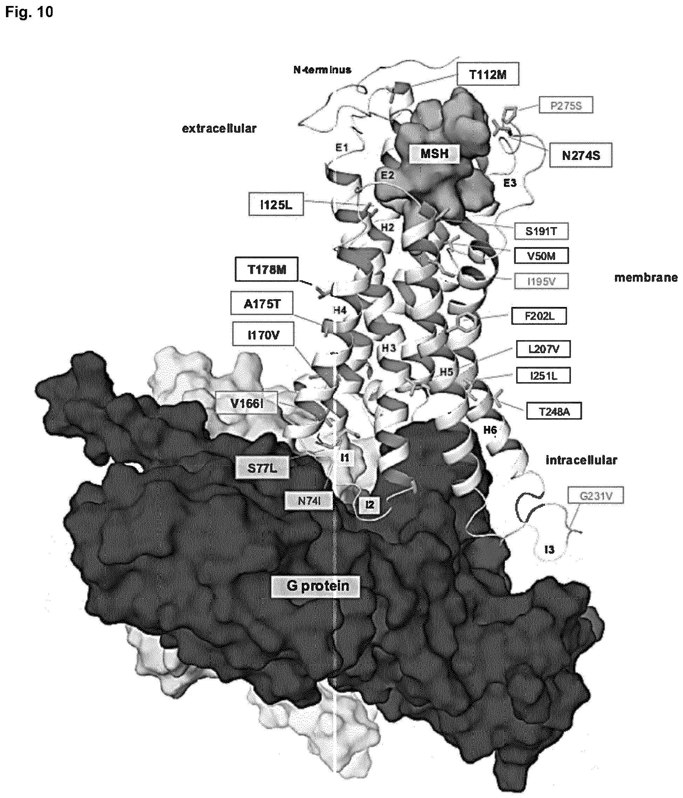

**Fig. 10****.** Wild-type amino acids of in this study experimentally tested MC4R mutants concerning their Gaq and Gas signaling properties Table 5) are highlighted at a MC4R/ligand complex model extended by a heterotrimeric Gprotein molecule (surface representation, α-subunit, β-subunit, γ-subunit). These positions are located in helices (H) H2, H3 and are cumulated in H4. Moreover, we also exemplarily show further wild-type residues which were reported to be identified in obese patients and characterized in vitro as "like wild-type" mutations for cAMP accumulation. Of note, from the around 160 reported naturally occurring hMC4R single side chain variants approximately 30% were characterized so far "like wild-type" in Gαs mediated signaling.

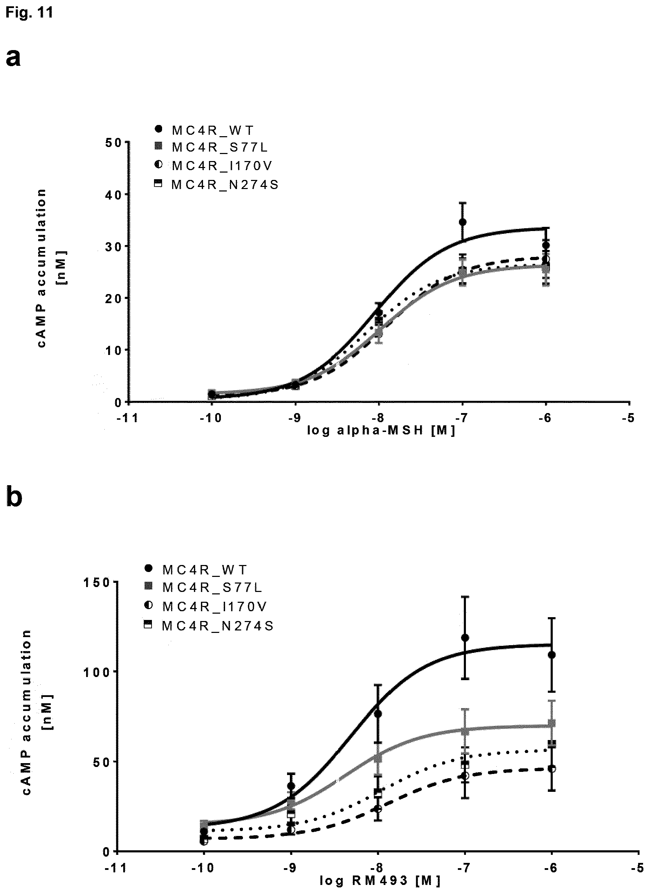

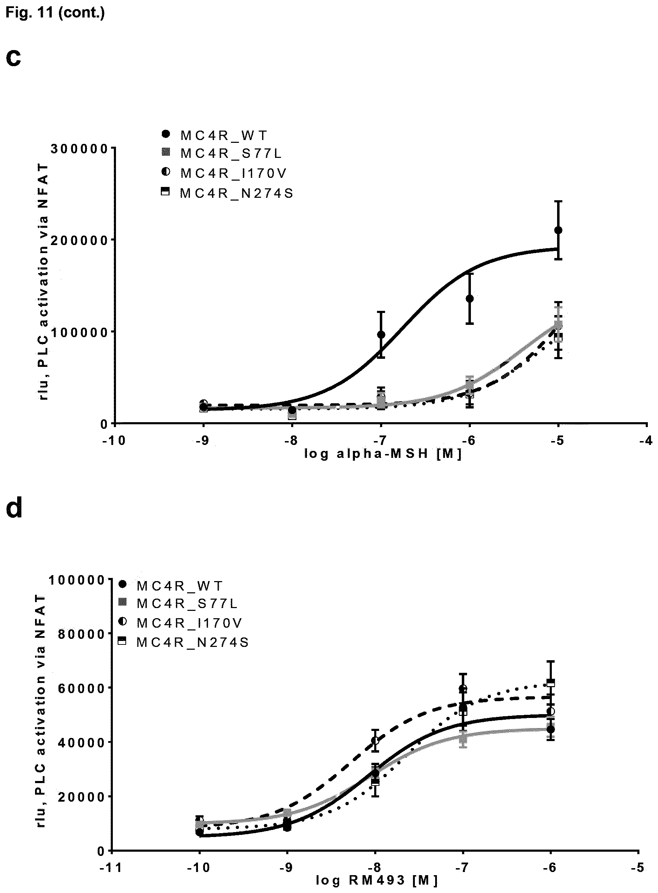

**Fig. 11****.** HEK293 cells were transfected with MC4R wild-type and indicated MC4R variants and stimulated with indicated ligand concentrations. (a, b) cAMP accumulation after stimulation with alpha-MSH or setmelanotide. (c, d) MC4R mutants were tested for PLC signaling after co-transfection with NFAT reporter gene. Stimulation was performed with alpha-MSH (c) and setmelanotide (d). Raw data of four independent experiments are shown, data are mean ± s.e.m..

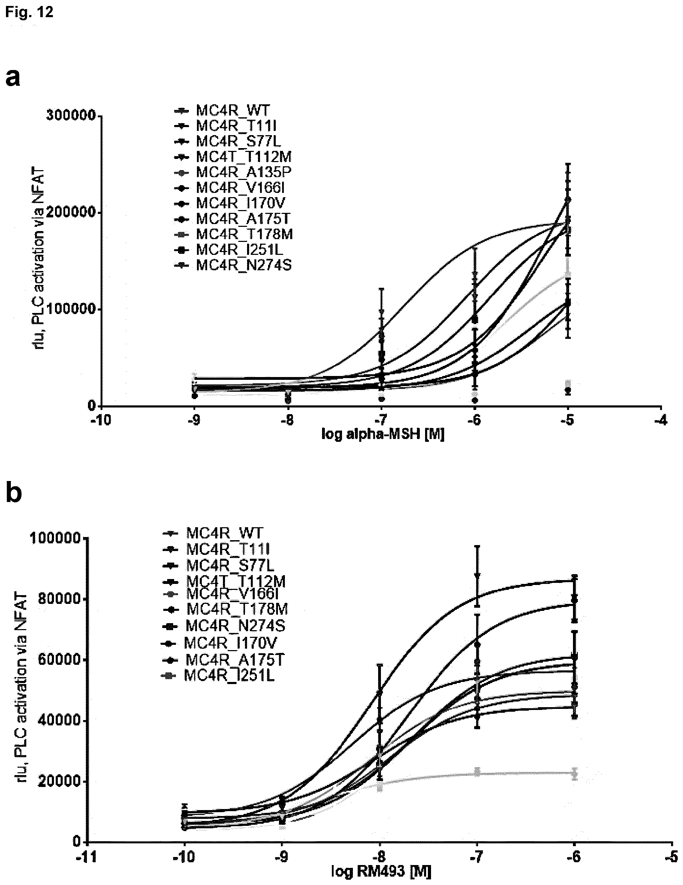

**Fig. 12****.** HEK293 cells were transfected with MC4R wild-type and indicated MC4R variants and stimulated with indicated ligand concentrations. All MC4R mutants were tested for PLC signaling after co-transfection with NFAT reporter gene. Stimulation was performed with alpha-MSH (a) and setmelanotide (b). Raw data of four independent experiments are shown, data are mean ± s.e.m..

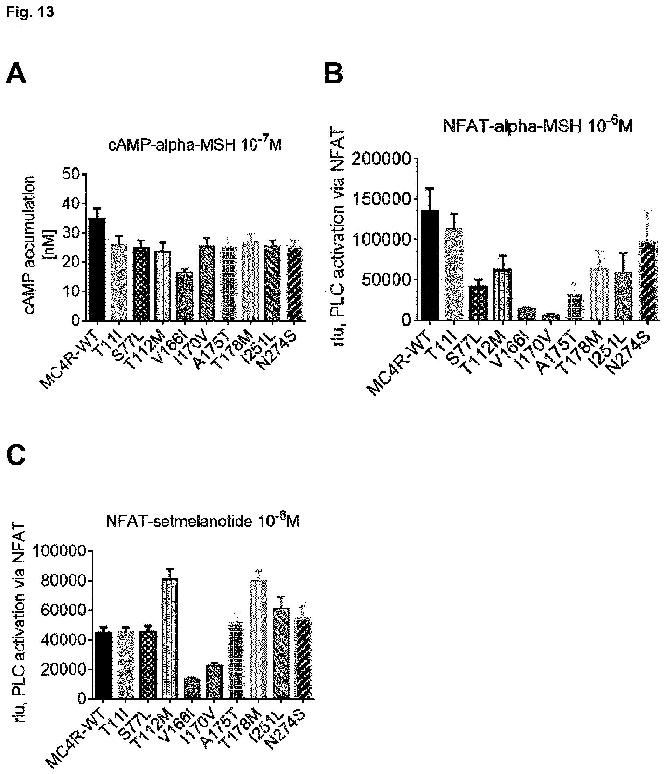

**Fig. 13****.** MC4R deficiency associated with impaired NFAT. (A) cAMP accumulation after alpha-MSH stimulation, all variants perform essentially as MC4R-WT. (B) NFAT signaling after alpha-MSH stimulation, essentially nearly all variants show a loss of function. (C) NFAT signaling after setmelanotide stimulation, essentially nearly all variants show a rescue in NFAT signaling. The assays for A, B and C were performed independently, raw data of four independent experiments performed in triplicates are shown.

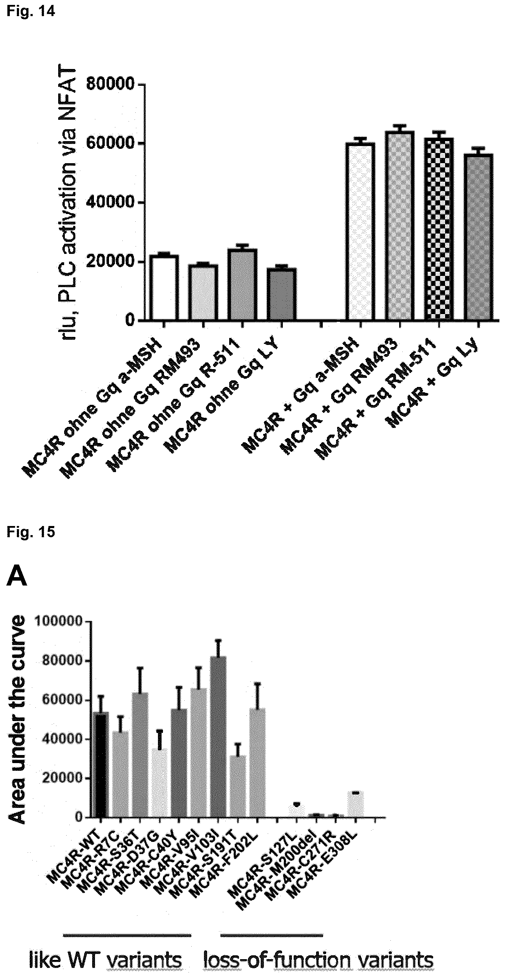

**Fig. 14****.** Investigation of NFAT signaling in mouse embryonic fibroblasts (MEF) deficient in Gq (MEF Dq). MEF were transfected with MC4R and the NFAT reporter in the absence and presence of Gq and NFAT signaling was measured after challenge with alpha-MSH, setmelanotide, RM-511 and Ly (10-6M). One out of four independent experiments is shown.

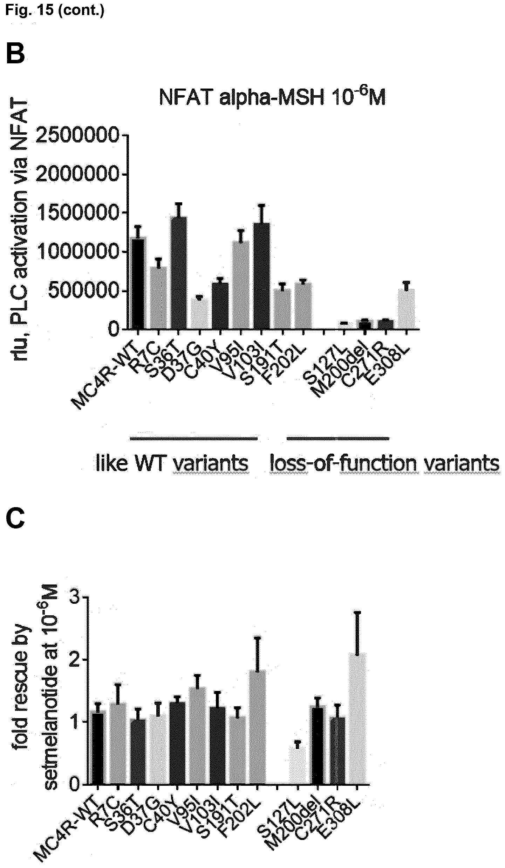

**Fig. 15****.** Test of further MC4R variants. Additional experiments were conducted with MC4R variants R7C, S36T, D37G, C40Y, V95I, V103I, S127L, S191T, M200del, F202L, C271R and E308L. For testing Gs signaling, dynamic formation of cAMP was measured after alpha-MSH challenge. Variants classified as Gs like wild-type as well as several Gs loss-of-function variants were included. (A) GloSensor data of 4 independent experiments performed in triplicates are given as area under the curve. This data represents cAMP accumulation after alpha-MSH treatment. (B) NFAT signaling was assessed after alpha-MSH (B) and setmelanotide (C) challenge was performed in one assay set, 4 times independently. The fold rescue of each variant after setmelanotide challenge was calculated (in C).

**Fig. 16****.** Schematic illustration of the classification of melanocortin-4 receptor (MC4R) variants. The main signalling pathway of wildtype MC4R (dark grey folded protein schematic) is most likely via Gs/adenylyl cyclase activation (labelled Gsα), thereby enhancing cAMP. In addition, other pathways can be activated following endogenous ligand stimulation. Unaffected signalling cascades are marked in dark grey (Gs labelled Gsα; non-Gs signaling labelled as such); impaired cascades are marked in light grey. Class 1: MC4R variant (variant indicated as white dot) signalling is identical to the wild-type MC4R in all signalling pathways. Class 2: Gs signalling of MC4R variants is comparable with the wild-type MC4R; only signalling of other, non-Gs pathways is reduced (light grey). Class 3: Gs signalling and other signalling pathways of MC4R variants are decreased. Class 4: Only Gs signalling of MC4R variants is reduced; non-Gs signalling pathways are unaffected.

### EXAMPLES

The invention is further described by the following examples. These are not intended to limit the scope of the invention, but represent preferred embodiments of aspects of the invention provided for greater illustration of the invention described herein.

### Materials and methods of the examples

### Study protocol

The investigator-initiated phase 2 trial (sponsor: Charité Universitätsmedizin Berlin, EudraCT No. 2014-002392-28, clinicaltrials.gov No. NCT02507492) started with a baseline phase in which a physical examination, measurement of height- and body weight, blood tests (including oral glucose tolerance test (OGTT), GnRH test, measurement of full blood count, serum lipids, liver and kidney function parameter, leptin, IGF-1), analysis of body composition (BodPod) and energy expenditure (indirect calorimetry, CareFusion, VMAX^{®} Encore system), careful dermatological examination and documentation of the skin and psychological evaluation was performed. This phase was followed by a dose-titration phase starting with a dosage of 0.5-mg setmelanotide s.c./q.d. Every two weeks the dosage was increased for 0.5 mg/q.d. depending on the weight course and tolerability accompanied by a careful monitoring of vital parameters including blood pressure. After 12-13 weeks following the treatment start study subjects could enter the extension phase if a reduction of the body weight was achieved of more than 10 kg. Within this phase the study subjects were regularly seen by the study doctor, at least every 3 months, and blood tests and analysis of vital signs and safety parameters were performed.

### Functional characterization of MC4R ligands

Characterization of setmelanotide, first generation MSH analog LY2112688 and alpha-MSH was performed in *HEK293* cells. *HEK293* cells were authenticated by SNP analysis and analysis of mycoplasm infection was performed every four weeks. *HEK293* cells for cAMP accumulation assays and PLC activation (NFAT-luc assays) were cultured in L-glutamine containing MEM Earle's media supplemented with 5 % FBS (Biochrom AG, Berlin, Germany) and 1x non-essential amino acids (Biochrom AG, Berlin, Germany) at 37 °C with 5 % CO₂.

For cAMP and NFAT-luc measurements, 1.5 *10⁴ *HEK293* cells per well were seeded in poly-L-lysine-coated (Biochrom AG, Berlin, Germany) 96-well plates (15,000 cells/well). For functional characterization of MC4R variants all variants were generated by standard mutagenesis techniques and verified by Sanger sequencing. Transient transfection of *HEK293* cells with *MC4R* plasmid-DNA or co-transfection with *NFAT* reporter gene (0.45 ng/µl of each plasmid) was performed 24 hours after seeding in supplement-free advanced MEM (Life technologies, Carlsbad, CA), using Metafectene (Biontex, Munich, Germany) according to the manufactures protocol. *CHO-K1* cells stably expressing MC4R (Discoverx Corp, Fremont CA, USA; Product code 05-0050E2 for cAMP Hunter^{™} eXpress cells, tested for mycoplasma every four weeks) were utilized to study the antagonism of setmelanotide and LY2112688 response by AGRP-83-132-amide (Anaspec (Fremont, CA). These cells were plated in a 96-well plate for the assay as per manufacturer's instructions.

### Measurement of cAMP accumulation

Gαs signaling were determined by measuring cAMP accumulation. Forty-eight hours following transfection in *HEK293* cells, stimulation was conducted with compounds diluted in a HEPES-buffered solution containing 1 mM 3-isobutyl-1-methylxanthine (IBMX, Sigma Aldrich, St. Louis, MO) to inhibit cAMP degradation by phosphodiesterases. Cells were incubated for 40 minutes with setmelanotide, LY2112688 and alpha-MSH in concentrations ranging from10⁻⁶ M to 10⁻¹² M in order to stimulate adenylyl cyclase. Substance incubation was performed in triplicates and at 37 °C with 5 % CO₂ and was stopped by aspirating the medium. Cells were then lysed at 4 °C on a shaking platform with lysis buffer containing 5 nM HEPES, 0.1% BSA, 0.3% Tween20 and 1 mM IBMX. Intracellular cAMP accumulation was determined by a competitive immunoassay based on the AlphaScreen technology (Perkin-Elmer Life Science, Boston, MA) as previously described¹⁸.

### Measurement of PLC by luciferase reporter gene assay

PLC activation was determined by NFAT-luc assays (Promega, Fitchburg, WI). This method requires the co-transfection of equal amounts (0.45 ng/µL DNA of each plasmid) of MC4R and the reporter constructs pGL4.30[luc2P/NFAT-RE/Hygro] (Promega, Fitchburg, WI) containing a response element and firefly luciferase gene under the control of NFAT. Two days post-transfection, cells were incubated for six hours with ligands in supplement-free MEM at 37 °C with 5 % CO₂. In case of determination the effect of G activation of Gi/o, blocking with PTX (50 ng/ml) was done 18 hours before the experiment. In case of AgRP interfering with PLC activation, co-stimulation of ligands in the presence of 100 nM AgRP was performed. The reaction was terminated by aspirating of the media. Cells were lysed for 15 minutes on a shaking platform at room temperature using 1x passive lysis buffer (Promega, Fitchburg, WI). Measurement was conducted with automatic luciferase substrate injection of 40 µL in a black 96-well plate using a Berthold Microplate Reader (Berthold Technologies GmbH & Co KG, Bad Wildbad, Germany).

### Melanocortin-4 receptor structure homology modeling and docking of alpha-MSH and setmelanotide

The hMC4R modeling was performed in its putative active state conformation. The hMC4R is characterized by several specific properties concerning the amino acid composition and related structural features, in particular: (*i*) a short second extracellular loop 2 (E2, constituted by four amino acids), *(ii)* a missing cysteine disulfide bridge between the E2 and transmembrane helix 3 (TMH3) that is highly conserved among GPCRs, *(iii)* a regular α-helical conformation of TMH5 because of a methionine instead of a proline that is conserved in class A GPCRs and induces a helical kink and bulge, *(iv)* a disulfide bridge in the third extracellular loop (E3)¹⁹.

Our hMC4R homology model was built by using the solved crystal structure of the β2-adrenergic receptor (ADRB2)/Gs complex (PDB entry 3SN6²⁰) as a template, because this receptor structure represents an active state conformation which complements to our purpose of docking agonistic peptides into the hMC4R model.

General modifications for template preparation were deletion of the extracellularly fused T4 lysozyme, removal of the ADRB2-ligand and of the interacting Gs molecule. Moreover, the ECL2 of the ADRB2 template was deleted, because of differences in length and amino acid composition compared to the sequence of the hMC4R E2. Gaps of missing residues in the loops (e.g. in I3) of the template structure were closed manually. Moreover, the upper part of the kinked helix 5 was removed and substituted by a regular α-helix, because crystal structures of few GPCRs like the S1PR1 (PDB entry 3V2Y²¹) have shown a regular α-helical conformation without a proline in TMH5. This secondary structure must be assumed also for the hMC4R with a methionine at this corresponding position. All structural modifications were performed with the software Sybyl X2.0 (Certara, NJ, US). The AMBER F99 force field was used for energy minimization and dynamic simulations.

Amino acids of this prepared receptor-template were substituted with residues of the hMC4R, followed by side chain minimization with constraint backbone atoms of the transmembrane helices until converging at a termination gradient of 0.1 kcal/mol*Å. The constraints were finally released in a second minimization step until converging at a termination gradient of 0.05 kcal/mol*Å. This first initial model was further refined by molecular dynamic simulations (30 0K, 2 ns) and energetic minimizations of the side chain orientations with constrained backbone atoms of helical parts until converging at a termination gradient of 0.1 kcal/mol*Å. The resulting model was energetically minimized without any constraint.

### Ligand models

The cyclic ligand setmelanotide was modeled based on the structurally determined oxytocin peptide (PDB entry 2MGO²²), which is characterized by a disulfide bridge between two cysteines as also assumed for setmelanotide. Missing residues were inserted or added manually, setmelanotide specific residues were substituted, and the N- and C-terminal specific groups added. For alpha-MSH neither direct structural information nor determined structures of a homologous peptides are available. For this reason the oxytocin structure was also used as a fragmental template for the central part of alpha-MSH (sequence MEHFRWG). This procedure was used in accordance to the purpose to start the molecule dynamics based docking with a comparable structural conformation of the central HFRW motif in MSH (without a Cys-Cys disulfide bridge) and setmelanotide (with a Cys-Cys disulfide bridge). Missing residues at the alpha-MSH N- and C-terminus were added manually. The ligand structures were energetically minimized and used for docking into the hMC4R model.

### Ligand/receptor complex-assembling

Computational docking of the two ligands into MC4R was performed under consideration of an already known and evidenced receptor/ligand interaction pair. One of the most prominent interaction is experimentally evidenced between MSH amino acid Arg8 (in setmelanotide Arg6) and receptor amino acids Asp122 and Asp126 of TMH3 (reviewed in²³). These complementary charged amino acids are essential for ligand/receptor recognition and ligand activity. Therefore, we used this supposed interaction as a functional structural constraint. We manually placed both ligands above the extracellular loops of the MC4R model and initiated molecular dynamic simulations (300 K, 3 ns) with a defined distant constraint of 2 Å between the side chains of MSH Arg8 (or Arg6 in setmelanotide, respectively) and MC4R Asp126. All backbone atoms of the receptor helices were constrained. The resulting models were energetically minimized, followed by a second dynamic simulation (2 ns) without any distance constraint. The resulting complex models were energetically minimized without any constraint.

### Full receptor/ligand/complex

The resulting hMC4R alpha-MSH complex was superimposed with the initial structural receptor template from the ADRB2/Gs complex (PDB entry 3SN6²⁰). The heterotrimeric G-protein was substituted into the hMC4R complex model. Dynamic simulations (300 K, 2 ns) of the side chains and loop structures were used to optimize interactions and intra-molecular distances, whereby the backbone atoms of the receptor helices were constrained. The resulting model was energetically minimized without any structural constraint.

### Data presentation and Statistical analysis

In most cases, we show results as raw data of pooled experiments performed in triplicated. The number of independent experiments is given in each figure legend. At minimum four experiments performed in triplicates were done. Data analysis was performed using GraphPad prism 6 software. Because raw data are shown the measurement of cAMP in nM or PLC in rlu may different between different sets experiments. The reason for this are e.g. changing room temperature and different charges of kits. Statistical analyses were performed using GraphPad Prism 6.0. The kind of statistical test in given in the figure legends. In general, we use a one-way ANOVA with Kruskal-Wallis test or unpaired t-test with Welsh correction as we do not expect normal distribution of values. In each figure legend it is indicated which data are tested against which data. Values were only excluded if they were identified as outliers based on a Grubbs test. Statistical significance was accepted at P< 0.05. Data are reported as mean ± s.e.m.. For Schild's analysis agonist concentration response curves were fitted using GraphPad Prism 6 using the Gaddum/Schild EC50 shift to drive pA2 and Schild slope directly from the dose response using global non-linear regression.

### Results of the Examples

### Example 1: Body weight course and hunger-score during Setmelanotide treatment.

Individuals with POMC deficiency have been treated with setmelanotide for more than 2 years, resulting in profound reductions of hyperphagia and body-weight, without signs of serious adverse side effects (Fig. 1 a,b). We hypothesized that impaired activation of POMC neurons due to LEPR signaling deficiencies, based on *LEPR* gene mutations, might similarly contribute to a lack of MSH signaling. Thus MC4R agonist supplementation therapy might be of therapeutic benefit.

In previous studies we observed, that intraperitoneal injections of setmelanotide in leptin-receptor-deficient (*db*/*db*) mice potently reduced appetite when compared to wild-type mice (data not shown). This data highlighted a likely MSH deficiency due to the LEPR defects. We tested whether setmelanotide treatment of individuals with LEPR deficiency might similarly result in profound reductions of hunger and body-weight. Three individuals with confirmed *LEPR* homozygous loss-of-function mutations were enrolled in an investigator-initiated phase 2 trial (EudraCT number: 2014-002392-28; ClinicalTrials.gov number: NCT02507492). The subjects and their relatives gave informed consent and the study was performed according to the declaration of Helsinki (see Materials and Methods).

Subject 1 is a 23 years old male from France in which a homozygous c.2357T>C (p.L786P) *LEPR* mutation was identified. Despite intensive efforts at life style intervention weight gain persisted and this patient underwent bariatric surgery (gastric-banding-operation) at age 18. Although following surgery the patient lost 36 kg in 6 months, he started to regain weight with persistent hyperphagia (regain of 20 kg in the subsequent 24 months; Fig. 2a). At the time this subject was enrolled in the setmelanotide study his body-weight was stable (130.6 kg; -9 kg from pre-operative weight, 49 months post-surgery; BMI 39.9 ^{kg}/ₘ²; BMI SDS: 3.57, height 181 cm). His global morning hunger-score measured 9/10, (Likert rating scale: 0 points= no hunger; 10 points=severe hunger). Following a careful dose escalation, prolonged treatment with a final dosage of 1.5 mg setmelanotide injected subcutaneously once per day (s.c./q.d.) notably reduced hyperphagia (from 9 to 1-2). Body weight was reduced by 21.6% (28.2 kg) after 26 weeks of treatment (Fig. 1d). On this dose, his weight has remained stable over further 35 weeks (-25.1 kg [19.2%]) At this time he had been on treatment for 61 weeks. The treatment has been well tolerated.

The second adult individual (a 22 year old male from the United Kingdom), with a homozygous *LEPR* mutation (p.H684P), had a history of sustained weight gain since his first month of life⁷ (Fig. 2b). He had been unable to stabilize his body weight for any prolonged period of time; at time of enrollment, his weight was 122.1 kg (BMI 40.7 ^{kg}/ₘ², BMI SDS 3.26, height 173.3 cm) and his hunger-score showed severe hunger (initial score of 9.5 out of 10 points). He was marginally responsive to setmelanotide at the initial lower dosages, but his hunger-score decreased when his dose was increased from 1.5 to 2 mg s.c. per day (hunger-score 4). On this dose, he lost 9.6 kg by 17 weeks and 13.9 kg (11.4%) body-weight over 36 weeks (Fig. 1d). Despite good tolerance of treatment and marked weight loss the subject still felt dissatisfaction at the rate of weight loss and independently discontinued his setmelanotide administration for ~2 weeks. During this time, he regained 5.2 kg and his hunger-score increased to severe hunger (hunger-score 9). The subject reported that he felt hungry every hour and was struggling to control his appetite. As a result, treatment was re-initiated at the 2 mg daily dose, and then was increased to 2.5 mg/q.d. with the goal of accelerating his weight loss; as a result, he demonstrated a significant reduction in hunger (hunger-score 2) and body-weight (Fig. 1e). He continues treatment with good tolerability.

The third subject is the 14-year-old adolescent sister of subject 1, carrying the same homozygous *LEPR* mutation (c.2357T>C, p.L786P). She had an initial body-weight, before treatment, of 120.6 kg (BMI 44.2 ^{kg}/ₘ²; BMI SDS 3.65; height 165 cm) (Fig. 2c). Again, she consistently gained weight throughout her life. She lost 10.0 kg during the first 13 weeks when titrated to a maximum dose of 1.5 mg. Her hunger-score was reduced from 9 to 5 (Fig. 1f). Before and during the treatment the subject showed adolescent behavior, which compromised compliance with the s.c./q.d. treatment regimen. This was manifested by a misunderstanding, which led her to perform the injections late in the afternoon and at an incorrect (lower) dosage of 1.0 mg setmelanotide. Since we have noted a dose-threshold for therapeutic benefit, and with a 10-12 hour half-life of s.c. setmelanotide injections we concluded that the combination of a lower dose and the maximum drug concentration occurring during the night most likely precipitated an interval of weight regain (Fig. 1f). In support of this explanation, her hunger feeling was reduced during the night but started to increase during the day. Eventually, this dosing error was recognized and corrected. As a result, she continued on a dosage of 2.0 mg/q.d. setmelanotide, under careful monitoring, leading again to reduced hunger. Weight data are being accumulated.

Safety laboratory parameters and vital parameters including blood pressure did not change significantly in the three patients (Tables 1-3 and Fig. 3). All subjects tolerated the injections well. Setmelanotide treatment led to only mild adverse events (Table 4). As seen in prior trials, the treatment led to darkening of the skin and a change in hair-color (subject 2 from red to brown; Fig. 4); these effects reflect melanocortin-1-receptor activation by setmelanotide. Metabolic parameters, including pretreatment hyperinsulinemia, showed clear trends towards normalization, in parallel with the reduction of body weight (Tables 1-3).

These clinical data support the notion that treatment with setmelanotide results in a reduction of hyperphagia and body weight, without occurrence of severe side-effects and extends the therapeutic effects observed in individuals with POMC-deficiency to subjects with LEPR defects (mean weight loss - 11.07 kg within the first 13 weeks). The severe weight-regain and marked recurrence of hunger, evident following treatment withdrawal in subject 2, and the effects of misdosing in subject 3 strongly support the consistent results of setmelanotide treatment when used optimally. These subjects with LEPR deficiency experienced profound and long-lasting reductions of body-weight for the first time in their lives as even a previous attempt to reduce weight by bariatric surgery had failed (subject 1). We anticipate that the weight loss observed will drive long-term clinical benefits, along with improved quality of life due to reduction in hunger and food craving. It is still unknown whether the magnitude of weight loss in individuals with LEPR deficiency will approach the effects seen following long-term treatment of subjects with POMC deficiency. These observations suggest that other individuals with monogenic obesity due to mutations in the *LEPR* gene⁸ and other genes (*POMC*) in the MC4R-pathway^{9,10} may benefit from setmelanotide treatment.

### Example 2: Determination of phospholipase C activation after setmelanotide, alpha-MSH and LY2112688 challenge.

While the results of the clinical trial are striking we aim to elucidate the underlying molecular mechanisms to explain setmelanotide's clinical effects, which will be helpful for improving personalized treatment regimens for this drug and for the future development of new MC4R agonists. In addition, we aim to understand the different side effect profile of setmelanotide when compared to former first generation MC4R agonists (e.g. LY2112688). The main signaling pathway of MC4R described to date involves Gαs/adenylyl cyclase¹¹. Alternative signaling pathways have been proposed¹². Several lines of evidence argue for additional MC4R-mediated intracellular responses; e.g. Gβγ of Gi/o and Gα_{q}¹³. The importance of these findings was first highlighted after targeted inactivation of Gα_{q} in the hypothalamic paraventricular nucleus of mice, the predominant site of MC4R-regulated body-weight, which resulted in increased food intake and body-weight¹⁴. We first measured the EC50 values of ligand-induced cAMP accumulation (Gαs/adenylyl cyclase signaling) in MC4R expressing *HEK293* cells, following exposure to increasing concentrations of setmelanotide, LY2112688 or alpha-MSH. In these studies, setmelanotide was more more potent in activating Gαs signaling (EC₅₀: 3.9 ± 1.7*10⁻⁹M) than alpha-MSH (EC₅₀: 2.3 ± 0.7 *10⁻⁸M) or LY2112688 (EC₅₀: 1.4 ± 0.4 *10⁻⁸M) (fig. 5). We next investigated signaling mediated through phospholipase C (beta) (PLC) using a nuclear factor of activated T-cells (NFAT) reporter gene assay (Fig. 6a). In these experiments, we identified potency differences between setmelanotide and first generation MC4R agonists: Setmelanotide (Fig. 6b) was over 100-fold more potent to stimulate NFAT signaling (EC₅₀: 5.9 ± 1.8 +10⁻⁹M) compared to alpha-MSH (EC₅₀: 4.8 ± 2.6 *10⁻⁷M) or LY2112688 (EC₅₀: 3.3 ± 1.9 *10⁻⁷M) (Fig. 6c,d). This effect was specific for the MC4R as the MC3R did not mediate PLC via NFAT for either ligand (Fig. 7). Because activation of PLC might result either from Gα_{q} or/and Gβγ of Gi/o, we measured NFAT reporter activity in the presence of pertussis toxin (PTX), which blocks Gi/o activation (Fig. 6e-h), establishing that the effects most likely resulted from setmelanotide specific Gα_{q} activation. Taken together this data demonstrates a striking difference of MC4R activation by setmelanotide compared to alpha-MSH and a first-generation synthetic agonist LY2112688 with a higher efficacy via Gαs/adenylyl cyclase signaling and a 100-fold higher activation of non-Gαs, most likely Gα_{q} signaling when compared to the other two ligands.

We also investigated whether setmelanotide might differentially impact the effects of the inverse agonist AgRP¹⁵ when compared to other MC4R agonists. We analyzed the effect of AgRP on activation of PLC via NFAT in *HEK293* cells (Fig. 6i). Interestingly, setmelanotide activation of the MC4R could not be antagonized by 100 nM AgRP (Fig. 6j), which contrasts the ability of 100 nM AgRP to fully antagonize alpha-MSH (Fig. 6k) or LY2112688 (Fig. 6I). We conclude that MC4R activation by setmelanotide is only marginally inhibited by AgRP, where PLC signaling was not affected and Gαs signaling was less affected when setmelanotide and LY2112688 are compared. The higher potency of setmelanotide to activate PLC/ NFAT signaling compared to LY2112688 or alpha-MSH and its increased capacity to overcome AgRP antagonism compared to LY2112688 may explain the remarkable efficacy of setmelanotide for appetite control in individuals with severe hyperphagia. This is relevant as Gαq activation is mainly linked to body weight regulation while Gαs activation is predicted to increase sympathetic tone and blood pressure.

### Example 3: Structural models of MC4R-ligand complexes.

To gain additional structural insights into the molecular mechanisms of alpha-MSH and setmelanotide agonist action at the MC4R, we built a three-dimensional MC4R model and docked both ligands within this model by using short-time molecular dynamic simulations where ligand binding mode differences could be predicted when compared between setmelanotide and LY2112688 (Fig. 8-10).

Our human (h) MC4R/alpha-MSH and hMC4R/setmelanotide complex models suggest that the peptidic agonists bind generally into a cleft between the extracellular loops (E1-3) and the transmembrane helices (TMHs or Hs) (Fig. 8 and 9). Approximately twenty hMC4R amino acids constituting the ligand binding pockets. Specific parts of the N-terminus are also supposed to participate in alpha-MSH binding, e.g. by interactions of Lys33 or Asp37. However, these residues are not conserved among members of the MCR group, which may exclude a significant role for alpha-MSH binding.

Analyzing the docking complexes specific emphasis relays on the interactions and localization of the conserved ligand motif HFRW. For alpha-MSH this central amino acid motif (6HFRW9) is known to be involved in ligand recognition and induction of ligand effects and here supposed main interactions with the receptor are (α-MSH/MC4R): Trp9/Phe261; Arg8/Glu100, Asp126, Asp122; Phe7/Phe184; and His6/Tyr268. Most of these interactions are also supposed for setmelanotide, e.g. the Arg6 (corresponds with Arg8 in α-MSH) interact with negatively charged side chains of MC4R Glu100, Asp122 and Asp126. Moreover, Trp9 in alpha-MSH and corresponding Trp7 in setmelanotide are both embedded by aromatic interactions with Phe261 in H6 and Phe284 in H7, in close proximity also to a MC4R activation related residues24,25 Tyr268 (H6) and His283 in the H7-E3 transition.

Despite partial overlap in the MC4R/alpha-MSH and MC4R/setmelanotide interaction pattern also differences can be observed, which might be reasoned by the fact that setmelanotide is a cyclic peptide with a disulfide bridge between two cysteines and in consequence is more rigid in the backbone compared to MSH. Specifically the ligand histidines in the ligand HFRW motifs are oriented differently in both docking poses. The histidine in setmelanotide is located between Asn123 (H3) and Phe184 (H4), but in the α-MSH/MC4R complex the corresponding His6 is directed between Tyr268 and Ser191 of the receptor. Moreover, alpha-MSH Phe7 interacts with Cys130 in H3 and Phe184 in H4, whereby the corresponding Phe5 of setmelanotide is oriented towards H6 and interacts additionally with Phe261. Therefore, it can be postulated that despite shared interactions of both ligands with MC4R these differences in detailed interactions contribute to the diverse pharmacological profiles (Fig. 11).

### Example 4: Functional characterization of like wild-type MC4R variants in Gas and PLC signaling after alpha-MSH and setmelanotide challenge.

Given the fact that cardiovascular-related adverse effects have not been described in individuals treated with setmelanotide, which contrasts the data obtained with LY2112688, it is tempting to speculate that setmelanotide's differential signaling profile and MC4R binding mode, may contribute to its safety profile. Based on this *in vitro* data and based on the previous findings in rodent models¹⁶ we have identified that MC4R signaling through non- Gαs pathways is of importance for body weight regulation.

Most of the *MC4R* variants identified in humans so far have been characterized^{11,13} focusing on Gαs signaling. As setmelanotide strongly activates PLC signaling via NFAT activation when compared to LY2112688, we re-analyzed *MC4R* genetic variants that were assumed to be "like wild-type" based on Gαs activation, for a potential NFAT signaling defect. These *MC4R* variants with normal Gαs signaling have been identified in subjects with severe obesity and in whom a mechanistic explanation for their obesity has not been available.

We identified that several of these *MC4R* variants, that were not affected in their cAMP response to alpha-MSH (Fig. 11a and Table 5), showed notably impaired alpha-MSH induced NFAT activation that did not allow the determination of EC50 values (Fig. 11c and Table 5). Strikingly, PLC signaling via NFAT for these variants was restored following setmelanotide challenge (Fig. 11d).

Therefore, it seems - in addition to individuals with MC4R deficiency with impaired Gαs signaling in which setmelanotide treatment resulted in reductions of body weight¹⁰ - that a significant number of subjects with obesity who carry an *MC4R* mutation that was hitherto assessed as "functionally wild-type", are affected by a pathogenic non-Gαₛ MC4R signaling defect. Approximately 5% of subjects with early onset obesity are carriers of *MC4R* mutations but only 1.7% of them are carriers of loss-of function mutations impacting MC4R cell surface expression and/or Gαs signaling^{10,17}. Treatment of these variants is now warranted, to establish the broader impact of non-Gas MC4R signaling in obesity.

### Example 5: MC4R deficiency associated with impaired NFAT:

Characterization of MC4R variants with impaired NFAT signaling was carried out. As is shown in Fig. 13, setmelanotide rescues impaired NFAT signaling in cAMP-functional MC4R variants. A number of MC4R mutations have been identified, as mentioned above, that exhibit "wild-type" like characteristics. In other words, these MC4R variants do not respond effectively to treatment with alpha MSH. This is observed by the lack of significant response when assessing cAMP accumulation after alpha-MSH treatment (Fig. 13a). These MC4R variants refer to, without limitation, T111, S77L, T112M, V166I, I170V, A175T, T178M, I251L and N274S.

However, when assessing NFAT signaling via PLC activation, these MC4R variants exhibit clearly reduced NFAT signaling compared to MC4R-WT (Fig. 13b). After alpha-MSH treatment the PLC activation is significantly reduced. Importantly, the treatment of these MC4R variants leads to rescue of this phenotype. As shown in Fig. 13c, setmelanotide treatment leads to an increase in NFAT signaling (as measured by PLC activation) of these variants, in the majority of cases bringing NFAT signaling back to levels comparable with WT.

### Example 6: Investigation of NFAT signaling in mouse embryonic fibroblasts (MEF) deficient in Gq (MEF Dq):

MEF were transfected with MC4R and the NFAT reporter in the absence and presence of Gq. NFAT signaling was measured after challenge with alpha-MSH, setmelanotide, RM-511 and Ly (10⁻⁶M). As can be observed in Fig. 14, in the presence of Gq, NFAT signaling is strongly increased, indicating that MC4R coupling to Gq is involved in the NFAT signaling.

### Example 7: Test of further MC4R variants:

Additional experiments were conducted with MC4R variants R7C, S36T, D37G, C40Y, V95I, V103I, S127L, S191T, M200del, F202L, C271R and E308L. For testing Gs signaling, dynamic formation of cAMP was measured after alpha-MSH challenge. Variants classified as Gs like wild-type as well as several Gs loss-of-function variants were included.

As shown in Fig. 15A, cAMP accumulation after alpha-MSH treatment is essentially not affected in the "like-WT" MC4R variants. These variants continue to show WT-similar cAMP levels after alpha-MSH treatment. The loss-of-function variants show inhibited cAMP levels after alpha-MSH treatment. As shown in Fig. 15B, NFAT signaling was assessed after alpha-MSH treatment. The variants typically show reduced NFAT signaling, lower than WT levels. Treatment with alpha-MSH was unable to rescue the NFAT signaling back to WT levels. Setmelanotide challenge was performed and the fold rescue of each variant after setmelanotide challenge was calculated (Fig. 15C). As is hown in Fig. 15C, setmelanotide treatment leads to an increase in NFAT signaling (as measured by PLC activation) of these variants, in the majority of cases bringing NFAT signaling back to levels comparable with WT.

### Conclusion of examples

In summary, we demonstrate that setmelanotide treatment was well tolerated and led to significant amelioration of hunger and profound weight reduction in individuals with LEPR-deficiency. Our observations open new avenues to treat subjects with LEPR-deficiency, and additional patient populations with genetic deficiencies *(MC4R, Leptin, POMC, PCSK1, Magel2, CPE)* leading to impaired functioning of the MC4R-pathway^{11,12}.

We provide novel molecular evidence of setmelanotide action on the MC4R showing a potential role of Gα_{q} signaling. PLC/ NFAT signaling and the ability to overcome AgRP antagonism by setmelanotide explains the improved efficacy of setmelanotide compared to former MC4R agonists.

Moreover, impaired PLC/ NFAT signaling through a subset of *MC4R* variants plays an important role in the development of obesity. In addition to LEPR and POMC deficiency, MC4R variant carriers with impaired PLC signaling via NFAT, will benefit from setmelanotide treatment and other similar agents, similar to the proposal that some Gαs impaired MC4R variants may respond disproportionally well to setmelanotide¹⁰. As pharmacotherapy treatment options for genetic defects of central weight regulation are lacking, setmelanotide-mediated neuropeptide replacement in the MC4R-pathway now leads to weight and appetite control for obese MC4R-mediated pathway-deficient patients, as described herein.

### TABLES

**Table 1. Laboratory parameters subject 1. Laboratory safety parameter and oral glucose tolerance test results of subject 1 with LEPR deficiency.**

| Laboratory values subject with LEPR deficiency 1: | | | | | |
|---|---|---|---|---|---|
| | **Pre-study** | **After 13 weeks** | **After 27 weeks** | **After 61 weeks** | **Reference value** |
| Cholesterol (mg/dl) | 168 | 135 | 138 | 127 | <200 |
| HDL (mg/dl) | 52 | 46 | 55 | 62 | >45 |
| LDL (mg/dl) | 102 | 78 | 76 | 63 | <130 |
| Triglyceride (mg/dl) | 57 | 53 | 40 | 36 | <200 |
| ALT (U/I) | 20 | 19 | 10 | 28 | <41 |
| AP (U/l) | 62 | 64 | 67 | 60 | 40-130 |
| gamma-GT (U/l) | 21 | 13 | 10 | 17 | 8-61 |
| Cortisol (nmol/l) | 199.8 | 319.4 | 243.2 | 287.0 | |
| Testosterone (µg/l) | 4.64 | 2.38 | 6.81 | 5.53 | 2.18-9.06 |
| fT4 (ng/l) | 12.48 | 11.7 | 10.64 | 13.04 | 9.3-17 |
| TSH (mU/l) | 0.59 | 0.99 | 0.88 | 0.52 | 0.27-4.2 |
| HbA1c (%) | 5.1 | 5.1 | 5.2 | 5.2 | <6.0 |

### Oral glucose tolerance test subject with LEPR deficiency 1:

| | **Pre-study** | | **After 13 weeks** | | **After 27 weeks** | | **After 61 weeks** | |
|---|---|---|---|---|---|---|---|---|
| Time point (min) | Blood glucose (mg/dl) | Insulin (mU/l) | Blood glucose (mg/dl) | Insulin (mU/l) | Blood glucose (mg/dl) | Insulin (mU/l) | Blood glucose (mg/dl) | Insulin (mU/l) |
| 0 | 75 | 6.09 | 76 | 69.74 | 73 | 3.72 | 71 | 1.89 |
| 30 | 164 | 119.8 | 108 | 58.03 | 131 | 9.23 | 113 | 39.80 |
| 60 | 147 | 89.92 | 152 | 92.55 | 74 | 56.24 | 161 | 106.00 |
| 90 | 130 | 67.02 | 128 | 66.44 | 64 | 30.25 | 136 | 65.10 |
| 120 | 114 | 46.02 | 117 | 64.44 | 101 | 42.94 | 104 | 29.40 |

**Table 2. Laboratory parameters subject 2. Laboratory safety parameter and oral glucose tolerance test results of subject 2 with LEPR deficiency.**

| Laboratory values subject with LEPR deficiency 2: | | | | |
|---|---|---|---|---|
| | **Pre-study** | **After 12 weeks** | **After 52 weeks** | **Reference value** |
| Cholesterol (mg/dl) | 173 | 186 | 184 | <200 |
| HDL (mg/dl) | 34 | 44 | 49 | >45 |
| LDL (mg/dl) | 122 | 138 | 124 | <130 |
| Triglyceride (mg/dl) | 141 | 123 | 103 | <200 |
| AP (U/I) | 74 | 80 | 64 | 40-130 |
| Cortisol (nmol/l) | 184.8 | 364.4 | 216 | |
| Testosterone (µg/l) | 2.11 | 3.78 | 2.82 | 2.18-9.06 |
| fT4 (ng/l) | 11.49 | 10.55 | 9.73 | 9.3-17 |
| TSH (mU/l) | 1.27 | 2.43 | 1.85 | 0.27-4.2 |
| HbA1c (%) | 5.3 | 5.0 | 5.3 | <6.0 |

### Oral glucose tolerance test subject with LEPR deficiency 2:

| | **Pre-study** | | **After 12 weeks** | | **After 52 weeks** | |
|---|---|---|---|---|---|---|
| Time point (min) | Blood glucose (mg/dl) | Insulin (mU/l) | Blood glucose (mg/dl) | Insulin (mU/l) | Blood glucose (mg/dl) | Insulin (mU/l) |
| 0 | 74 | 15.25 | 77 | 17.29 | 73 | 9.12 |
| 30 | 131 | 128.5 | 109 | 95.59 | 152 | 11.23 |
| 60 | 153 | 126.8 | 121 | 75.20 | 137 | 69.40 |
| 90 | 136 | 115.4 | 161 | 102.20 | 126 | 100.40 |
| 120 | 129 | 131.2 | 145 | 93.11 | 114 | 107.40 |

**Table 3. Laboratory parameters subject 3. Laboratory safety parameter and oral glucose tolerance test results of subject 3 with LEPR deficiency.**

| Laboratory values subject with LEPR deficiency 3: | | | | |
|---|---|---|---|---|
| | **Pre-study** | **After 13 weeks** | **After 34 weeks** | **Reference values** |
| Cholesterol (mg/dl) | 150 | 134 | 151 | 92-234 |
| HDL (mg/dl) | 34 | 34 | 43 | >45 |
| LDL (mg/dl) | 99 | 92 | 102 | <130 |
| Triglyceride (mg/dl) | 92 | 82 | 46 | <200 |
| AP (U/I) | 24 | na | 67 | <187 |
| Cortisol (nmol/l) | 206 | 175.1 | 266 | 48.0-579.0 |
| Prolactin (µg/l) | 8.38 | 6.06 | 9.27 | 4.2-29.01 |
| T4 (µg/l) | 79.5 | 75.8 | 81.7 | 59.1-132.0 |
| fT4 (ng/l) | 12.2 | 13.04 | 13.21 | 8.90-14.90 |
| TSH (mU/l) | 1.45 | 0.63 | 1.14 | 0.60-4.00 |
| HbA1c (%) | 5.3 | 5.0 | 5.4 | <6.0 |

### Oral glucose tolerance test subject with LEPR deficiency 3:

| | **Pre-study** | | **After 13 weeks** | | **After 34 weeks** | |
|---|---|---|---|---|---|---|
| Time point (min) | Blood glucose (mg/dl) | Insulin (mU/l) | Blood glucose (mg/dl) | Insulin (mU/l) | Blood glucose (mg/dl) | Insulin (mU/l) |
| 0 | 86 | 395.7 | 69 | 5.86 | 83 | 32.90 |
| 30 | 161 | 231.3 | 99 | 127.90 | 132 | 220.00 |
| 60 | 140 | 296.2 | 144 | 162.30 | 146 | 281.00 |
| 90 | 129 | na | 149 | 260.80 | 103 | 284.00 |
| 120 | 137 | 378.0 | 94 | 54.85 | 112 | 230.00 |

**Table 4. Summary of adverse events. Adverse events (AEs), which have occurred during the treatment duration of the subjects with LEPR deficiency, are listed with information about frequency, severity and duration. In cases where more than one subject reported an AE, the duration for each individual is listed in days. The total duration of treatment reported in this AE table is ~574 days for subject 1, ~483 days for subject 2, and ~343 days for subject 3.**

| **AE description** | **Number of patients in which AE occurred** | **Severity** | **Duration (days; listed for each patient)** |
|---|---|---|---|
| Reduced appetite | 3 | severe | ongoing |
| Increased tanning of the skin/ nevi | 3 | severe | ongoing |
| Skin folliculitis | 1 | mild | ongoing |
| Pain at injection site | 2 | mild | 3/8 |
| Induration/ hematoma at injection site | 2 | mild | 2/2 |
| Bone & muscular pain | 2 | mild | 2/4 |
| Dry mouth | 3 | mild | 2 / 7 / ongoing |
| Headache | 3 | mild | 1 / 2 / 7 |
| Nausea | 1 | mild | 2 |
| Abdominal pain | 1 | mild | 1 |
| | | | |

| **SAE description** | | | |
|---|---|---|---|
| none | | | |

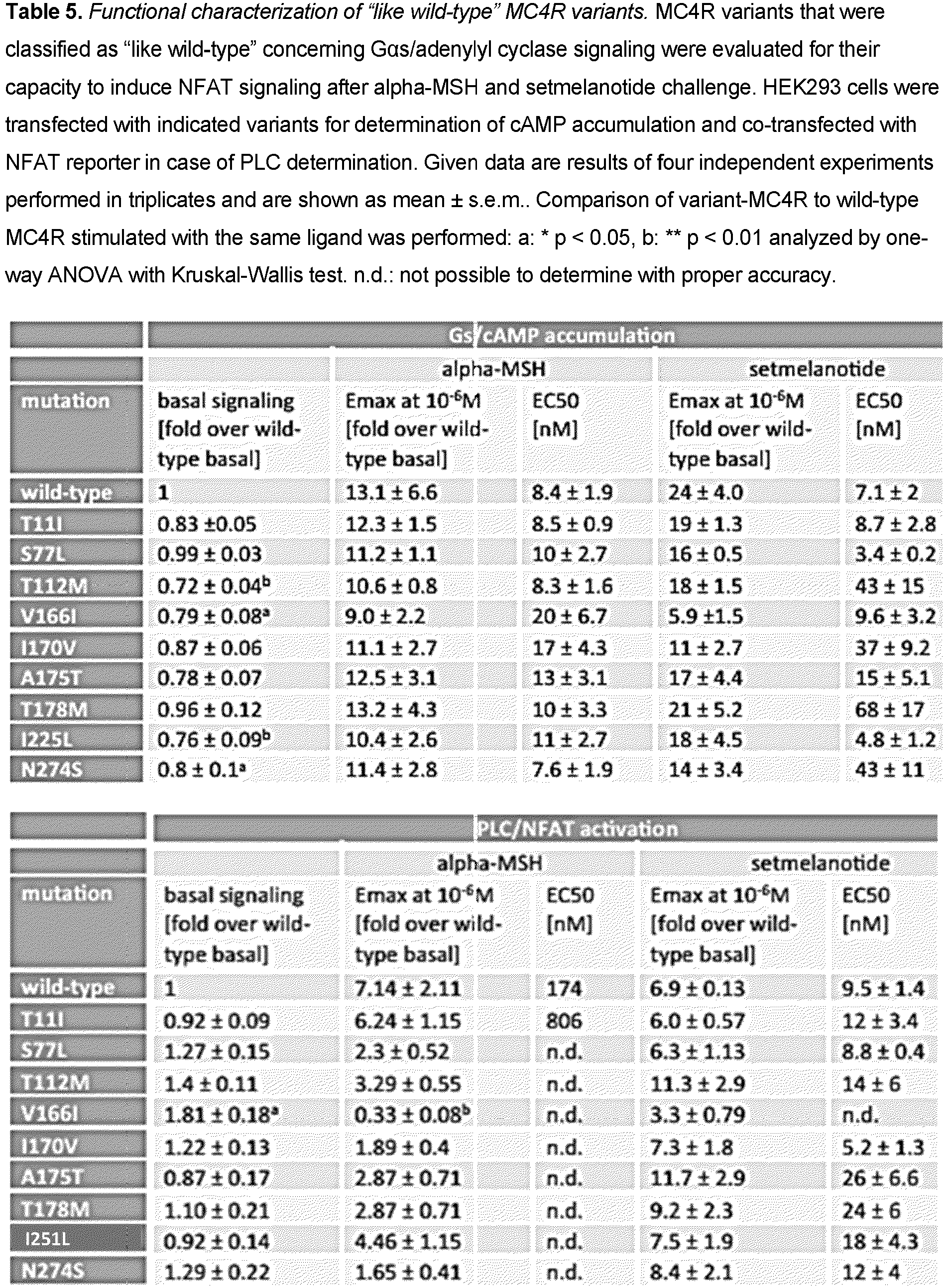

### References

1. Farooqi, I.S. & O'Rahilly, S. 20 years of leptin: human disorders of leptin action. J Endocrinol 223, T63-70 (2014).
2. Clement, K., et al. A mutation in the human leptin receptor gene causes obesity and pituitary dysfunction. Nature 392, 398-401 (1998).
3. Farooqi, I.S., et al. Effects of recombinant leptin therapy in a child with congenital leptin deficiency. N Engl J Med 341, 879-884 (1999).
4. Krishna, R., et al. Potent and selective agonism of the melanocortin receptor 4 with MK-0493 does not induce weight loss in obese human subjects: energy intake predicts lack of weight loss efficacy. Clin Pharmacol Ther 86, 659-666 (2009).
5. Jackson, V.M., Price, D.A. & Carpino, P.A. Investigational drugs in Phase II clinical trials for the treatment of obesity: implications for future development of novel therapies. Expert Opin Investig Drugs 23, 1055-1066 (2014).
6. Kuhnen, P., et al. Proopiomelanocortin Deficiency Treated with a Melanocortin-4 Receptor Agonist. N Engl J Med 375, 240-246 (2016).
7. Farooqi, I.S., et al. Clinical and molecular genetic spectrum of congenital deficiency of the leptin receptor. N Engl J Med 356, 237-247 (2007).
8. Huvenne, H., et al. Seven novel deleterious LEPR mutations found in early-onset obesity: a DeltaExon6-8 shared by subjects from Reunion Island, France, suggests a founder effect. J Clin Endocrinol Metab 100, E757-766 (2015).
9. Bischof, J.M., Van Der Ploeg, L.H., Colmers, W.F. & Wevrick, R. Magel2-null mice are hyper-responsive to setmelanotide, a melanocortin 4 receptor agonist. Br J Pharmacol 173, 2614-2621 (2016).
10. Collet, T.H., et al. Evaluation of a melanocortin-4 receptor (MC4R) agonist (Setmelanotide) in MC4R deficiency. Molecular Metabolism 1-9(2017).
11. Gantz, I., et al. Molecular cloning, expression, and gene localization of a fourth melanocortin receptor. J Biol Chem 268, 15174-15179 (1993).
12. Ghamari-Langroudi, M., et al. G-protein-independent coupling of MC4R to Kir7.1 in hypothalamic neurons. Nature 520, 94-98 (2015).
13. Yang, L.K. & Tao, Y.X. Biased signaling at neural melanocortin receptors in regulation of energy homeostasis. Biochim Biophys Acta (2017).
14. Li, Y.Q., et al. G(q/11)alpha and G(s)alpha mediate distinct physiological responses to central melanocortins. J Clin Invest 126, 40-49 (2016).
15. Ollmann, M.M., et al. Antagonism of central melanocortin receptors in vitro and in vivo by agouti-related protein. Science 278, 135-138 (1997).
16. Buch, T.R., Heling, D., Damm, E., Gudermann, T. & Breit, A. Pertussis toxin-sensitive signaling of melanocortin-4 receptors in hypothalamic GT1-7 cells defines agouti-related protein as a biased agonist. J Biol Chem 284, 26411-26420 (2009).
17. Stutzmann, F., et al. Prevalence of melanocortin-4 receptor deficiency in Europeans and their age-dependent penetrance in multigenerational pedigrees. Diabetes 57, 2511-2518 (2008).
18. Dinter, J., et al. 3-iodothyronamine differentially modulates alpha-2A-adrenergic receptor-mediated signaling. J Mol Endocrinol 54, 205-216 (2015).
19. Tarnow, P., Schoneberg, T., Krude, H., Gruters, A. & Biebermann, H. Mutationally induced disulfide bond formation within the third extracellular loop causes melanocortin 4 receptor inactivation in patients with obesity. J Biol Chem 278, 48666-48673 (2003).
20. Rasmussen, S.G., et al. Crystal structure of the beta2 adrenergic receptor-Gs protein complex. Nature 477, 549-555 (2011).
21. Hanson, M.A., et al. Crystal structure of a lipid G protein-coupled receptor. Science 335, 851-855 (2012).
22. Koehbach, J., et al. Oxytocic plant cyclotides as templates for peptide G protein-coupled receptor ligand design. Proc Natl Acad Sci U S A 110, 21183-21188 (2013).
23. Ericson, M.D., et al. Bench-top to clinical therapies: A review of melanocortin ligands from 1954 to 2016. Biochim Biophys Acta 1863, 2414-2435 (2017).
24. Tao, Y.X. The melanocortin--4 receptor: physiology, pharmacology, and pathophysiology. Endocr Rev 31, 506--543 (2010).
25. Hogan, K., et al. Mapping the binding site of melanocortin 4 receptor agonists: a hydrophobic pocket formed by I3.28(125), I3.32(129), and I7.42(291) is critical for receptor activation. J Med Chem 49, 911--922 (2006).
26. Laskowski, R.A. & Swindells, M.B. LigPlot+: multiple ligand--protein interaction diagrams for drug discovery. J Chem Inf Model 51, 2778--2786 (2011).
27. Kromeyer--Hauschild, M.W., D. Kunze, F. Geller, H.C. Geiß, V. Hesse, A. von Hippel, U. Jaeger, D. Johnsen, W. Korte, K. Menner, G. Müller, J.M. Müller, A. Niemann-Pilatus, T. Remer, F. Schaefer, H.--U. Wittchen, S. Zabransky, K. Zellner, A. Ziegler, J. Hebebrand. Perzentile für den Body--mass--Index für das Kindes-- und Jugendalter unter Heranziehung verschiedener deutscher Stichproben. Monatszeitschrift Kinderheilkude 149, 807--818 (2001).
28. Ericson, M.D., et al. Bench--top to clinical therapies: A review of melanocortin ligands from 1954 to 2016. Biochim Biophys Acta 1863, 2414--2435 (2017).
29. McDonald, I.K. & Thornton, J.M. Satisfying hydrogen bonding potential in proteins. J Mol Biol 238, 777--793 (1994).

## Claims

1. Melanocortin-4 receptor (MC4R) agonist, selected from setmelanotide and RM511, for use in the treatment and/or prevention of a medical condition associated with MC4R deficiency, selected from obesity, a metabolic syndrome and/or hyperphagia, in a subject having an MC4R deficiency associated with impaired Nuclear factor of activated T-cells (NFAT) signaling.

2. MC4R agonist for use according to the preceding claim, wherein the MC4R deficiency in the subject is not associated with impaired G_{αs} signaling.

3. MC4R agonist for use according to any one of the preceding claims, wherein the MC4R deficiency is a genetic deficiency.

4. MC4R agonist for use according to the preceding claim, wherein the MC4R genetic deficiency is a MC4R mutation at one or more of the following positions of the MC4R, namely T112, S77, V166, 1170, A175, T178, I251 and N274, more preferably S77, 1170 and N274, or a mutation selected from the list consisting of T112M, S77L, V166I, I170V, A175T, T178M, I251L and N274S, more preferably S77L, I170V and N274S.

5. MC4R agonist for use according to any one of the preceding claims, wherein the MC4R deficiency is an epigenetic deficiency.

6. MC4R agonist for use according to any one of the preceding claims, wherein the medical condition associated with MC4R deficiency is early onset obesity.

7. In vitro method for the diagnosis, prognosis and/or assessment of likelihood of whether a subject with, or at risk of having and/or developing, a medical condition associated with MC4R deficiency, selected from obesity, a metabolic syndrome and/or hyperphagia, will respond to treatment with an MC4R agonist, selected from setmelanotide and RM511, the method comprising:
- providing a sample from said subject, and
- determining whether the subject has an MC4R deficiency associated with impaired NFAT signaling by assessing said sample,
- wherein the presence of an MC4R deficiency associated with impaired NFAT signaling is indicative that treatment with the MC4R agonist will be effective in said subject.

8. Method for determining whether a subject has an MC4R deficiency associated with impaired NFAT signaling, wherein said subject has, or is at risk of having and/or developing, a medical condition associated with MC4R deficiency, selected from obesity, a metabolic syndrome and/or hyperphagia, the method comprising providing a sample from said subject and determining whether the subject has an MC4R deficiency associated with impaired NFAT signaling by assessing said sample.

9. Method according to claim 7 or 8, wherein assessing the sample comprises assessing NFAT signaling in a reporter system comprising nucleic acid sequences corresponding to those determined in patient-specific MC4R genetic material.

10. Method according to the preceding claim, wherein reporter system comprises means for determining phospholipase C (PLC) activation.

11. Method according to claim 7 or 8, wherein assessing the sample comprises determining nucleic acid sequence characteristics of patient-specific MC4R genetic material and preferably incorporating nucleic acid sequences corresponding to those determined in patient-specific MC4R genetic material in a reporter system.

## Patentansprüche

1. Melanocortin-4-Rezeptor-(MC4R-)Agonist, ausgewählt aus Setmelanotid und RM511, zur Verwendung bei der Behandlung und/oder Prävention eines medizinischen Zustands, der mit einem MC4R-Mangel assoziiert ist, ausgewählt aus Fettleibigkeit, einem metabolischen Syndrom und/oder Hyperphagie, bei einem Patienten mit einem MC4R-Mangel, der mit einer gestörten Signalisierung des Nukleären Faktors aktivierter T-Zellen (NFAT) assoziiert ist.

2. MC4R-Agonist zur Verwendung nach dem vorhergehenden Anspruch, wobei der MC4R-Mangel bei dem Patienten nicht mit einer gestörten G_{αs}- Signalisierung assoziiert ist.

3. MC4R-Agonist zur Verwendung nach einem der vorhergehenden Ansprüche, wobei der MC4R-Mangel ein genetischer Mangel ist.

4. MC4R-Agonist zur Verwendung nach dem vorhergehenden Anspruch, wobei der genetische MC4R-Mangel eine MC4R-Mutation an einer oder mehreren der folgenden Positionen des MC4R ist, nämlich T112, S77, V166, I170, A175, T178, I251 und N274, bevorzugter S77, I170 und N274, oder eine Mutation, ausgewählt aus der Liste bestehend aus T112M, S77L, V166I, I170V, A175T, T178M, 1251L und N274S, bevorzugter S77L, I170V und N274S.

5. MC4R-Agonist zur Verwendung nach einem der vorhergehenden Ansprüche, wobei der MC4R-Mangel ein epigenetischer Mangel ist.

6. MC4R-Agonist zur Verwendung nach einem der vorhergehenden Ansprüche, wobei der mit dem MC4R-Mangel assoziierte medizinische Zustand früh einsetzende Fettleibigkeit ist.

7. In-vitro-Verfahren zur Diagnose, Prognose und/oder Beurteilung der Wahrscheinlichkeit, ob ein Patient mit einem medizinischen Zustand, der mit einem MC4R-Mangel assoziiert ist, ausgewählt aus Fettleibigkeit, einem metabolischen Syndrom und/oder Hyperphagie, oder mit dem Risiko, diesen zu haben und/oder zu entwickeln, auf die Behandlung mit einem MC4R-Agonisten, ausgewählt aus Setmelanotid und RM511, ansprechen wird, wobei das Verfahren umfasst:
- Bereitstellen einer Probe von dem Patienten, und
- Bestimmen, ob der Patient einen MC4R-Mangel aufweist, der mit einer gestörten NFAT-Signalisierung assoziiert ist, durch Beurteilen der Probe,
- wobei das Vorhandensein eines MC4R-Mangels, der mit einer gestörten NFAT-Signalisierung assoziiert ist, indikativ dafür ist, dass die Behandlung mit dem MC4R-Agonisten bei dem Patienten wirksam sein wird.

8. Verfahren zum Bestimmen, ob ein Patient einen MC4R-Mangel aufweist, der mit einer gestörten NFAT-Signalisierung assoziiert ist, wobei der Patient einen medizinischen Zustand aufweist oder das Risiko aufweist, diesen zu haben und/oder zu entwickeln, der mit einem MC4R-Mangel assoziiert ist, ausgewählt aus Fettleibigkeit, einem metabolischen Syndrom und/oder Hyperphagie, wobei das Verfahren das Bereitstellen einer Probe von dem Patienten und das Bestimmen, ob der Patient einen MC4R-Mangel aufweist, der mit einer gestörten NFAT-Signalisierung assoziiert ist, durch Beurteilen der Probe umfasst.

9. Verfahren nach Anspruch 7 oder 8, wobei das Beurteilen der Probe das Beurteilen der NFAT-Signalisierung in einem Reportersystem umfasst, das Nukleinsäuresequenzen umfasst, die denen entsprechen, die in patientenspezifischem genetischem MC4R-Material bestimmt wurden.

10. Verfahren nach dem vorhergehenden Anspruch, wobei das Reportersystem Mittel zum Bestimmen der Aktivierung der Phospholipase C (PLC) umfasst.

11. Verfahren nach Anspruch 7 oder 8, wobei das Beurteilen der Probe das Bestimmen von Nukleinsäuresequenzeigenschaften von patientenspezifischem genetischem MC4R-Material und vorzugsweise das Einbeziehen von Nukleinsäuresequenzen, die denen entsprechen, die in patientenspezifischem genetischem MC4R-Material bestimmt wurden, in ein Reportersystem umfasst.

## Revendications

1. Agoniste du récepteur de la mélanocortine-4 (MC4R), choisi parmi le setmélanotide et le RM511, pour une utilisation dans le traitement et/ou la prévention d'une condition médicale associée à une déficience en MC4R, choisie parmi l'obésité, un syndrome métabolique et/ou l'hyperphagie, chez un sujet présentant une déficience en MC4R associée à une signalisation altérée du facteur nucléaire des lymphocytes T activés (NFAT).

2. Agoniste du MC4R pour une utilisation selon la revendication précédente, dans lequel la déficience en MC4R chez le sujet n'est pas associée à une signalisation G_{αs} altérée.

3. Agoniste du MC4R pour une utilisation selon l'une quelconque des revendications précédentes, dans lequel la déficience en MC4R est une déficience génétique.

4. Agoniste du MC4R pour une utilisation selon la revendication précédente, dans lequel la déficience génétique en MC4R est une mutation du MC4R à l'une ou plusieurs des positions suivantes du MC4R, à savoir T112, S77, V166, I170, A175, T178, I251 et N274, de manière davantage préférée S77, I170 et N274, ou une mutation choisie dans la liste constituée de T112M, S77L, V166I, I170V, A175T, T178M, I251L et N274S, de manière davantage préférée S77L, I170V et N274S.

5. Agoniste du MC4R pour une utilisation selon l'une quelconque des revendications précédentes, dans lequel la déficience en MC4R est une déficience épigénétique.

6. Agoniste du MC4R pour une utilisation selon l'une quelconque des revendications précédentes, dans lequel la condition médicale associée à une déficience en MC4R est l'obésité précoce.

7. Procédé in vitro permettant le diagnostic, le pronostic et/ou l'évaluation de la probabilité qu'un sujet présentant, ou risquant de présenter et/ou de développer, une condition médicale associée à une déficience en MC4R, choisie parmi l'obésité, un syndrome métabolique et/ou l'hyperphagie, réponde à un traitement par un agoniste du MC4R, choisi parmi le setmélanotide et le RM511, le procédé comprenant :
- la fourniture d'un échantillon prélevé sur ledit sujet, et
- le fait de déterminer si le sujet présente une déficience en MC4R associé à une signalisation NFAT altérée en évaluant ledit échantillon,
- dans lequel la présence d'une déficience en MC4R associée à une signalisation NFAT altérée est indicative de l'efficacité d'un traitement par l'agoniste du MC4R chez ledit sujet.

8. Procédé permettant de déterminer si un sujet présente une déficience en MC4R associée à une signalisation NFAT altérée, dans lequel ledit sujet présente, ou risque de présenter et/ou de développer, une condition médicale associée à une déficience en MC4R, choisie parmi l'obésité, un syndrome métabolique et/ou l'hyperphagie, le procédé comprenant la fourniture d'un échantillon prélevé sur ledit sujet et le fait de déterminer si le sujet présente une déficience en MC4R associée à une signalisation NFAT altérée en évaluant ledit échantillon.

9. Procédé selon la revendication 7 ou 8, dans lequel l'évaluation de l'échantillon comprend l'évaluation de la signalisation NFAT dans un système rapporteur comprenant des séquences d'acide nucléique correspondant à celles déterminées dans un matériel génétique MC4R spécifique au patient.

10. Procédé selon la revendication précédente, dans lequel le système rapporteur comprend un moyen permettant de déterminer l'activation de la phospholipase C (PLC).

11. Procédé selon la revendication 7 ou 8, dans lequel l'évaluation de l'échantillon comprend la détermination de caractéristiques de séquence d'acide nucléique d'un matériel génétique MC4R spécifique au patient et de préférence l'incorporation de séquences d'acide nucléique correspondant à celles déterminées dans un matériel génétique MC4R spécifique au patient dans un système rapporteur.
